# EUROPEAN PATENT SPECIFICATION

(11) **EP 1 558 577 B1**
(45) Date of publication and mention of the grant of the patent: **30.12.2009**
(21) Application number: 03762615.7
(22) Date of filing: 02.07.2003
(51) Int. Cl.: C07D 211/34, C07D 211/64, C07D 223/04, C07D 223/06

(54) **SUBSTITUTED 4-PHENYL-PIPERIDIN-AMIDES AS TACHYKININ ANTAGONISTS AND SEROTONIN REPTAKE INHIBITORS**
SUBSTITUIERTE 4-PHENYLPIPERIDINAMIDE ALS TACHYKININANTAGONISTEN UND SEROTONINWIEDERAUFNAHMEHEMMER
4-PHENYL-PIPERIDINE-AMIDES SUBSTITUES COMME ANTAGONISTES DE TACHYKININE ET INHIBITEURS DE RECAPTURE DE SEROTINE

(30) Priority: 03.07.2002 GB 0215393; 20.03.2003 GB 0306454
(43) Date of publication of application: 03.08.2005
(73) Proprietor: GLAXO GROUP LIMITED, Greenford, Middlesex UB6 ONN (GB)
(72) Inventor: ALVARO, Giuseppe, GlaxoSmithKline, I-37135 Verona (IT); CARDULLO, Francesca, GlaxoSmithKline, I-37135 Verona (IT); DI FABIO, Romano, GlaxoSmithKline, I-37135 Verona (IT); GIOVANNINI, Riccardo, GlaxoSmithKline, I-37135 Verona (IT); PIGA, Elisabetta, GlaxoSmithKline, I-37135 Verona (IT); TRANQUILLINI, Maria, Elvira, GlaxoSmithKline, I-37135 Verona (IT)
(74) Representative: Giddings, Peter John
(86) International application number: PCT/EP2003/007127
(87) International publication number: WO 2004/005256

(56) References cited:
- WO-A-00/21931
- WO-A-94/13639
- WO-A-99/59972
- WO-A-02/083134
- WO-A-03/033486

## Description

The present invention relates to cyclic amine derivatives, to processes for their preparation, to pharmaceutical compositions containing them and to their medical use.

WO 9413639 discloses inter alia compounds of formula(A) as tachykinin antagonists 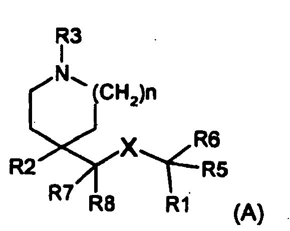 wherein
X is inter alia NR₄ in which R₄ is hydrogen or C ₁₋₆ alkyl ;
R₂ and R₅ represent interalia phenyl optionally substituted by 1,2 or 3 groups;
R₇ and R₈ may together represent an oxygen atom;
R₅ and R₆ represent interalia hydrogen or C ₁₋₆ alkyl.

WO 02083134 describes inter alia aryl and biaryl piperidine compounds of formula(B) having activity as antagonists for melanin concentrating hormone, 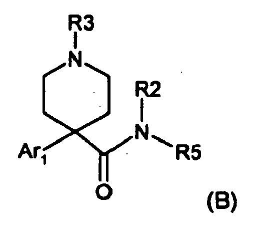 in which Ar₁ and R₅ defined as a phenyl group optionally substituted and R₂ is alkyl or hydrogen.

However, the compounds claimed according to the present invention are neither specifically disclosed nor suggested in the above cited documents.

The present invention thus provides compounds of formula (I) 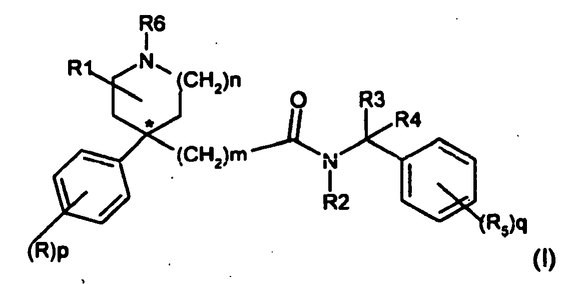 wherein
R represents halogen, C₁₋₄ alkyl, cyano, C₁₋₄ alkoxy, trifluoromethyl or trifluoromethoxy;
R₁ represents hydrogen, halogen, C₃₋₇cycloalkyl, hydroxy, nitro, cyano or C₁₋₄ alkyl optionally substituted by halogen, cyano or C₁₋₄ alkoxy;
R₂ represents hydrogen or C₁₋₄ alkyl;
R₃ and R₄ independently represent hydrogen, cyano, c₁₋₄ alkyl or R₃ together with R₄ represents C₃₋₇ cycloalkyl;
R₅ represents trifluoromethyl, S(O)t C ₁₋₄ alkyl, C₁₋₄ alkyl, C₁₋₄ alkoxy, trifluoromethoxy, halogen or cyano;
R₆ represents hydrogen or (CH₂)rR₇;
R₇ represents hydrogen, C₃₋₇cycloalkyl, NH(C₁₋₄alkylOC₁₋₄alkoxy), NH(C₁₋₄alkyl), N(C₁₋₄alkyl)₂, OC(O)NR₉R₈, NR₈ C(O) R₉ or C(O)NR₉R₈;
R₉ and R₈ independently represent hydrogen, C₁₋₄ alkyl or C₃₋₇ cycloalkyl;
m represents 1
n represents 1 or 2;
p is zero or an integer from 1 to 3;
q is an integer from 1 to 3;
r is an integer from 1 to 4;
t is 0 , 1 or 2;
   and pharmaceutically acceptable salts and solvates thereof.

Suitable pharmaceutically acceptable salts of the compounds of general formula (I) include acid addition salts formed with pharmaceutically acceptable organic or inorganic acids, for example hydrochlorides, hydrobromides, sulphates, alkyl- or arylsulphonates (e.g. methanesulphonates or p-toluenesulphonates), phosphates, trifluoroacetates, acetates, citrates, succinates, tartrates, lactates, malates, fumarates and maleates.

The solvates may, for example, be hydrates.

References hereinafter to a compound according to the invention include both compounds of formula (I) and their pharmaceutically acceptable acid addition salts and their pharmaceutically acceptable solvates.

It will be appreciated by those skilled in the art that the compounds of formula (I) provided that when n is 1 and R₁ is not hydrogen or n is 2 contain at least one chiral centre (namely the carbon atom shown as * in formula (I)) and may be represented by formula (1a) and (1 b).

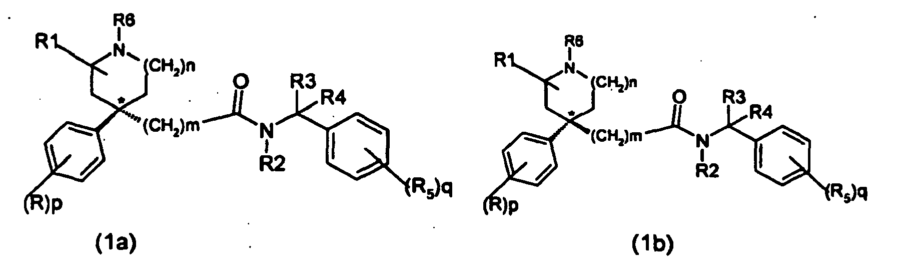

The wedge bond indicates that the bond is above the plane of the paper. The broken bond indicates that the bond is below the plane of the paper.

At least two asymmetyric carbon atoms are present in the compounds of formula (I) when R₁ is different from hydrogen (namely the carbon atom shown as * in formula (I) and the carbon atom to which the group R1 is attached) and may be represented by formula (1a) (1b) (1c) and (1d).

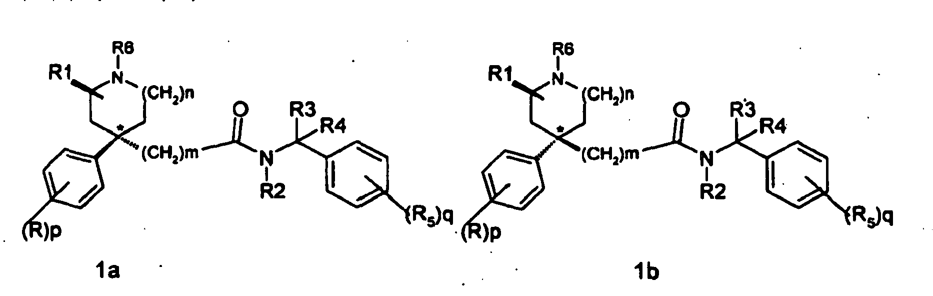

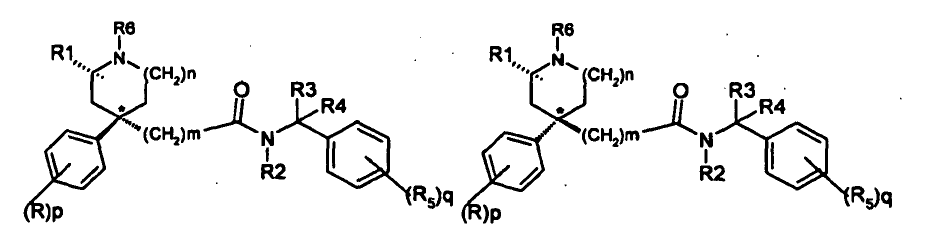

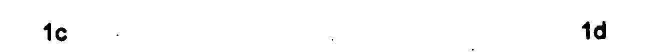

The configuration of the chiral carbon atoms of the compounds shown in formulae 1a and 1d is hereinafter referred to as syn configuration and in formulae 1b and 1c as the anti configuration.

Further asymmetric carbon atoms are possible when R₃ and R₄ are not the same group.

It is to be understood that all stereoisomeric forms including all enantiomers and mixtures thereof are encompassed within the scope of the present invention and the reference to compound of formula (I) include all stereisomeric forms unless otherwise stated.

The term C₁₋₄ alkyl as used herein as a group or a part of the group refers to a straight or branched alkyl group containing from 1 to 4 carbon atoms; examples of such groups include methyl, ethyl, propyl, isopropyl, n-butyl, isobutyl, tert-butyl, 1 methylethyl or 2-methyl propyl.

The term halogen refers to fluorine, chlorine, bromine or iodine.

The term C₃₋₇ cycloalkyl group means a non aromatic monocyclic hydrocarbon ring of 3 to 7 carbon atoms such as, for example, cyclopropyl, cyclobutyl, cyclopentyl, cyclohexyl or cycloheptyl.

The term C₁₋₄ alkoxy group may be a straight chain or a branched chain alkoxy group, for example methoxy, ethoxy, propoxy, prop-2-oxy, butoxy, but-2-oxy or methylprop-2-oxy.

In the compounds of formula (I) wherein n is 1 the group R₁ may be in position 2, 3, 5 or 6 of the piperidine ring as represented in formula (Ia)

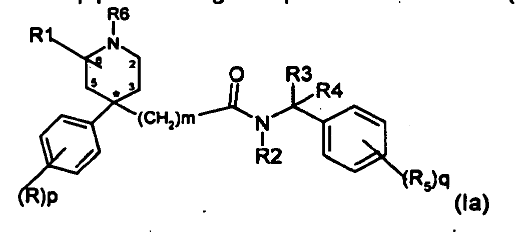

In the compounds of formula (I) wherein n is 2 the group R₁ may be in position 2, 3, 5, 6 or 7 of the ring as represented in formula (Ib) 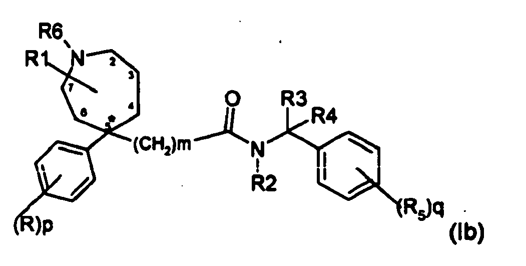
R is preferably halogen (e.g. fluorine or chlorine), cyano, trifluoromethyl or a C₁₋₄ alkyl (e.g. methyl) group and within this class p is preferably 0 or an integer from 1 to 2.
R₁ is preferably hydrogen, halogen (e.g. fluorine) or methyl.
R₂ is preferably hydrogen or methyl.
R₃ is preferably hydrogen or methyl.
R₄ is preferably hydrogen, methyl or together with R₃ is cyclopropyl.
R₅ is preferably trifluoromethyl, cyano, methyl or halogen and, within this class q, is preferably an integer from 1 to 2.
R₆ is preferably hydrogen or (CH₂)ᵣR₇ in which r is 1 or 2 and R₇ is hydrogen, cyclopropyl, C(O)N(C ₁₋₄ alkyl)₂, C(O)NH(C ₁₋₄ alkyl) or C ₁₋₄ alkoxy.

A preferred class of compounds of the invention is that wherein n is 1..

A further preferred class of compounds of formula (I) is that wherein R is C₁₋₄ alkyl, halogen (i.e chlorine or fluorine), trifluoromethyl or cyano; R₁ is hydrogen, methyl, ethyl or halogen (e.g. fluorine), R₂ is a methyl or hydrogen, R₃ and R₄ are independently hydrogen or methyl, R₅ is trifluoromethyl, cyano, methyl, chlorine, bromine or fluorine, R₆ hydrogen, methyl, ethyl methylcyclopropyl (CH₂)₂OCH₃ or CH₂C(O)N(CH₃)₂, p is 0 or an integer from 1 to 2, n is 1 or 2, q is 1 or 2.

A further preferred class of compounds of formula (I) is that wherein R is C₁₋₄ alkyl and/or halogen (i.e chlorine or fluorine), R₁ is hydrogen, methyl, ethyl or halogen (e.g. fluorine), R₂ is a methyl or hydrogen, R₃ and R₄ are independently hydrogen or methyl, R₅ is trifluoromethyl, cyano, methyl, chlorine, bromine or fluorine, R₆ hydrogen, methyl, ethyl methylcyclopropyl or CH₂C(O)N(CH₃), p is 0 or an integer from 1 to 2, n is 1 or 2, q is 1 or 2.

Preferred compounds according to the invention are:
N-(3,5-Dichlorobenzyl)-2-[4-(4-fluorophenyl)-piperidin-4-yl]-N-methyl-acetamide;
N-(3,5-Dichlorobenzyl)-2-[3-fluoro-4-(4-fluorophenyl)-piperidin-4-yl]-N-methyl-acetamide ;
N-(3,5-Bis-trifluoromethyl)-benzyl-2-[(4-fluoro-2-methyl-phenyl)-piperidin-4-yl]-N-methylacetamide;
N-(3,5-Dichlorobenzyl)-2-[4-(4-fluoro-2-methyl-phenyl)-piperidin-4-yl]-N-methyl-acetamide;
N-(3,5-Bis-trifluoromethyl-benzyl)-2-[4-(4-fluorophenyl)-azepin-4-yl]-N-methyl-acetamide;
N-(3,5-Bis-trifluoromethyl-benzyl)-2-[4-(4-fluoro-2-methyl-phenyl)-azepin-4-yl]-N-methylacetamide;
N-(3,5-Dichlorobenzyl)-2-[4-(4-fluoro-2-methyl-phenyl)-azepin-4-yl]-N-methyl-acetamide;
N-(3,5-Bis-trifluoromethyl-benzyl)-2-[3-fluoro-4-(4-fluoro-2-methyl-phenyl)-azepin-4-yl]-N-methyl-acetamide;
N-(3,5-Dichlorobenzyl)-2-[3-fluoro-4-(4-fluoro-2-methyl-phenyl)-azepin-4-yl]-N-methylacetamide;
N-(3,5-Dichlorobenzyl)-2-[3-fluoro4-(4-fluoro-2-methyl-phenyl)-azepin4-yl]-N-methylacetamide;
N-(3,5-Bis-trifluoromethyl-benzyl)-2-[3-fluoro-4-(4-fluoro-2-methyl-phenyl)-azepin-4-yl]-N-methyl-acetamide;
N-(3,5-Dibromobenzyl)-2-[4-(4-fluorophenyl)-piperidin-4-yl]-N-methyl-acetamide;
N-(3,5-Dibromo-benzyl)-2-[4-(4-fluoro-phenyl)-1-methyl-piperidin-4-yl]-N-methylacetamide;
N-(3,5-Dibromobenzyl)-2-(4-phenyl-piperidin-4-yl)-N-methyl-acetamide;
N-(3,5-Dibromo-benzyl)-2-(4-phenyl-1-methyl-piperidin-4-yl)-N-methyl-acetamide;
N-[1-(3,5-Dichloro-phenyl)-ethyl]-2-[4-(4-fluoro-phenyl)-piperidin-4-yl]-N-methyl-acetamide;
N-[1-(3,5-Dichloro-phenyl)-ethyl]-2-[4-(4-fluoro-phenyl)-1-methyl-piperidin-4-yl]-N-methylacetamide;
N-[1-(3,5-Bis-trifluoromethyl-phenyl)-ethyl]-2-[4-(4-fluoro-phenyl)-piperidin-4-yl]-N-methylacetamide;
N-[1-(3,5-Bis-trifluoromethyl-phenyl)-ethyl]-2-[4-(4-fluoro-phenyl)-1-methyl-piperidin-4-yl]-N-methyl-acetamide;
N-[1-(3,5-Dibromo-phenyl)-ethyl]-2-[4-(4-fluoro-phenyl)-piperidin-4-yl]-N-methylacetamide;
N-[1-(3,5-Dibromo-phenyl)-ethyl]-2-[4-(4-fluoro-phenyl)-1-methyl-piperidin-4-yl]-N-methylacetamide;
N-[1-(3,5-Bis-trifluoromethyl-phenyl)-ethyl]-2-(4-phenyl-piperidin-4-yl)-N-methylacetamide;
N-[1-(3,5- Bis-trifluoromethyl-phenyl)-ethyl]-2-(4-phenyl-1-methyl-piperidin-4-yl)-N-methylacetamide;
N-[1-(3,5-Dibromo-phenyl)-ethyl]-2-(4-phenyl-piperidin-4-yl)-N-methyl-acetamide;
N-[1-(3,5-Dibromo-phenyl)-ethyl]-2-(4-phenyl-1-methyl-piperidin-4-yl)-N-methylacetamide;
N-[1-(3,5-Dibromo-phenyl)-ethyl]-2-[4-(4-fluoro-phenyl)-piperidin-4-yl]-N-methylacetamide;
N-[1-(3,5-Dibromo-phenyl)-ethyl]-2-[4-(4-fluoro-phenyl)-1-methyl-piperidin-4-yl]-N-methylacetamide;
*N*-[(3,5-Dichlorophenyl)methyl]-2-{4-(4-fluoro-2-methylphenyl)-1-[2-(methyloxy)ethyl]-4-piperidinyl}-*N*-methylacetamide;
*N*-{1-[3,5-Bis(trifluoromethyl)phenyl]ethyl}-2-[4-(4-fluoro-2-methylphenyl)-4-piperidinyl]-*N-*methylacetamide;
*N*-[(3,5-Dibromophenyl)methyl]-2-[4-(4-fluoro-2-methylphenyl)-4-piperidinyl]-*N-*methylacetamide;
*N*-{[3,5-Bis(trifluoromethyl)phenyl]methyl}-2-[4-(4-fluoro-2-methylphenyl)-1-methyl-4-piperidinyl]-*N*-methylacetamide;
*N*-[(3,5-Dichlorophenyl)methyl]-2-[4-(4-fluoro-2-methylphenyl)-1-methyl-4-piperidinyl]-*N-*methylacetamide;
*N*-{[3,5-Bis(trifluoromethyl)phenyl]methyl}-2-[4-(4-fluoro-2-methylphenyl)-4-piperidinyl]acetamide;
*N*-{[3,5-Bis(trifluoromethyl)phenyl]methyl}-2-[4-(4-fluoro-2-methylphenyl)-1-methyl-4-piperidinyl]acetamide;
*N*-[(3,5-Dibromophenyl)methyl]-2-[4-(4-fluoro-2-methylphenyl)-1-methyl-4-piperidinyl]-*N-*methylacetamide;
*N*-[(3,5-Dibromophenyl)methyl]-*N*-methyl-2-[4-(2-methylphenyl)-4-piperidinyl]acetamide;
*N*-[(3,5-Dibromophenyl)methyl]-*N*-methyl-2-[1-methyl-4-(2-methylphenyl)-4-piperidinyl]acetamide;
*N*-[(3,5-Dichlorophenyl)methyl]-2-[4-(4-fluorophenyl)-1-methyl-4-piperidinyl]-*N-*methylacetamide;
*N*-{[3,5-Bis(trifluoromethyl)phenyl]methyl}-2-[4-(4-fluorophenyl)-1-methyl-4-piperidinyl]-*N-*methylacetamide;
*N*-[1-(3,5-Dibromophenyl)-1-methylethyl]-2-[4-(4-fluorophenyl)-4-piperidinyl]-*N-*methylacetamide;
*N*-[1-(3,5-Dibromophenyl)-1-methylethyl]-2-[4-(4-fluorophenyl)-1-methyl-4-piperidinyl]-*N-*methylacetamide;
*N*-[1-(3,5-Bis-trifluoromethyl-phenyl)-ethyl]-2-[4-(4-fluoro-phenyl)-piperidin-4-yl]-N-methylacetamide ;
N-[1-(3,5-Bis-trifluoromethyl-phenyl)-ethyl]-2-[4-(4-fluoro-phenyl)-1-methyl-piperidin-4-yl]-N-methyl-acetamide;
2-[1-(Cyclopropylmethyl)-4-(4-fluorophenyl)-4-piperidinyl]-*N*-[(3,5-dibromophenyl)methyl]-*N*-methylacetamide;
2-[4-{2-[(3,5-Dibromophenyl)methyl](methyl)aminol-2-oxoethyl}-4-(4-fluorophenyl)-1-piperidinyl]-*N,N*-dimethylacetamide;
*N*-[(3,5-Dibromophenyl)methyl]-2-[1-ethyl-4-(4-fluorophenyl)-4-piperidinyl]-*N-*methylacetamide;
*N*-{1-[3,5-Bis(trifluoromethyl)phenyl]ethyl}-2-[4-(4-fluorophenyl)hexahydro-1*H*-azepin-4-yl]-*N*-methylacetamide;
*N*-{1-[3,5-Bis(trifluoromethyl)phenyl]ethyl}-2-[4-(4-fluorophenyl)-1-methylhexahydro-1*H-*azepin-4-yl]-*N*-methylacetamide,
*N*-[(3,5-Dibromophenyl)methyl]-2-[4-(4-fluorophenyl)hexahydro-1*H*-azepin-4-yl]-*N-*methylacetamide;
*N*-[(3,5-Dibromophenyl)methyl]-2-[4-(4-fluorophenyl)-1-methylhexahydro-1*H*-azepin-4-yl]-*N*-methylacetamide;
*N*-[(3-Bromo-5-cyanophenyl)methyl]-2-[4-(4-fluorophenyl)-4-piperidinyl]-N-methylacetamide;
*N*-[(3-Bromo-5-cyanophenyl)methyl]-2-[4-(4-fluorophenyl)-1-methyl-4-piperidinyl]-*N-*methylacetamide;
*N*-[(3,5-Dibromophenyl)methyl]-*N*-methyl-2-{4-[3-(trifluoromethyl)phenyl]-4-piperidinyl}acetamide;
*N*-[(3,5-Dibromophenyl)methyl]-*N*-methyl-2-{1-methyl-4-[3-(trifluoromethyl)phenyl]-4-piperidinyl}acetamide;
*N*-[(3,5-Dibromophenyl)methyl]-2-[4-(3,4-dimethylphenyl)-4-piperidinyl]-*N-*methylacetamide;
*N*-[1-(3,5-Dibromophenyl)ethyl]-2-[4-(3-fluorophenyl)-1-methyl-4-piperidinyl]-*N-*methylacetamide;
*N*-[1-(3,5-Dibromophenyl)ethyl]-2-[4-(4-fluoro-3-methylphenyl)-4-piperidinyl]-*N-*methylacetamide;
*N*-[1-(3,5-Dibromophenyl)ethyl]-2-[4-(4-fluoro-3-methylphenyl)-1-methyl-4-piperidinyl]-*N-*methylacetamide;
2-[4-(3-Chlorophenyl)-4-piperidinyl]-*N*-[1-(3,5-dibromophenyl)ethyl]-*N*-methylacetamide;
*N*-[1-(3,5-Dibromophenyl)ethyl]-2-[4-(3,4-difluorophenyl)-4-piperidinyl]-*N-*methylacetamide;
*N*-[1-(3,5-Dibromophenyl)ethyl]-2-[4-(3,4-difluorophenyl)-1-methyl-4-piperidinyl]-*N-*methylacetamide;
*N*-[1-(3,5-Dibromophenyl)ethyl]-2-[4-(3-fluorophenyl)-4-piperidinyl]-*N*-methylacetamide ;
*N*-[1-(3,5-Dibromophenyl)ethyl]-2-[4-(3-fluorophenyl)-1-methyl-4-piperidinyl]-*N-*methylacetamide;
*N*-[1-(3,5-Dibromophenyl)ethyl]-2-[4-(4-fluoro-3-methylphenyl)-4-piperidinyl]-*N-*methylacetamide;
*N*-[1-(3,5-Dibromophenyl)ethyl]-2-[4-(4-fluoro-3-methylphenyl)-1-methyl-4-piperidinyl]-*N-*methylacetamide;
2-[4-(3-Chlorophenyl)-4-piperidinyl]-*N*-[1-(3,5-dibromophenyl)ethyl]-*N*-methylacetamide;
2-[4-(3-Chlorophenyl)-1-methyl-4-piperidinyl]-*N*-[1-(3,5-dibromophenyl)ethyl]-*N-*methylacetamide;
2-[4-(3-Chlorophenyl)-4-piperidinyl]-*N*-[1-(3,5-dichlorophenyl)ethyl]-*N*-methylacetamide;
2-[4-(3-Chlorophenyl)-1-methyl-4-piperidinyl]-*N*-[1-(3,5-dichlorophenyl)ethyl]-*N-*methylacetamide;
2-[4-(3-Chlorophenyl)-4-piperidinyl]-*N*-[(3,5-dibromophenyl)methyl]-*N*-methylacetamide;
*N*-[1-(3,5-Dichlorophenyl)ethyl]-2-[4-(4-fluoro-3-methylphenyl)-4-piperidinyl]-*N-*methylacetamide;
*N*-[(3,5-Dibromophenyl)methyl]-2-[4-(4-fluoro-3-methylphenyl)-4-piperidinyl]-*N-*methylacetamide;
*N*-[(3,5-Dibromophenyl)methyl]-2-[4-(4-fluoro-3-methylphenyl)-1-methyl-4-piperidinyl]-*N-*methylacetamide ;
*N*-[(3,5-Dibromophenyl)methyl]-2-[4-(3-fluorophenyl)-4-piperidinyl]-*N*-methylacetamide;
*N*-[(3,5-Dibromophenyl)methyl]-2-[4-(3-fluorophenyl)-1-methyl-4-piperidinyl]-*N-*methylacetamide;
*N*-[(3,5-Dibromophenyl)methyl]-2-[4-(3,4-difluorophenyl)-4-piperidinyl]-*N-*methylacetamide;
*N*-[(3,5-Dibromophenyl)methyl]-2-[4-(3,4-difluorophenyl)-1-methyl-4-piperidinyl]-*N-*methylacetamide;
2-[4-(4-Cyanophenyl)-4-piperidinyl]-*N*-[1-(3,5-dibromophenyl)ethyl]-*N*-methylacetamide;
2-[4-(4-Cyanophenyl)-1-methyl-4-piperidinyl]-*N*-[1-(3,5-dibromophenyl)ethyl]-*N-*methylacetamide; diastereoisomers or enantiomers thereof and pharmaceutically acceptable salts thereof (e.g. hydrochloride).

Particularly preferred compounds of the invention are
[N-(3,5-Dibromo-benzyl)-2-[4-(4-fluoro-phenyl)-1-methyl-piperidin-4-yl]-N-methylacetamide;
N-[1-(*S*)-1-(3,5-Bis-trifluoromethyl-phenyl)-ethyl]-2-[4-(4-fluoro-phenyl)-1-methyl-piperidin-4-yl]-N-methyl-acetamide;
N-[1-(3,5-Dibromo-phenyl)-ethyl]-2-[4-(4-fluoro-phenyl)-1-methyl-piperidin-4-yl]-N-methylacetamide (enantiomer 1);
N-[1-(3,5-Dibromo-phenyl)-ethyl]-2-(1-methyl-4-phenyl-piperidin-4-yl)-N-methyl-acetamide (enantiomer 1);
N-[1-(3,5-Dichloro-phenyl)-ethyl]-2-[4-(4-fluoro-phenyl)-1-methyl-piperidin-4-yl]-N-methylacetamide (enantiomer 1);
   and pharmaceutically acceptable salts thereof (e.g. hydrochloride). The compounds of the invention are antagonists of tachykinin receptors, including substance P and other neurokinins, both in vitro and in vivo and are thus of use in the treatment of conditions mediated by tachykinins, including substance P and other neurokinins.

Tachykinins are a family of peptides that share a common carboxyl-terminal sequence (Phe-X-Gly-Leu-Met-NH2). They are actively involved in the physiology of both lower and advanced lifeforms. In mammalian lifeforms the main tachykinins are subtance P (SP), Neurokinin A (NKA) and Neurokinin B (NKB) which act as neurotransmitters and neuromodulators. Mammalian tachykinins may contribute to the pathophysiology of a number of human diseases.

Three types of tachykinins receptors have been identified, namely NK1(SP-preferring), NK2 (NKA-preferring) and NK3 (NKB-preferring) which are widely distributed throughout the central nervous (CNS) and peripheral nervous system.

Particularly, the compounds of the invention are antagonists of the NK1 receptor.

The compounds of the present invention also have activity as selective serotonin reuptake inhibitors (hereinafter referred to as SSRIs) and are thus of use in the treatment of conditions mediated by selective inhibition of the serotonin reuptake transporter protein.

Thus, the compounds of the present invention combine dual activity as tachykinin antagonists, including substance P and other neurokinins and as SSRIs. In particular, the compounds of the invention combine dual activity as NK1 receptor antagonists and as SSRIs.

NK₁-receptor binding affinity has been determined in vitro by measuring the compounds' ability to displace [3H] - substance P (SP) from recombinant human NK₁ receptors expressed in Chinese Hamster Ovary (CHO) cell membranes.

CHO cell membranes were prepared by using a modification of the method described by Beattie D.T. et al. (Br. J. Pharmacol, 116:3149-3157, 1995). Briefly, ligand binding was performed in 0.2 ml of 50 mM HEPES, pH 7.4, containing 3 mM MnCl₂, 0.02% BSA, 0.5 nM [³H]-Substance P (30ö56 Ci/mmol, Amersham), a final membrane concentration of 20 ö30 µg of protein/ml, and the test compounds. The incubation proceeded at room temperature for 40 min and stopped by filtration. Non-specific binding was determined using excess of substance P (1 µM) and represents about 6ö10% of the total binding.

NK₁-receptor binding affinity has been also determined in vitro in a binding Scintillation proximity assay (SPA) by measuring compounds' ability to displace [125I]Tyr8-Substance P (SP) from recombinant human NK₁ receptors expressed in Chinese Hamster Ovary (CHO) cell membrane prepared as described above. Briefly, polystrene Leadseeker WGA-SPA beads (Amersham Biosciences) was mixed with cell membrane in a bead/membrane ratio of 100:1 (w/w) in assay buffer (75 mM Tris pH 7.8, 75 mM NaCl, 4 mM MnCl2, 1 mM EDTA, 0.05% Chaps, 1 mM PMSF). The mixture was placed on ice for 30 minutes to allow the formation of membrane/bead complex before BSA was added to a final concentration of 1%. After another 30 minutes incubation on ice, the bead/membrane complex was washed twice and suspended in assay buffer. [125I]Tyr8-Substance P (2200 Ci/mmol, PerkinElmer) was then added to the bead/membrane complex with a final concentration of 0.4 nM. 30 ul of the resulting mixture was then dispensed to each well of Nalgen NUNC 384-well plate with 1 ul compound pre-dispensed in DMSO. The plates were then sealed and pulse spin at 1100 rpm. After 3 hours incubation at room temperature with shaking, the plates were spin for 2 min at 1100 rpm and measured in Viewlux imager (PerkinElmer) for 5 minutes with a 618-nm filter. Inhibition of [125I]Tyr8-Substance P binding to NK₁-receptor was measured by the reduction of luminescent signal. IC50 of each compound was determined by an 11-point 3x-dilution inhibition curve. pKi was calculated using Kd of [125I]Tyr8-Substance P determined in a separate experiment.

Compounds of the invention were further characterised in a functional assay for the determination of their effect to inhibit the intracellular calcium increase induced by SP in Human-NK₁-CHO cells using FLIPR technology. Briefly, after 30min incubation with the cytoplasmic calcium indicator Fluo-4 AM (2µM), cells were washed and incubated in the absence or presence of three different concentrations of antagonist for 60min, at 37°C in Hank's balanced salts with 20mM Hepes and then non-cumulative concentration-response curves of SP (2pM-300nM) was performed. The potency of the antagonist (pK_{B} value) was calculated from Schild's analysis.

The ability to bind at the serotonin transporter may be determined using one of the following conventional binding or uptake assays.

The binding activity of the compounds of the invention at the human Serotonin Transporter (hSERT) binding affinity has been determined in vitro by the compounds' ability to displace [³H]- Imipramine from human serotonin transporter expressed in Human Embryonic Kidney HEK293 cell membranes (Receptor Biology Inc.). For the binding reaction, 4 nM of [³H]- Imipramine (703 GBq/mmol, Amersham) were incubated with 0.02 mg/ml of cell membrane and the compound to be tested at different concentrations (7 concentration points) in 50 mM Tris HCl, pH 7.5, 120 mM of NaCl and 5 mM KCl. The reaction was performed for 60 min at 4°C and was terminated by through GF/B Unifilter (pre-soaked in 0.5 % PEI) using a Cell Harvester (Packard). Scintillation fluid was added to each filtered spot and radioactivity was determined using a scintillation counter (TopCount (Packard)). Non-specific binding was determined using Imipramine (100µM) and represents about 5% of the total binding. Competition experiments were conducted with duplicate determination for each point.

Msat601 software package was used to elaborate the competition binding data.

IC₅₀ values were converted to Kᵢ values using Cheng-Prusoff equation.

The inhibitory activity of the compounds at the human Serotonin Transporter(hSERT) has been determined in vitro using hSERT-LLCPK cells (LLCPK cells tranfected with the h SERT). The cells have been plated onto 96-well plates (10000 cells/well). After 24 hr, cells have been washed in uptake buffer (Hank's balanced salt solution + 20 mM Hepes) and pre-incubated for 10 min at RT with 50 µl of buffer containing the test compounds. 50 µl of 50 nM [3H] Serotonin (5HT) solution (final concentration: 25 nM [3H] 5HT) have been added and plates have been incubated for 7 min at RT, during which cells take up radiolabelled 5HT. Aspirating the solution and rapidly washing the cells with cold buffer has terminated the uptake.

The amount of radioactive 5HT incorporated in the cells has been then measured by adding the scintillation cocktail directly onto the cells and reading the plate in the Top

Count. The data have been digitally processed to obtain the pIC50 values of the antagonists.

The inhibitory activity of the compounds at the rat Serotonin Transporter(rSERT) has been determined in vitro using rSERT-LLCPK cells (LLCPK cells tranfected with the rat SERT). The cells have been plated onto 96-well plates (60000 cells/well). After 24 hr, cells have been washed in uptake buffer (Hank's balanced salt solution + 20 mM Hepes) and pre-incubated for 10 min at RT with 50 µl of buffer containing the test compounds. 50 µl of 50 nM [3H] Serotonin (5HT) solution (final concentration: 25 nM [3H] 5HT) have been added and plates have been incubated for 7 min at RT, during which cells take up radiolabelled 5HT. Aspirating the solution and rapidly washing the cells with cold buffer has terminated the uptake.

The amount of radioactive 5HT incorporated in the cells has been then measured by adding the scintillation cocktail directly onto the cells and reading the plate in the Top Count. The data have been digitally processed to obtain the pIC50 values of the antagonists. The pKi values have been calculated using the Chen-Prusoff equation.

The action of the compounds of the invention at the NK₁ receptor and/or serotonin transporter may be determined by using conventional animal models. Thus, the ability to bind at the NK₁ receptor and/or serotonin transporter was determined using the guinea pig pup isolation calls model as described by Pettijohn, Psychol. Rep., 1979 and Rupniak et al., Neuropharmacology, 2000.

Compounds of the invention are useful in the treatment of CNS disorders and psychotic disorders, in particular in the treatment or prevention of depressive states and /or in the treatment of anxiety as defined in, but not restricted to, Diagnostic Statistical of Mental Disorder (DSM) IV edition edit by American Psychiatric Association and International Classification Diseases 10th revision ( ICD10).

Thus, for example, depressive states include Major Depressive Disorders (MDD), including bipolar depression, unipolar depression, single or recurrent major depressive episodes, recurrent brief depression, with or without psychotic features, catatonic features, melancholic features including anorexia, weight loss, atypical features, anxious depression, cyclothymic or postpartum onset.

Other mood disorders encompassed within the term major depressive disorders include dysthymic disorders with early or late onset and with or without atypical features, neurotic depression, post-traumatic stress disorders and social phobia; dementia of the Alzheimer's type, with early or late onset, with depressed mood; vascular dementia with depressed mood; mood disorders induced by alcohol, amphetamines, cocaine, hallucinogens, inhalants, opioids, phencyclidine, sedatives, hypnotics, anxiolytics and other substances; schizoaffective disorder of the depressed type; and adjustment disorder with depressed mood. Major depressive disorders may also result from a general medical condition including, but not limited to, myocardial infarction, diabetes, miscarriage or abortion, etc.

The term anxiety includes anxiety disorders, such as panic disorders with or without agoraphobia, agoraphobia, phobias, for example, social phobias or agoraphobia, obsessive-compulsive disorder, stress disorders including post-traumatic stress disorders, generalised anxiety disorders, acute stress disorders and mixed anxiety-depression disorders.

Compounds of the invention are useful as analgesics. In particular, they are useful in the treatment of traumatic pain such as postoperative pain; traumatic avulsion pain such as brachial plexus; chronic pain such as arthritic pain such as occurring in osteo-, rheumatoid or psoriatic arthritis; neuropathic pain such as post-herpetic neuralgia, trigeminal neuralgia, segmental or intercostal neuralgia, fibromyalgia, causalgia, peripheral neuropathy, diabetic neuropathy, chemotherapy-induced neuropathy, AIDS related neuropathy, occipital neuralgia, geniculate neuralgia, glossopharyngeal neuralgia, reflex sympathetic dystrophy, phantom limb pain; various forms of headache such as migraine, acute or chronic tension headache, temporomandibular pain, maxillary sinus pain, cluster headache; odontalgia; cancer pain; pain of visceral origin; gastrointestinal pain; nerve entrapment pain; sport's injury pain; dysmennorrhoea; menstrual pain; meningitis; arachnoiditis; musculoskeletal pain; low back pain e.g. spinal stenosis; prolapsed disc; sciatica; angina; ankylosing spondyolitis; gout; bums; scar pain; itch and thalamic pain such as post stroke thalamic pain.

Compounds of the invention are also useful in the treatment of sleep disorders including dysomnia, insomnia, sleep apnea, narcolepsy, and circadian ritmic disorders.

Compounds of the invention are also useful in the treatment or prevention of the cognitive disorders. Cognitive disorders include dementia, amnestic disorders and cognitive disorders not otherwise specified.

Furthermore, compounds of the invention are also useful as memory and/or cognition enhancers in healthy humans with no cognitive and/or memory deficit.

Compounds of the invention are also useful in the treatment of tolerance to and dependence on a number of substances. For example, they are useful in the treatment of dependence on nicotine, alcohol, caffeine, phencyclidine (phencyclidine like compounds) or in the treatment of tolerance to and dependence on opiates (e.g. cannabis, heroin, morphine) or benzodiazepines; in the treatment of addiction to cocaine, sedative ipnotic, amphetamine or amphetamine-related drugs (e.g. dextroamphetamine, methylamphetamine) or a combination thereof.

Compounds of the invention are also useful as anti-inflammatory agents. In particular, they are useful in the treatment of inflammation in asthma, influenza, chronic bronchitis and rheumatoid arthritis; in the treatment of inflammatory diseases of the gastrointestinal tract such as Crohn's disease, ulcerative colitis, inflammatory bowel disease and non-steroidal anti-inflammatory drug induced damage; inflammatory diseases of the skin such as herpes and eczema; inflammatory diseases of the bladder such as cystitis and urge incontinence; and eye and dental inflammation.

Compounds of the invention are also useful in the treatment of allergic disorders, in particular allergic disorders of the skin such as urticaria, and allergic disorders of the airways such as rhinitis.

Compounds of the invention are also useful in the treatment or prevention of schizophrenic disorders including paranoid schizophrenia, disorganised schizophrenia, catatonic schizophrenia, undifferentiated schizophrenia, residual schizophrenia.

Compounds of the invention are also useful in the treatment of emesis, i.e. nausea, retching and vomiting. Emesis includes acute emesis, delayed emesis and anticipatory emesis. The compounds of the invention are useful in the treatment of emesis however induced. For example, emesis may be induced by drugs such as cancer chemotherapeutic agents such as alkylating agents, e.g. cyclophosphamide, carmustine, lomustine and chlorambucil; cytotoxic antibiotics, e.g. dactinomycin, doxorubicin, mitomycin-C and bleomycin; anti-metabolites, e.g. cytarabine, methotrexate and 5-fluorouracil; vinca alkaloids, e.g. etoposide, vinblastine and vincristine; and others such as cisplatin, dacarbazine, procarbazine and hydroxyurea; and combinations thereof; radiation sickness; radiation therapy, e.g. irradiation of the thorax or abdomen, such as in the treatment of cancer; poisons; toxins such as toxins caused by metabolic disorders or by infection, e.g. gastritis, or released during bacterial or viral gastrointestinal infection; pregnancy; vestibular disorders, such as motion sickness, vertigo, dizziness and

Meniere's disease; post-operative sickness; gastrointestinal obstruction; reduced gastrointestinal motility; visceral pain, e.g. myocardial infarction or peritonitis; migraine; increased intercranial pressure; decreased intercranial pressure (e.g. altitude sickness); opioid analgesics, such as morphine; and gastro-oesophageal reflux disease (GERD) such as erosive GERD and symptomatic GERD or non erosive GERD, acid indigestion, over-indulgence of food or drink, acid stomach, sour stomach, waterbrash/regurgitation, heartburn, such as episodic heartburn, nocturnal heartburn, and meal-induced heartburn, dyspepsia and functional dyspepsia.

Compounds of the invention are also useful in the treatment of gastrointestinal disorders such as irritable bowel syndrome, gastro-oesophageal reflux disease (GERD) such as erosive GERD and symptomatic GERD or non erosive GERD, acid indigestion, over-indulgence of food or drink, acid stomach, sour stomach, waterbrash/regurgitation, heartburn, such as episodic heartburn, nocturnal heartburn, and meal-induced heartburn, dyspepsia and functional dyspepsia (such as ulcer-like dyspepsia, dysmotility-like dyspepsia and unspecified dyspepsia) chronic constipation; skin disorders such as psoriasis, pruritis and sunburn; vasospastic diseases such as angina, vascular headache and Reynaud's disease; cerebral ischeamia such as cerebral vasospasm following subarachnoid haemorrhage; fibrosing and collagen diseases such as scleroderma and eosinophilic fascioliasis; disorders related to immune enhancement or suppression such as systemic lupus erythematosus and rheumatic diseases such as fibrositis; and cough.

The compounds of the invention are also useful in premenstrual dysphoric disorder (PMDD), in chronic fatigue syndrome and Multiple sclerosis.

Compounds of the invention have been found to exhibit anxiolytic and antidepressant activity in conventional tests. For example, in Guinea pig pups separation-induced vocalisations (Molewijk et al., 1996).

The invention therefore provides a compound of formula (I) or a pharmaceutically acceptable salt or solvate thereof for use in therapy, in particular in human medicine.

There is also provided as a further aspect of the invention the use of a compound of formula (I) or a pharmaceutically acceptable salt or solvate thereof in the preparation of a medicament for use in the treatment of conditions mediated by tachykinins (including substance P and other neurokinins) and/or by selective inhibition of serotonin reuptake.

Accordingly, the invention also provides a pharmaceutical composition which comprises at least one compound of formula (I) or a pharmaceutically acceptable salt thereof and formulated for administration by any convenient route. Such compositions are preferably in a form adapted for use in medicine, in particular human medicine, and can conveniently be formulated in a conventional manner using one or more pharmaceutically acceptable carriers or excipients.

Thus, compounds of formula (I) may be formulated for oral, buccal, parenteral, topical (including ophthalmic and nasal), depot or rectal administration or in a form suitable for administration by inhalation or insufflation (either through the mouth or nose).

For oral administration, the pharmaceutical compositions may take the form of, for example, tablets or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium starch glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, cellulose derivatives or hydrogenated edible fats); emulsifying agents (e.g. lecithin or acacia); non-aqueous vehicles (e.g. almond oil, oily esters, ethyl alcohol or fractionated vegetable oils); and preservatives (e.g. methyl or propyl-p-hydroxybenzoates or sorbic acid). The preparations may also contain buffer salts, flavouring, colouring and sweetening agents as appropriate.

Preparations for oral administration may be suitably formulated to give controlled release of the active compound.

For buccal administration the composition may take the form of tablets or formulated in conventional manner.

The compounds of the invention may be formulated for parenteral administration by bolus injection or continuous infusion. Formulations for injection may be presented in unit dosage form e.g. in ampoules or in multi-dose containers, with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use.

The compounds of the invention may be formulated for topical administration in the form of ointments, creams, gels, lotions, pessaries, aerosols or drops (e.g. eye, ear or nose drops). Ointments and creams may, for example, be formulated with an aqueous or oily base with the addition of suitable thickening and/or gelling agents. Ointments for administration to the eye may be manufactured in a sterile manner using sterilised components.

Lotions may be formulated with an aqueous or oily base and will in general also contain one or more emulsifying agents, stabilising agents, dispersing agents, suspending agents, thickening agents, or colouring agents. Drops may be formulated with an aqueous or non-aqueous base also comprising one or more dispersing agents, stabilising agents, solubilising agents or suspending agents. They may also contain a preservative.

The compounds of the invention may also be formulated in rectal compositions such as suppositories or retention enemas, e.g. containing conventional suppository bases such as cocoa butter or other glycerides.

The compounds of the invention may also be formulated as depot preparations. Such long acting formulations may be administered by implantation (for example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

For intranasal administration, the compounds of the invention may be formulated as solutions for administration via a suitable metered or unitary dose device or alternatively as a powder mix with a suitable carrier for administration using a suitable delivery device.

A proposed dose of the compounds of the invention is 1 to about 1000mg per day. It will be appreciated that it may be necessary to make routine variations to the dosage, depending on the age and condition of the patient and the precise dosage will be ultimately at the discretion of the attendant physician or veterinarian. The dosage will also depend on the route of administration and the particular compound selected.

Thus, for parenteral administration a daily dose will typically be in the range of 1 to about 100 mg, preferably 1 to 80 mg per day. For oral administration a daily dose will typically be within the range 1 to 300 mg e.g. 1 to 100 mg.

Compounds of formula (I), and salts and solvates thereof, may be prepared by the general methods outlined hereinafter. In the following description, the groups R, R₁ R₂, R₃, R₄, R₅, R₆, R₇, R₈, R₉, m, n, p, q, r and t have the meaning as previously defined for compounds of formula (I) unless otherwise stated.

Compounds of formula (I) may be prepared by reaction of an activated derivative of the carboxylic acid (II), wherein R₆ is a nitrogen protecting group or (CH2)rR₇, with amine (III) 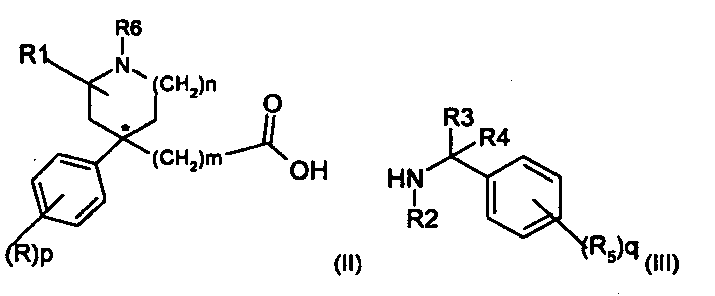 wherein R₂ is hydrogen, C₁₋₄ alkyl or a nitrogen protecting group, followed where necessary by removal of any nitrogen protecting group.

Suitable activated derivatives of the carboxyl group include the acyl halide, mixed anhydride, activated ester such as thioester or the derivative formed between the carboxylic acid group and a coupling agent such as that used in peptide chemistry, for example carbonyl diimidazole or dicyclohexylcarbodiimide.

The reaction is preferably carried out in an aprotic solvent such as hydrocarbon, halohydrocarbon such as dichloromethane or an ether such as tetrahydrofuran.

The activated derivatives of the carboxylic acid (II) may be prepared by conventional means. A particular suitable activated derivative for use in this reaction is O-(Benzotriazol-1-yl) -N,N,N',N'-tetramethyluronium tetrafluoroborate or O-(7-Azabenzotriazol-1-yl)-N,N,N'N'-tetramethyluroniumhexafluorophosphate.
The reaction is suitably carried out in a solvent such as NN-dimethylformamide or dichloromethane.

Compounds of formula(I), wherein R₂ is C₁₋₄ alkyl may be prepared by reaction of a compound of formula(Ia), in which R₂ is hydrogen, with (C ₁₋₄ alkyl)L wherein L is a suitable leaving group selected from iodine, bromine.

The reaction is conveniently carried out in a solvent such as NN-dimethylformamide or tetrahydrofuran.

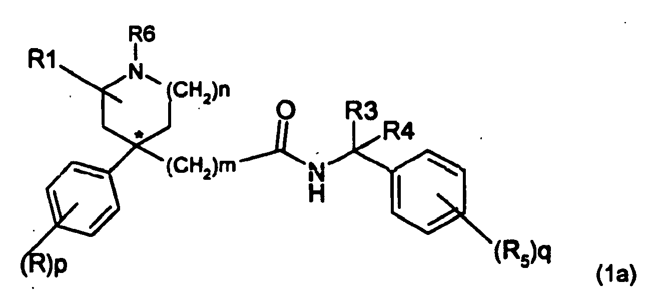

Compounds of formula (II), may be prepared by reaction of a derivative (IV), wherein R₁₀ is (CH₂)ₘ₋₁CH(CN)CO₂R₁₁ in which R₁₁ is a suitable carboxyl protecting group, with
an acid such as for example concentrated sulfuric acid. 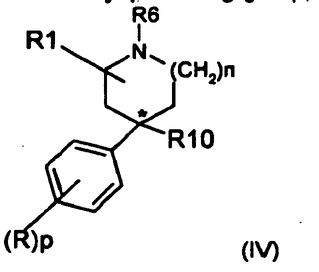 acid, followed (if it is still necessary) by removal of the carboxyl protecting group R₁₁.
The reaction is conveniently carried out in a solvent such as acetic acid and heating the reaction mixture up to 150°.

Alternatively compounds of formula(II),, may be prepared by reaction of a derivative (IV) wherein R₁₀ is the group (XI) 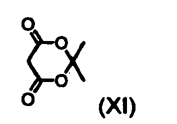 in 3-pentanone and water by heating the reaction mixture to reflux. Alternatively, the reaction can be carried out in the presence of an acid such as for example hydrochloric acid and a solvent such as tetrahydrofuran by heating the reaction mixture to reflux..

Compounds of formula (IV), wherein R₁₀ is (CH₂)ₘ₋₁CH(CN)CO₂R₁₁, in which R₁₁ is a suitable carboxyl protecting group, may be prepared by reaction of a compound of formula (VI) with a compound of formula (VII) , wherein L is a halogen group (i.e bromine).

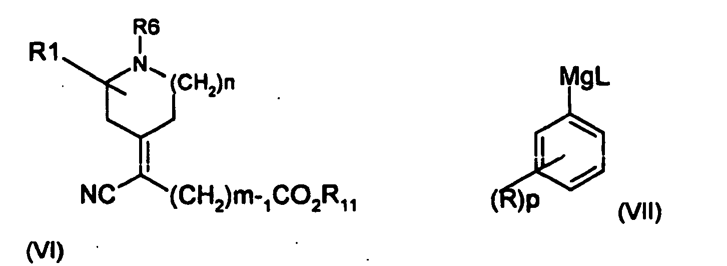

The reaction conveniently takes place in an aprotic solvent such as a hydrocarbon (e.g toluene), ethers (e.g tetrahydrofuran) and at a temperature within the range 0-25°C, optionally in the presence of Cupper(I) salts such as for example Cupper lodide.

Suitable carboxyl protecting groups R₁₁ for use in the above reactions include alkyl, such as methyl or ethyl, trichloroalkyl, trialkylsilylalkyl, or arylmethyl groups such as benzyl, nitrobenzyl or trityl.

Compounds of formula (IV), wherein R₁₀ is the group (XI) may be prepared by reaction of a compound of formula (VIII) 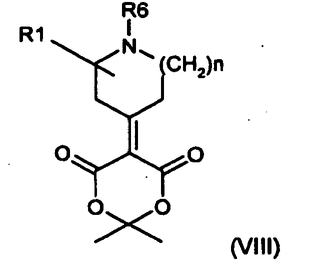 with a compound of formula (VII) , wherein L is a halogen group (i.e bromine). or a compound of formula(VIIa) in which W is an alkali metal base such as for example litium or magnesium.

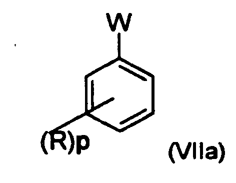

The reaction conveniently takes place in an aprotic solvent such as a hydrocarbon (e.g toluene), ethers (e.g tetrahydrofuran) and at a temperature within the range -80-25°C, optionally in the presence of Cupper(I) salts such as for example Cupper Iodide.

Compounds of formula (VI) may be prepared by reaction of a compound of formula (IX) with a cyano derivative (X) wherein R₁₁ has the meaning defined above.

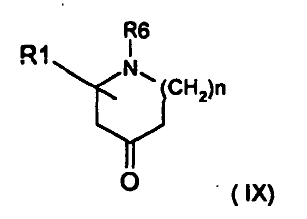

CN(CH₂)mCO₂R₁₁ (X)

Compounds of formula (VIII) may be prepared by reaction of a compound of formula (IX) with the derivative (XI).

Compounds of formulae (V) and (IX) may be prepared with analogous methods to those used for known compounds. Thus, compounds of formula (V) may be prepared according to the procedure described in Cammack et al., Heterocyclic 23,73 (1986) and compounds of formula (IX) may be prepared according to the procedure described in WO 2001/000206.

When R₆ and/or R₂ are a nitrogen protecting group, examples of suitable groups include alkoxycarbonyl e.g. t-butoxycarbonyl, benryloxycarbonyl, arylsulphonyl e.g. phenysulphonyl or 2-trimethylsilylethoxymethyl.

Protection and deprotection may be effected using conventional techniques such as those described in "Protective Groups in Organic Synthesis 2nd Ed." by T.W. Greene and P. G. M. Wuts (John Wiley and Sons. 1991) and as described in the examples hereinafter.

When a specific enantiomer of a compound of general formula (I) is required, this may be obtained for example by resolution of a corresponding enantiomeric mixture of a compound of formula (I) using conventional methods.

Thus, for example, specific enantiomers of the compounds of formula (I) may be obtained from the corresponding enantiomeric mixture of a compound of formula (I) using chiral HPLC procedure.

Alternatively, enantiomers of a compound of general formula (I) may be synthesised from the appropriate optically active intermediates using any of the general processes described herein.

Thus, in one embodiment of the invention the enantiomers of the compound of formula (I) may be prepared by reaction of a chiral amine (III) using any of the processes described above for preparing compounds of formula (I) from amine (III).

The chiral amine (III) may be prepared from the corresponding racemic amine (III) using any conventional procedures such as salt formation with a suitable optically active acid such as for example di-p-toluoyl-D-tartaric acid or di-p-toluoyl-L-tartaric acid, or using chiral HPLC procedure.

Where it is desired to isolate a compound of formula (I) as a salt, for example a pharmaceutically acceptable salt, this may be achieved by reacting a compound of formula (I) in the form of the free base with an appropriate amount of suitable acid and in a suitable solvent such as an alcohol (e.g. ethanol or methanol), an ester (e.g. ethyl acetate) or an ether (e.g. diethyl ether, *tert*-butylmethyl ether or tetrahydrofuran).

### In the Intermediates and Examples unless otherwise stated:

Melting points (m.p.) were determined on a Buchi m.p. apparatus and are uncorrected. R.T. or r.t. refer to room temperature.

Infrared spectra (IR) were measured in chloroform or nujol solutions on a FT-IR instrument. Proton Magnetic Resonance (NMR) spectra were recorded on Varian instruments at 300, 400 or 500 MHz, on Bruker instrument at 300 MHz, chemical shifts are reported in ppm (δ) using the residual solvent line as internal standard. Splitting patterns are designed as s, singlet; d, double; t, triple; q, quartet; m, multiplet; b, broad. The NMR spectra were recorded at temperature ranging from 25 to 90°C; when more than one conformer were detected the chemical shifts for the most abundat one is reported. Mass spectra (MS) were taken on a VG Quattro mass spectrometer. In the mass spectra only one peak in the molecular ion cluster is reported. Optical rotations were determined at 20°C with a Jasco DIP360 instrument (I=10 cm, cell volume = 1 mL, λ = 589 nm). Flash silica gel chromatography was carried out over silica gel 230-400 mesh supplied by Merck AG Darmstadt, Germany. T.I.c. refers to thin layer chromatography on 0.25 mm silica gel plates (60F-254 Merck) and visualized with UV light. For phase separations performed by using microfiltration device: phase separation cartridge with polypropylene frit by Whatman and Alltech. For purifications carried out by using SCX: SCX-cartridges (loading 0.75mmol\g) by Varian.

Solutions were dried over anhydrous sodium sulphate.

Methylene chloride was redistilled over calcium hydride and tetrahydrofuran was redistilled over sodium.

The following abbreviations are used in the text: AcOEt = ethyl acetate, CH = cyclohexane, DCM = methylene chloride, DIPEA = N,N-diisopropylethylamine, DMF = N,N'-dimethylformamide, Et2O = diethyl ether, EtOH = ethanol, MeOH = methanol TEA = triethylamine, THF = tetrahydrofuran, TFA = trifluoroacetic acid.
Enantiomer 1 or enantiomer 2 means a compound of the invention or an intermediate thereof as a single enantiomer whose configuration was not determined.

Anti isomer means a compound of the invention or intermediate thereof wherein the group R1 is different from hydrogen and with reference to the plane of paper it is on the opposite site of the phenyl attached to the cyclic amine ring.

### Intermediate 1

### 4-[(1-Cyano-1-ethoxycarbonyl)-methylene]-piperidine-1-carboxylic acid tert-butyl ester

### Method A

A round bottom flask equipped with a Dean Stark apparatus was charged with *tert*-butyl-4-oxo-1-piperidine carboxylate (1.0 g), ethyl cyanoacetate (0.587 mL), ammonium acetate (0.193 g) and acetic acid (0.286 mL) in anhydrous benzene (20 mL). The mixture was heated to reflux overnight, then it was allowed to cool to r.t. and washed with water (20 mL). The aqueous layer was extracted with AcOEt (2 x 10 mL). The combined organic extracts were washed with a 1M sodium hydroxide solution (10 mL), dried and concentrated *in vacuo* to a residue, which was purified by flash chromatography (CH/AcOEt 8:2) to give the title compound (1.5 g) as a yellow oil.

### Method B

Ethyl cyanoacetate (13.9 mL), ammonium acetate (4.64 g) and acetic acid (6.9 mL) were added, under a Nitrogen atmosphere, to a solution of *tert*-butyl-4-oxo-1-piperidine carboxylate (20 g) in anhydrous toluene (200 mL) in a round bottom flask equipped with a Dean Stark apparatus. The mixture was heated to 110°C for 2 hours, then to 85°C overnight and finally to 130°C for 4 hours. The mixture was allowed to cool to r.t. and washed with 1 M sodium hydroxide solution, water and brine. The organic layer was dried and concentrated *in vacuo* to a residue, which was purified by flash chromatography (CH/AcOEt 8:2) to give the title compound (15.54 g) as a yellow oil. T.I.c.: CH/AcOEt 8:2, Rf=0.35 (detection with ninhydrine).
IR (nujol, cm⁻¹): 2229 (C≡N), 1720 and 1694 (C=O).
NMR (CDCl₃): δ (ppm) 4.29 (q, 2H); 3.61 (t, 2H); 3.55 (t, 2H); 3.13 (t, 2H); 2.78 (t, 2H); 1.49 (s, 9H); 1.36 (t, 3H).
MS (ES/+): m/z=295 [M+H]⁺.

### Intermediate 2

### 4-(1-Cyano-1-ethoxycarbonyl-methyl)-4-(4-fluorophenyl)-piperidine-1-carboxylic acid tert-butyl ester

### Method A

A solution of intermediate **1** (1.5 g) in anhydrous toluene (15 mL) was dropped over 30 minutes into a solution of 4-fluorophenyl magnesium bromide (2M in Et2O - 6.1 mL) in anhydrous Et2O (50 mL) previously cooled to 0°C under a Nitrogen atmosphere. The mixture was allowed to warm to r.t. and left at this temperature for 2 days. The mixture was treated with 3N sulfuric acid solution (5 mL), water (30 mL) and AcOEt (10 mL). The layers were separated and the aqueous phase was extracted with further AcOEt (2 x 30 mL). The combined organic layers were washed with a saturated sodium hydrogen carbonate solution (60 mL) and water (60 mL), then dried and concentrated *in vacuo*. The residue was purified by flash chromatography (CH/AcOEt 8:2) to give the title compound (0.448 g) as a yellow oil.

### Method B

A solution of 4-fluorophenyl magnesium bromide (1.0M in THF, 49 mL) was added drop-wise to a mixture of intermediate **1** (8 g) and copper iodide (1.57 g) in anhydrous THF (65 mL) previously cooled to 0°C under a Nitrogen atmosphere. The mixture was stirred under these conditions for 1 hour and then allowed to warm to r.t. and stirred at 23°C for 2 hours. The mixture was cooled to 0°C, treated with 3M hydrochloric acid solution until pH=5 and extracted with AcOEt (3 x 100 mL). The combined organic extracts were washed with a saturated ammonium chloride solution (200 mL), dried and concentrated *in vacuo*. The residue was purified by flash chromatography (CH/AcOEt 85:15) to give the title compound (9.8 g) as a yellow oil.
T.I.c.: CH/AcOEt 6:4, Rf=0.35 (detection with ninhydrine).
NMR (CDCl₃): δ (ppm) 7.31 (dd, 2H); 7.06 (t, 2H); 3.95 (q, 2H); 3.87 (bs, 2H); 3.54 (t, 1H); 2.85 (bt, 2H); 2.53 (dt, 2H); 1.9 (m, 2H); 1.4 (s, 9H); 1.02 (t, 3H).
MS (ES/+): m/z=391 [M+H]⁺.

### Intermediate 3

### 4-Carboxymethyl-(4-fluoro-phenyl)-piperidine-1-carboxylic acid tert-butyl ester

### Method A

A mixture of intermediate **2** (0.448 g) in acetic acid (2 mL), conc. sulfuric acid (1 mL) and water (1 mL) was heated to 140°C overnight. The solution was allowed to cool to r.t. and dropped into a 2.5M sodium hydroxide solution (50 mL). Then, di-tert-butyl-dicarbonate (500 mg) was added and the resulting mixture was stirred at r.t. for 5 hours. It was cooled to 0°C and treated with 6M hydrochloric acid solution until pH=1 and then extracted with AcOEt (20 mL). The organic phase was dried, concentrated *in vacuo* and the residue was purified by flash chromatography (CH/AcOEt 7:3) to give the title compound (210 mg) as a pale yellow foam.

### Method B

A mixture of intermediate **113** (0.045 g) in 3-pentanone (2 mL), and water (1 mL) was heated to 102°C for 1 day. The mixture was allowed to cool to r.t and concentrated *in vacuo.* The residue was purified by preparative HPLC (column: Supelcosil ABZ+Plus 5 µm, 100 x 21.2 mm; mobile phase: **A:** H₂O+0.1% Formic acid; **B:** CH₃CN+0.1% Formic acid; gradient: 5% (B) for 1 min, from 5% (B) to 95% (B) in 10 min, from 95% (B) to 100% (B) in 3 min.; flow rate=20 mL/min; detection: λ=210-400 nm) to give the title compound (0.007 g) as white solid.

T.I.c.: CH/AcOEt 1:1, Rf=0.25 (detection with ninhydrine).

NMR (d₆-DMSO): δ (ppm) 11.78 (bs, 1H); 7.4 (dd, 2H); 7.15 (t, 2H); 3.45 (m, 2H); 3.11 (m, 2H); 2.54 (s, 2H); 2.04 (m, 2H); 1.89 (m, 2H); 1.37 (s, 9H).

MS (ES/-): m/z=336 [M-H]⁻.

### Intermediate 4

### 4-{[(3,5-Dichlorobenzyl)methylcarbamoyl]methyl}-4-(4-fluorophenyl)-piperidine-1-carboxylic acid tert-butyl ester

3,5-Dichlorobenzyl-methylamine (37 mg), 1-(3-dimethylaminopropyl)-3-ethylcarbodiimide hydrochloride (37 mg) and 1-hydroxybenzotriazole (26 mg) were added to a solution of intermediate **3** (60 mg) in anhydrous DMF (2 mL) under a Nitrogen atmosphere. The mixture was stirred at r.t. overnight, then it was diluted with AcOEt (10 mL) and washed with water (10 mL), 0.005M hydrochloric acid solution (15 mL) and a saturated sodium hydrogen carbonate solution (15 mL). The organic layer was dried, concentrated *in vacuo* and the residue was purified by flash chromatography (CH/AcOEt 6:4) to give the title compound (80 mg) as a colourless oil.
T.I.c.: CH/AcOEt 1:1, Rf=0.35 (detection with ninhydrine).
NMR (d₆-DMSO - 70°C): δ (ppm) 7.37 (m, 3H); 7.03 (m, 4H); 4.28 (bs, 2H); 3.48 (m, 2H);
3.12 (m, 2H); 2.71 (bs, 2H); 2.57 (s, 3H); 2.2-1.9 (m, 4H); 1.38 (s, 9H).

### Intermediate 5

### 3-Fluoro-4-oxo-piperidine-1-carboxylic acid tert-butyl ester

Trimethylsilyl trifluoromethanesulfonate (2.17 mL) was added to a solution of *tert*-butyl-4-oxo-1-piperidine carboxylate (2.0 g) and TEA (3.34 mL) in anhydrous DCM (20 mL) previously cooled to -30°C under a Nitrogen atmosphere. The mixture was stirred at this temperature for 1 hour, then it was quickly treated with cold saturated sodium hydrogen carbonate solution (20 mL). The layers were separated and the the organic phase was dried and concentrated *in vacuo* to give 1-(*tert*-butoxycarbonyl)-1,2,3,6-tetrahydro-4-pyridinyl-trifluoromethanesulfonate as a yellow oil, which was dissolved in anhydrous acetonitrile (80 mL) and the resulting solution was treated with Selectfluor (3.89 g). The mixture was stirred at r.t. for 1 hour, then it was concentrated *in vacuo* and the residue was dissolved in AcOEt (50 mL). The organic layer was washed with a saturated sodium hydrogen solution (30 mL), dried and concentrated *in vacuo* to a residue, which was purified by chromatography on neutral Alumina Brochmann type 1 (initially AcOEt then AcOEt/MeOH 95:5) to give the title compound (0.99 g) as a colourless oil.
T.I.c.: CH/AcOEt 1:1, Rf=0.3 (detection with ninhydrine).
NMR (d₆-DMSO): δ (ppm) 5.08 (dd, 1 H); 4.32 (bm, 1H); 4.0 (bm, 1 H); 3.19 (m, 2H); 2.56 (m, 1H); 2.36 (m, 1H); 1.43 (s, 9H).

### Intermediate 6

### 4-(1-Cyano-1-ethoxycarbonyl-methylene)-3-fluoro-piperidine-1-carboxylic acid tert-butyl ester

A round bottom flask equipped with a Dean Stark apparatus was charged with intermediate **5** (0.99 g), ethyl cyanoacetate (0.534 mL), ammonium acetate (0.176 g) and acetic acid (0.261 mL) in anhydrous benzene (20 mL). The mixture was heated to reflux overnight, then it was allowed to cool to r.t. and washed with water (20 mL). The aqueous layer was extracted with AcOEt (2 x 10 mL). The combined organic extracts were washed with a 1 M sodium hydroxide solution, dried and concentrated *in vacuo* to a residue, which was purified by flash chromatography (CH/AcOEt 8:2) to give the title compound (0.696 g - cis/trans mixture) as a yellow oil.
T.I.c.: CH/AcOEt 7:3, Rf=0.45 (detection with ninhydrine).
NMR (CDCl₃): δ (ppm) 6.49 and 5.55 (2bd, 1H); 4.54 (bm, 1H); 4.34 (bm, 3H); 3.72 (m, 1H); 3.12 (bm, 1H); 2.9 (bm, 1H); 2.74 (m, 1H); 1.49 (s, 9H); 1.38 (t, 3H).

### Intermediate 7

### 4-(1-Cyano-1-ethoxycarbonyl-methyl)-3-fluoro-4-(4-fluorophenyl)-piperidine-1-carboxylic acid tert-butyl ester 8 (anti isomer)

A solution of intermediate **6** (0.696 g) in anhydrous toluene (5 mL) was dropped over 30 minutes into a solution of 4-fluorophenyl magnesium bromide (2M in Et2O - 2.67 mL) in anhydrous Et2O (15 mL) previously cooled to 0°C under a Nitrogen atmosphere. The mixture was allowed to warm to r.t. and stirred at this temperature overnight. The mixture was treated with 3N sulfuric acid solution (3 mL), water (10 mL) and AcOEt (25 mL). The layers were separated and the aqueous phase was extracted with further AcOEt (2 x 30 mL). The combined organic layers were washed with a saturated sodium hydrogen carbonate solution (60 mL) and water (60 mL), then dried and concentrated *in vacuo.* The residue was purified by flash chromatography (CH/AcOEt 8:2) to give the title compound (0.383 g) as a yellow oil.
T.I.c.: CH/AcOEt 8:2, Rf=0.27 (detection with ninhydrine).
NMR (CDCl₃): δ (ppm) 7.5-6.8 (m, 4H); 4.8 (bm, 2H); 4.5-3.5 (bm; 3H); 3.85 (s, 1H); 3.2-2.4 (bm, 2H); 2.2 (bm, 2H); 1.4 (s, 9H); 1.05 (2t, 3H).

### Intermediate 8

### 4-Carboxymethyl-3-fluoro-4-(4-fluorophenyl)-piperidine-1-carboxylic acid tert-butyl ester (anti isomer)

A mixture of intermediate **7** (0.383 g) in acetic acid (2 mL), conc. sulfuric acid (1 mL) and water (1 mL) was heated to 140°C overnight. The solution was allowed to cool to r.t. and dropped into a 3M sodium hydroxide solution (50 mL). Then, di-*tert*-butyl-dicarbonate (409 mg) was added and the resulting mixture was stirred at r.t. overnight. It was cooled to 0°C and treated with 3M hydrochloric acid solution until pH=1 and then extracted with AcOEt (20 mL). The organic phase was dried, concentrated *in vacuo* and the residue was purified by flash chromatography (CH/AcOEt 7:3) to give the title compound (96 mg) as a pale yellow oil.
T.I.c.: CH/AcOEt 1:1, Rf=0.5 (detection with ninhydrine).
NMR (d₆-DMSO): δ (ppm) 11.89 (bs, 1H); 7.46 (dd, 2H); 7.16 (t, 2H); 5.36 (dd, 1H); 3.96 (bm, 1H); 3.75 (bm, 1H); 3.2-2.6 (bm, 3H); 2.5 (m, 1H); 2.36 (bd, 1H); 1.93 (bm, 1H); 1.38 (s, 9H).

### Intermediate 9

### 4-{[(3,5-Dichlorobenzyl)-methylcarbamoyl]-methyl}-3-fluoro-4-(4-fluorophenyl)-piperidine-1-carboxylic acid tert-butyl ester (anti isomer)

3,5-Dichlorobenzyl-methylamine (53 mg), 1-(3-dimethylaminopropyl)-3-ethylcarbodiimide hydrochloride (53 mg) and 1-hydroxybenzotriazole (38 mg) were added to a solution of intermediate **8** (90 mg) in anhydrous DMF (4 mL) under a Nitrogen atmosphere. The mixture was stirred at r.t. overnight, then it was diluted with AcOEt (20 mL) and washed with water (15 mL), 0.005M hydrochloric acid solution (15 mL) and a saturated sodium hydrogen carbonate solution (15 mL). The organic layer was dried, concentrated *in vacuo* and the residue was purified by flash chromatography (CH/AcOEt 8:2) to give the title compound (62 mg) as a white foam.
T.I.c.: CH/AcOEt 7:3, Rf=0.30 (detection with ninhydrine).
NMR (d₆-DMSO - 60°C): δ (ppm) 7.42 (m, 3H); 7.08 (m, 2H); 6.98 (bs, 2H); 5.58-5.5 (bd, 1H); 4.28 (m, 2H); 3.99 (m, 1H); 3.75 (bm, 1H); 3.0 (bm, 1H); 2.89 (d, 1 H); 2.79 (bm, 1H); 2.68 (d, 1H); 2.64-2.57 (2bs, 1H); 2.4 (bm, 1H); 2.04-1.98 (2bm, 1 H); 1.38 (s, 9H).

### Intermediate 10

### 3,4-Dimethoxybenzyl chloride

A solution of 3,4-dimethoxy-benzyl alcohol (1.5 g) in anhydrous DCM (5 mL) was dropped into a solution of thionyl chloride (1.3 mL) in anhydrous DCM (6 mL) under a Nitrogen atmosphere. The solution was heated to reflux for 1 hour, then allowed to cool to r.t. and concentrated *in vacuo.* The residue was purified by flash chromatography (CH/AcOEt 6:4) to give the title compound (1.63 g) as a white solid.
T.I.c.: CH/AcOEt 6:4, R=0.63.
NMR (CDCl₃): δ (ppm) 7.94 (dd, 1H); 6.92 (d, 1H); 6.83 (d, 1H); 4.57 (s, 2H); 3.9 (s, 3H); 3.89 (s, 3H).
MS (EI/+): m/z=186 [MS]⁺.

### Intermediate 11

### Bis-(2-hydroxyethyl)-3,4-diemthoxybenzylamine

Intermediate **10** (1.38 g) was added to a mixture of diethanolamine (0.71 mL) and potassium carbonate (1.12 g) in anhydrous toluene (13 mL) previously heated to 60°C under a Nitrogen atmosphere. The resulting mixture was heated to reflux for 1 hour, then allowed to cool to r.t., filtered off and the organic phase was concentrated *in vacuo* to give the title compound (1.87 g) as a yellow oil.
NMR (CDCl₃): δ (ppm) 6.85 (dd, 1H); 6.8 (d, 2H); 3.9 (2s, 6H); 3.6 (s, 2H); 3.55 (dd, 4H); 2.6-2.7 (d, 4H).

### Intermediate 27

### 4-(1-Cyano-1-ethoxycarbonyl-methyl)-4-(4-fluoro-2-methyl-phenyl)-piperidine-1-carboxylic acid tert-butyl ester

A solution of 2-bromo-5-fluorotoluene (10.8 mL) in anhydrous THF (40.5 mL) was dropped into a suspension of magnesium turning (2.2 g) and few crystals of iodine in anhydrous THF (21.5 mL) under a Nitrogen atmosphere. The mixture refluxed for 30 minutes, then it was allowed to cool to r.t. and added drop-wise to a mixture of intermediate **1** (8 g) and copper iodide (1.6 g) in anhydrous THF (66 mL) previously cooled to 0°C under a Nitrogen atmosphere. The mixture was allowed to warm to r.t. and stirred at 23°C for 1.5 hours. The mixture was cooled to 0°C, treated with 3M hydrochloric acid solution until pH=5 and extracted with AcOEt (3 x 100 mL). The combined organic extracts were washed with a saturated ammonium chloride solution (200 mL), dried and concentrated *in vacuo.* The residue was purified by flash chromatography (CH/AcOEt 8:2) to give the title compound (6.71 g) as a white foam.
T.I.c.: CH/AcOEt 8:2, Rf=0.17(detection with ninhydrine).
NMR (CDCl₃): δ (ppm) 7.33 (dd, 1H); 7.07 (t, 1H); 7.0 (td, 1H); 4.48 (s, 1H); 3.99-3.81 (m, 4H); 2.72-2.53 (m, 4H); 2.46 (s, 3H); 1.86-1.74 (m, 2H); 1.36 (s, 9H); 0.94 (t, 3H).
MS (ES/+): m/z=427 [M+Na]⁺.

### Intermediate 28

### 4-Carboxymethyl-(4-fluoro-2-methyl-phenyl)-piperidine-1-carboxylic acid tert-butyl ester

A mixture of intermediate **27** (298 mg) in acetic acid (4.3 mL), conc. sulfuric acid (1.1 mL) and water (1.1 mL) was heated to 140°C for 5 hours. The solution was allowed to cool to r.t. and dropped into a 2.5M sodium hydroxide solution (38 mL). Then, di-*tert*-butyl-dicarbonate (217.6 mg) was added and the resulting mixture was stirred at r.t. over week end. It was cooled to 0°C and treated with 6M hydrochloric acid solution until pH=6-7 and then extracted with AcOEt (3 x 20 mL). The organic phase was washed with brine, dried and concentrated *in vacuo.* The residue was purified by flash chromatography (CH/AcOEt from 8:2 to 6:4) to give the title compound (131 mg) as a white solid.
T.I.c.: CH/AcOEt 6:4, Rf=0.12 (detection with ninhydrine).
NMR (d₆-DMSO): δ (ppm) 11.8 (bs, 1H); 7.28 (dd, 2H); 6.96 (dd, 1H); 6.91 (td, 1H); 3.4 (bm, 2H); 3.22 (bm, 2H); 2.7 (s, 2H); 2.47 (s, 3H); 2.1 (m, 2H); 2.01 (m, 2H); 1.37 (s, 9H).
MS (ES/+): m/z=374 [M+Na]⁺.

### Intermediate 29

### 4-{[(3,5-Dichlorobenzyl)methylcarbamoyl]methyl}-4-(4-fluoro-2-methyl-phenyl)-piperidine-1-carboxylic acid tert-butyl ester

DIPEA (91 µL) and O-(benzotriazol-1-yl)-N,N,N'N'-tetramethyluronium tetrafluoroborate (54.6 mg) were added to a solution of intermediate **28** (46 mg) in anhydrous DMF (3.8 mL) under a Nitrogen atmosphere. After stirring for 30 minutes, 3,5-dichlorobenzyl-methylamine hydrochloride (59.3 mg) was added. The mixture was stirred at r.t. overnight, then it was diluted with AcOEt and washed with 5% sodium hydrogen carbonate solution and brine. The organic layer was dried, concentrated *in vacuo* and the residue was purified by flash chromatography (CH/AcOEt from 9:1 to 6:4) to give the title compound (34 mg) as a colourless oil.
T.I.c.: CH/AcOEt 6:4, Rf=0.16 (detection with ninhydrine). IR (nujol, cm⁻¹): 1699 and 1643 (C=O).
NMR (d₆-DMSO): δ (ppm) 7.42 (dd, 1H); 7.24 (dd, 1H); 6.98 (d, 2H); 6.88 (dd, 1H); 6.83 (td, 1H); 4.26 (s, 2H); 3.45-3.08 (m, 4H); 2.23-1.99 (m, 4H); 2.81 (s, 3H); 2.58 (s, 2H); 2.41 (s, 3H); 1.33 (s, 9H).
MS (ES/+): m/z=545 [M+Na]⁺.

### Intermediate 30

### 4-{[(3,5-Bis-trifluoromethyl-benzyl)methylcarbamoyl]methyl}-4-(4-fluoro-2-methylphenyl)-piperidine-1-carboxylic acid tert-butyl ester

DIPEA (241 µL) and O-(benzotriazol-1-yl)-N,N,N'N'-tetramethyluronium tetrafluoroborate (144.4 mg) were added to a solution of intermediate **28** (122 mg) in anhydrous DMF (10 mL) under a Nitrogen atmosphere. After stirring for 30 minutes, 3,5-(bis-trifluoromethylbenzyl)-methylamine hydrochloride (293.5 mg) was added. The mixture was stirred at r.t. for 2 hours, then it was diluted with AcOEt and washed with 5% sodium hydrogen carbonate solution and brine. The organic layer was dried, concentrated *in vacuo* and the residue was purified by flash chromatography (CH/AcOEt from 9:1 to 1:1) to give the title compound (159 mg) as a colourless oil.
T.I.c.: CH/AcOEt 6:4, Rf=0.1 (detection with ninhydrine).
IR (nujol, cm⁻¹): 1737 and 1638 (C=O).
NMR (d₆-DMSO): δ (ppm) 7.98 (s, 1H); 7.74 (s, 1H); 7.21 (dd, 1H); 6.87 (dd, 1 H); 6.74 (td, 1H); 4.47 (s, 2H); 3.49 (m, 2H); 3.1 (m, 2H); 2.87 (s, 2H); 2.67 (s, 3H); 2.42 (s, 3H); 2.23 (m, 2H); 2.02 (m, 2H); 1.36 (s, 9H).
MS (ES/+): m/z=613 [M+Na]⁺.

### Intermediate 31

### 5-Ethoxycarbonyl-4-oxo-azepine-1-carboxylic acid tert-butyl

Ethyl diazoacetate (6.84 mL) and boron trifluoride diethyl etherate (6.34 mL) were added simultaneously over 1 hour to a suspension of *tert*-butyl-4-oxo-1-piperidine carboxylate (10.0 g) in dry Et2O (60 mL) previously cooled to -25°C under a Nitrogen atmosphere.

The solution obtained was stirred at -25°C for 1 hour, then it was allowed to warm to 0°C and stirred at 0°C for 1 hour. The solution was treated with a saturated potassium carbonate solution (10 mL). The layers were separated; the organic phase was dried and concentrated *in vacuo* to a residue, which was purified by flash chromatography (petroleum/Et2O 6:4) to give the title compound (11.58 g) as a colourless oil.
T.I.c.: petroleum/Et2O 1:1, Rf=0.48 (detection with phosphomolybdic acid).
IR (nujol, cm⁻¹): 1744 and 1695 (C=O). NMR (d₆-DMSO): δ (ppm) 4.08 (q, 3H); 3.82-3.64 (m, 2H); 3.38 (m, 2H); 2.64 (m, 2H); 1.9-1.64 (m, 2H); 1.36 (s, 9H); 1.16 (t, 3H).
MS (ES/+): m/z=286 [MH]⁺.

### Intermediate 32

### Azepin-4-one hydrochloride

A suspension of intermediate **31** (11.58 g) in 3M hydrochloric acid (60 mL) was heated to reflux for 7 hours. The mixture was allowed to cool to r.t. and concentrated *in vacuo.* The residue was triturated with abs. EtOH (10 mL) to give the title compound (4.54 g) as a white solid.
M.p.: 189-191°C.
NMR (d₆-DMSO): δ (ppm) 9.17 (bs, 2H); 3.3 (m, 2H); 3.22 (m, 2H); 2.75 (m, 2H); 2.62 (m,
2H); 1.93 (m, 2H).

### Intermediate 33

### 4-Oxo-azepine-1-carboxylic acid tert-butyl ester

Di *tert*-butyl dicarbonate (0.7 g) was added to a suspension of intermediate **32** (0.4 g) and TEA (0.45 mL) in anhydrous DCM (5 mL) previously cooled to 0°C under a Nitrogen atmosphere. The mixture was stirred for 1 hour at 0°C then it was allowed to reach r.t..

The mixture was treated with a saturated ammonium chloride solution (10 mL) and extracted with further DCM (2 x 10 mL). The combined organic extracts were washed with water, dried and concentrated *in vacuo.* The residue was purified by flash chromatography (CH/AcOEt 7:3) to give the title compound (528 mg) as a colourless oil.

T.I.c.: CH/AcOEt 6:4, Rf=0.3 (detection with ninhydrine).
NMR (d₆-DMSO): δ (ppm) 3.53 (bm, 2H); 3.45 (bm, 2H); 2.58 (m, 2H); 2.52 (m, 2H); 1.61 (bm, 3H); 1.37 (s, 9H).

### Intermediate 34

### 4-(1-Cyano-1-ethoxycarbonyl-methylene)-azepine-1-carboxylic acid tert-butyl ester

A round bottom flask equipped with a Dean Stark apparatus was charged with intermediate **33** (1.87 g), ethyl cyanoacetate (1.03 mL), methyl ammonium acetate (0.339 g) and acetic acid (0.5 mL) in anydrous benzene (15 mL). The mixture was heated to reflux overnight, then it was allowed to cool to r.t. and washed with water (10 mL). The aqueous layer was extracted with AcOEt (2 x 10 mL). The combined organic extracts were washed with a 1M sodium hydroxide solution (10 mL), dried and concentrated *in vacuo* to a residue, which was purified by flash chromatography (CH/AcOEt 8:2) to give the title compound (2.49 g - cis/trans mixture) as a colourless oil.
T.I.c.: CH/AcOEt 8:2, Rf=0.25 (detection with ninhydrine). NMR (d₆-DMSO): δ (ppm) 4.23 (q, 2H); 3.57 (t, 1H); 3.51 (t, 1 H); 3.37 (m, 2H); 3.18 (t,
1H); 2.96 (m, 2H); 2.77 (m, 1H); 1.7 (m, 2H); 1.39-1.36 (m, 9H); 1.28-1.21 (m, 6H).

### Intermediate 35

### 4-(1-Cyano-1-ethoxycarbonyl-methyl)-4-(4-fluorophenyl)-azepine-1-carboxylic acid tert-butyl ester

A solution of intermediate **34** (0.5 g) in anhydrous toluene (5 mL) was dropped over 30 minutes into a solution of 4-fluorophenyl magnesium bromide (2M in Et2O - 1.95 mL) in anhydrous Et2O (15 mL) previously cooled to 0°C under a Nitrogen atmosphere. The mixture was allowed to warm to r.t. and left at this temperature for 2 days. The mixture was treated with 3N sulfuric acid solution (5 mL), water (10 mL) and AcOEt (15 mL). The layers were separated and the aqueous phase was extracted with further AcOEt (2 x 15 mL). The combined organic layers were washed with a saturated sodium hydrogen carbonate solution (30 mL) and water (30 mL), then dried and concentrated *in vacuo.* The residue was purified by flash chromatography (CH/AcOEt 8:2) to give the title compound (331 mg) as a colourless oil.
T.I.c.: CH/AcOEt 8:2, Rf=0.30 (detection with ninhydrine).
NMR (CDCl₃): δ (ppm) 7.3 (m, 2H); 7.05 (m, 2H); 4.0 (bm, 2H); 4.2-2.5 (bm, 4H); 3.4 (s, 1H); 2.4-1.5 (bm, 6H); 1.4 (s + t, 12H).

### Intermediate 36

### 4-Carboxymethyl-4-(4-fluorophenyl)-azepine-1-carboxylic acid tert-butyl ester

A mixture of intermediate **35** (0.33 g) and a 15% potassium hydroxide solution in ethylene glycol (7 mL) was heated to 180°C overnight. The solution was allowed to cool to r.t., diluted with water (5 mL), cooled at 0°C and acidified with 15% hydrochloric acid until pH=1. The glycol-aqueous phase was distilled off (70°C/2 mbar) to leave a brown residue which was further dried (140°C/2 mbar for 1 hour) to give a mixture of 4-carboxymethyl-4-(4-fluorophenyl)-azepine and potassium chloride (700 mg).

Sodium hydroxide (24 mg) and di-*tert*-butyl-dicarbonate (60 mg) were added to a part of this material (80 mg) suspended in water (5 mL). The solution was stirred at r.t. overnight, then it was cooled to 0°C and treated with 0.1 M hydrochloric acid solution until pH=1 and extracted with Et2O (20 mL). The organic phase was dried, concentrated *in vacuo* and the residue was purified by flash chromatography (CH/AcOEt 1:1) to give the title compound (17 mg) as a colourless oil.

T.I.c.: CH/AcOEt 1:1, Rf=0.25 (detection with ninhydrine).

NMR (CDCl₃): δ (ppm) 7.25 (m, 2H); 6.95 (t, 2H); 3.8-3.4 (m, 2H); 3.2 (m, 2H); 2.6 (s, 2H);2.5 (m, 3H); 2.3 (m, 1 H); 2.0-1.5 (m, 2H); 1.4 (bs, 9H).

### Intermediate 37

### 4-{[(3,5-Bis-trifluoromethyl-benzyl)methylcarbamoyl]-methyl}-4-(4-fluorophenyl)-azepine-1-carboxylic acid tert-butyl ester

3,5-(Bis-trifluoromethyl-benzyl)-methylamine (14 mg), 1-(3-dimethylaminopropyl)-3-ethylcarbodiimide hydrochloride (10 mg) and 1-hydroxybenzotriazole (7 mg) were added to a solution of intermediate **36** (17 mg) in anhydrous DMF (2 mL) under a Nitrogen atmosphere. The mixture was stirred at r.t. overnight, then it was diluted with AcOEt (10 mL) and washed with water (10 mL), 0.005M hydrochloric acid solution (10 mL) and a saturated sodium hydrogen carbonate solution (10 mL). The organic layer was dried, concentrated *in vacuo* and the residue was purified by flash chromatography (CH/AcOEt 6:4) to give the title compound (18 mg) as a colourless oil.
T.I.c.: CH/AcOEt 6:4, Rf=0.4.
NMR (d₆-DMSO - 70°C): δ (ppm) 7.93 (bs, 1H); 7.78 (bs, 2H); 7.31 (m, 2H); 6.97 (bt, 2H); 4.51 (m, 2H); 3.51 (bm, 1H); 3.35 (bm, 1 H); 3.15 (m, 2H); 2.71 (s, 3H); 2.7 (m, 2H); 2.48 (m, 1H); 2.26 (m, 1 H); 2.04 (m, 1H); 1.87 (m, 1 H); 1.69 (m, 1H); 1.51 (1 H); 1.35 (s, 9H).

### Intermediate 38

### 4-(1-Cyano-1-ethoxycarbonyl-methyl)-4-(4-fluoro-2-methyl-phenyl)-azepine-1-carboxylic acid tert-butyl ester

A small amount of iodine and 3 drops of methyl iodide were added to a suspension of magnesium turnings (655 mg) in dry Et2O (15 mL), at r.t., under a Nitrogen atmosphere, then the mixture was vigorously refluxed. 2-Bromo-5-fluoro-toluene (3.1 mL) was added over 1 hour to this mixture followed by further Et2O (5 mL). The suspension was heated under vigorous reflux for 1.5 hours, then further Et2O (40 mL) was added. A solution of intermediate **34** (2.5 g) in anhydrous Et2O (10 mL) was added to the solution of Grignard so obtained over 30 minutes. The resulting solution was refluxed for further 30 minutes then it was cooled to 0°C, a 3M sulfuric acid solution (15 mL) and water (25 mL) were added and the aqueous layer was extracted with AcOEt (2 x 30 mL).
The combined organic extracts were washed with a saturated sodium hydrogen carbonate solution (50 mL) and concentrated *in vacuo.* The residue was purified by flash chromatography (CH/AcOEt from 8:2 to 6:4) to give the title compound (531 mg) as a white foam.
T.I.c.: CH/AcOEt 1:1, Rf=0.76 (detection with ninhydrine).
NMR (CDCl₃): δ (ppm) 7.3 (m, 2H); 7.05 (m,2H); 4.0 (bm, 2H); 4.2-2.5 (bm, 4H); 3.4 (s, 1H); 2.4-1.5 (bm, 6H); 1.4 (s + t, 12H).

### Intermediate 39

### 4-Carboxymethyl-4-(4-fluoro-2-methyl-phenyl)-azepine-1-carboxylic acid tert-butyl ester

A mixture of intermediate **38** (0.531 g) in acetic acid (3 mL), conc. sulfuric acid (1 mL) and water (1 mL) was heated to 140°C overnight. The solution was allowed to cool to r.t. and dropped into a 2.5M sodium hydroxide solution (50 mL). Then, di-*tert*-butyl-dicarbonate (277 mg) was added and the resulting mixture was stirred at r.t. for 5 hours. It was cooled to 0°C and treated with 6M hydrochloric acid solution until pH=1 and then extracted with AcOEt (20 mL). The organic phase was dried, concentrated *in vacuo* and the residue was purified by flash chromatography (CH/AcOEt 7:3) to give the title compound (60 mg) as a pale yellow oil.
T.I.c.: CH/AcOEt 1:1, Rf=0.35 (detection with ninhydrine).
NMR (d₆-DMSO): δ (ppm) 11.88 (bs, 1H); 7.3 (m, 1H); 6.99 (m, 1H); 6.91 (m, 1H); 3.6-3.0 (m, 4H); 2.6-2.4 (m, 2H); 2.45 (s, 3H); 2.6-2.4 (m, 2H); 2.05-1.9 (m, 2H); 1.8-1.4 (m, 2H); 1.3 (s, 9H).

### Intermediate 40

### 4-{[(3,5-Bis-trifluoromethyl-benzyl)methylcarbamoyl]-methyl}-4-(4-fluoro-2-methylphenyl)-azepine-1-carboxylic acid tert-butyl ester

DIPEA (43 µL) and O-(benzotriazol-1-yl)-N,N,N'N'-tetramethyluronium tetrafluoroborate (34.3 mg) were added to a solution of intermediate **39** (30 mg) in anhydrous DMF (3 mL) under a Nitrogen atmosphere. After stirring for 10 minutes, 3,5-(bis-trifluoromethylbenzyl)-methylamine hydrochloride (25.5 mg), was added. The mixture was stirred at r.t. overnight, then it was diluted with AcOEt (10 mL) and washed with water (10 mL) and a saturated sodium hydrogen carbonate solution (10 mL). The organic layer was dried, concentrated *in vacuo* and the residue was purified by flash chromatography (CH/AcOEt 6:4) to give the title compound (30 mg) as a colourless oil.
T.I.c.: CH/AcOEt 1:1, Rf=0.4.
NMR (d₆-DMSO - 60°C): δ (ppm) 7.94 (s, 1H); 7.8 (s, 2H); 7.16 (m, 1H); 6.89 (m, 1H); 6.75 (m, 1H); 4.54 (s, 2H); 3.6-3.0 (m, 4H); 2.7 (s, 3H); 2.8-2.4 (m, 4H); 2.44 (s, 3H); 1.98 (m, 1H); 1.74 (m, 2H); 1.49 (m, 1H); 1.3 (bs, 9H).

### Intermediate 41

### 4-{[(3,5-Dichlorobenzyl)methylcarbamoyl]-methyl}-4-(4-fluoro-2-methyl-phenyl)-azepine-1-carboxylic acid tert-butyl ester

DIPEA (34.3 µL) and O-(benzotriazol-1-yl)-N,N,N'N'-tetramethyluronium tetrafluoroborate (34.3 mg) were added to a solution of intermediate **39** (30 mg) in anhydrous DMF (3 mL) under a Nitrogen atmosphere. After stirring for 10 minutes, 3,5-dichlorobenzyl-methylamine hydrochloride (20.5 mg) was added. The mixture was stirred at r.t. overnight, then it was diluted with AcOEt (10 mL) and washed with water (10 mL) and a saturated sodium hydrogen carbonate solution (10 mL). The organic layer was dried, concentrated *in vacuo* and the residue was purified by flash chromatography (CH/AcOEt 6:4) to give the title compound (30 mg) as a colourless oil.
T.I.c.: CH/AcOEt 1:1, Rf=0.4.
NMR (d₆-DMSO): δ (ppm) 7.55-7.45 (m, 1H); 7.25-7.15 (m, 1H); 7.11 (s, 1H); 7.08 (s, 1H); 7.0-6.8 (m, 2H); 4.45-4.3 (m, 2H); 3.6-3.0 (m, 4H); 2.85-2.4 (m, 4H); 2.69 (s, 3H); 2.44 (s, 3H); 2.0-1.8 (m, 1H); 1.72 (m, 2H); 1.5-1.4 (m, 1H); 1.3 (bs, 9H).

### Intermediate 42

### 3-Fluoro-4-oxo-piperidine-1-carboxylate tert-butyl ester

Trimethylsilyl trifluoromethanesulfonate (2.17 mL) was added to a solution of *tert*-butyl-4-oxo-1-piperidine carboxylate (2.0 g) and TEA (3.34 mL) in anhydrous DCM (20 mL) previously cooled to -30°C under a Nitrogen atmosphere. The mixture was stirred at this temperature for 1 hour, then it was quickly treated with cold saturated sodium hydrogen carbonate solution (20 mL). The layers were separated and the organic phase was dried and concentrated *in vacuo* to give 1-(*tert*-butoxycarbonyl)-1,2,3,6-tetrahydro-4-pyridinyl-trifluoromethanesulfonate as a yellow oil, which was dissolved in anhydrous acetonitrile (80 mL) and the resulting solution was treated with Selectfluor (3.89 g). The mixture was stirred at r.t. for 1 hour, then it was concentrated *in vacuo* and the residue was dissolved in AcOEt (50 mL). The organic layer was washed with a saturated sodium hydrogen solution (30 mL), dried and concentrated *in vacuo* to a residue, which was purified by chromatography on neutral Alumina Brochmann type 1 (initially AcOEt then AcOEt/MeOH 95:5) to give the title compound (0.99 g) as a colourless oil.
T.I.c.: CH/AcOEt 1:1, Rf=0.3.
NMR (d₆-DMSO): δ (ppm) 5.08 (dd, 1H); 4.32 (bm, 1H); 4.0 (bm, 1H); 3.19 (m, 2H); 2.56 (m, 1 H); 2.36 (m, 1H); 1.43 (s, 9H).

### Intermediate 43

### 5-Ethoxycarbonyl-3-fluoro-4-oxo-azepine-1-carboxylic acid tert-butyl ester

Ethyl diazoacetate (7.8 mL) and boron trifluoride diethyl etherate (7.2 mL) were added simultaneously over 50 minutes to a suspension of intermediate **42** (12.39 g) in dry Et2O (250 mL) previously cooled to -20°C under a Nitrogen atmosphere. The solution obtained was stirred at -20°C for 1 hour, then it was allowed to warm to 0°C, and treated with a saturated potassium carbonate solution (200 mL). The layers were separated and the organic phase was dried and concentrated *in vacuo.* The residue was purified by flash chromatography (CH/AcOEt 9:1) to give the title compound (5.42 g) as a yellow oil.
T.I.c.: CH/AcOEt 85:15, Rf=0.1 (detection with ninhydrine).
NMR (CDCl₃): δ (ppm) 5.06 (dt, 1H); 4.3-4.1 (m, 2H); 3.53 (m, 1H); 4.0-3.2 (bs, 4H); 2.2-2.0 (m, 2H); 1.4 (s, 9H); 1.3-1.2 (t, 3H).
MS (ES/+): m/z=304 [MH]⁺.

### Intermediate 44

### 3-Fluoro-4-oxo-azepine-1-carboxylic acid, tert-butyl ester

A suspension of intermediate **43** (100 mg) in 3M hydrochloric acid (5 mL) was heated to reflux for 6 hours. The mixture was concentrated *in vacuo* to give the 3-fluoro-azepin-4-one hydrochloride (60.5 mg) as a whitish solid.
Di *tert*-butyl dicarbonate (86 mg) was added to a suspension of this compound (60.5 mg) and TEA (0.1 mL) in anhydrous DCM (5 mL) under a Nitrogen atmosphere. The mixture was stirred at r.t. overnight. The mixture was treated with a saturated ammonium chloride solution (10 mL) and extracted with further DCM (2 x 10 mL) The combined organic extracts were dried and concentrated *in vacuo*. The residue was purified by flash chromatography (CH/AcOEt 7:3) to give the title compound (50 mg) as a colourless oil.
T.I.c.: CH/AcOEt 7:3, Rf=0.23 (detection with ninhydrine).
NMR (d₆-DMSO - 70°C): δ (ppm) 5.15 (dt, 1H); 3.76 (m, 2H); 3.53 (bm, 1H); 3.23 (bm, 1 H); 2.54 (m, 1H); 2.43 (m, 1H); 1.71 (m, 1H); 1.64 (m, 1 H); 1.35 (s, 9H).

### Intermediate 45

### 4-(1-Cyano-1-ethoxycarbonyl-methyl)-3-fluoro-azepine-1-carboxylic acid tert-butyl ester

A round bottom flask equipped with a Dean Stark apparatus was charged with intermediate **44** (1.136 g), ethyl cyanoacetate (0.576 mL), methyl ammonium acetate (0.189 g) and acetic acid (0.281 mL) in anydrous benzene (9 mL). The mixture was heated to reflux overnight, then it was allowed to cool to r.t. and washed with water (20 mL). The aqueous layer was extracted with AcOEt (2 x 20 mL). The combined organic extracts were washed with 1M sodium hydroxide solution (20 mL) and a saturated ammonium chloride solution (20 mL), dried and concentrated *in vacuo.* The residue was purified by flash chromatography (CH/AcOEt 8:2) to give the title compound (0.873 g - cis/trans mixture) as a colourless oil.
T.I.c.: CH/AcOEt 7:3, Rf=0.28 (detection with ninhydrine).
MS (ES/+): m/z= 326 [MH]⁺.

### Intermediate 46

### 4-(1-Cyano-1-ethoxycarbonyl-methyl)-3-fluoro-4-(4-fluoro-2-methyl-phenyl)-azepine-1-carboxylic acid tert-butyl ester (anti isomer)

A small amount of iodine and 3 drops of methyl iodide were added to a suspension of magnesium turnings (215 mg) in dry Et2O (5 mL), at r.t., under a Nitrogen atmosphere, then the mixture was vigorously refluxed. 2-Bromo-5-fluoro-toluene (1.016 mL) was added over 1 hour to this mixture followed by further Et2O (2 mL). The suspension was heated under vigorous reflux for 1 hour. A solution of intermediate **45** (0.873 g) in anhydrous Et2O (3 mL) was added to the solution of Grignard so obtained over 30 minutes. The resulting mixture was refluxed for further 30 minutes then it was cooled to 0°C, a 3M sulfuric acid solution (3 mL) was added and the aqueous layer was extracted with AcOEt (2 x 30 mL).

The combined organic layers were washed with a saturated sodium hydrogen carbonate solution (30 mL) and water (15 mL), then dried and concentrated *in vacuo.* The residue was purified by flash chromatography (CH/AcOEt from 9:1 to 8:2) to give the title compound (308.4 mg) as a colourless oil.
T.I.c.: CH/AcOEt 8:2, Rf=0.21 (detection with ninhydrine).
NMR (d₆-DMSO - 70°C): δ (ppm) 7.34-7.27 (2m, 2H); 7.05-6.96 (bm, 2H); 5.8 (2bd, 1H); 4.58-4.33 (2s, 1 H); 4.07 (m, 2H); 3.8-3.5 (bm, 2H); 3.4-3.0 (bm, 2H); 2.7-2.3 (bm, 1H); 1.8 (1.6 (bm, 1H); 1.8-1.6 (bm, 1H); 1.5-1.2 (bm, 1H); 1.23 (bs, 9H); 1.13-0.96 (2t, 3H).
MS (ES/+): m/z= 437 [MH]⁺.

### Intermediate 47

### 4-Carboxymethyl-3-fluoro-4-(4-fluoro-2-methyl-phenyl)-azepine-1-carboxylic acid tert-butyl ester (anti isomer

A mixture of intermediate **46** (0.29 g) in acetic acid (3 mL), conc. sulfuric acid (1 mL) and water (1 mL) was heated to 140°C for 6 hours. The solution was allowed to cool to r.t. and dropped into a 2.5M sodium hydroxide solution (30 mL). Then, di-*tert*-butyl-dicarbonate (145 mg) was added and the resulting mixture was stirred at r.t. overnight. It was cooled to 0°C and treated with 6M hydrochloric acid solution until pH=1 and then extracted with AcOEt (20 mL). The organic phase was dried, concentrated *in vacuo* and the residue was purified by flash chromatography (CH/AcOEt from 8:2 to 6:4) to give the title compound (79.7 mg) as a pale yellow oil.
T.I.c.: CH/AcOEt 7:3, Rf=0.18 (detection with ninhydrine).
NMR (d₆-DMSO - 70°C): δ (ppm) 11.64 (bs, 1H); 7.25 (dd, 1H); 6.96 (dd, 1H); 5.57 (dt, 1H); 3.78 (bm, 2H); 3.18 (bt, 2H); 2.83 (d, 1H); 2.68 (d, 1H); 2.47 (s, 3H); 2.33 (m, 1H); 2.09 (m, 1H); 1.79 (m, 1H); 1.59 (m, 1H); 1.31 (s, 9H).
MS (ES/+): m/z= 384 [MH]⁺.

### Intermediate 48

### 4-{[(3,5-Bis-trifluoromethyl-benzyl)methylcarbamoyl]-methyl}-3-fluoro-4-(4-fluoro-2-methyl-phenyl)-azepine-1-carboxylic acid, tert-butyl ester(anti isomer)

DIPEA (50 µL) and O-(benzotriazol-1-yl)-N,N,N'N'-tetramethyluronium tetrafluoroborate (37 mg) were added to a solution of intermediate **47** (34 mg) in anhydrous DMF (5 mL) under a Nitrogen atmosphere. After stirring for 10 minutes, 3,5-(bis-trifluoromethylbenzyl)-methylamine hydrochloride (28.7 mg), was added. The mixture was stirred at r.t. overnight, then it was diluted with AcOEt (10 mL) and washed with water (10 mL). The organic layer was dried, concentrated *in vacuo* and the residue was purified by flash chromatography (CH/AcOEt 7:3) to give the title compound (37.1 mg) as a colourless oil.
T.I.c.: CH/AcOEt 7:3, Rf=0.19.
NMR (d₆-DMSO): δ (ppm) 8.04-7.88 (2bs, 1H); 7.77-7.72 (2bs, 2H); 7.12 (m, 1H); 6.91 (m, 1H); 6.73 (m, 1H); 5.8-5.62 (2bd, 1H); 4.8-4.4 (m, 2H); 3.9-3.5 (bm, 2H); 3.13 (bm, 2H); 2.92 (bm, 2H); 2.83-2.81 (2bs, 3H); 2.44-2.42 (2bs, 3H); 2.35-2.0 (bm, 2H); 1.67 (bm,
1H); 1.48 (bm, 1 H); 1.28 (s, 9H).

### Intermediate 49

### 4-{[(3,5-Dichlorobenzyl)methylcarbamoyl]-methyl}-3-fluoro-4-(4-fluoro-2-methylphenyl)-azepine-1-carboxylic acid, tert-butyl ester(anti Isomer)

DIPEA (50 µL) and O-(benzotriazol-1-yl)-N,N,N'N'-tetramethyluronium tetrafluoroborate (36.2 mg) were added to a solution of intermediate **47** (33.2 mg) in anhydrous DMF (5 mL) under a Nitrogen atmosphere. After stirring for 10 minutes, 3,5-dichlorobenzyl-methylamine hydrochloride (21.6 mg), was added. The mixture was stirred at r.t. overnight, then it was diluted with AcOEt (10 mL) and washed with water (10 mL). The organic layer was dried, concentrated *in vacuo* and the residue was purified by flash chromatography (CH/AcOEt 75:25) to give the title compound (38.4 mg) as a colourless oil. T.I.c.: CH/AcOEt 7:3, Rf=0.21.
NMR (d₆-DMSO): δ (ppm) 7.49-7.42 (2t, 1H); 7.13 (m, 1H); 7.02 (d, 2H); 6.85 (m, 2H); 5.81-5.65 (2bd, 1H); 4.41-4.28 (2m, 2H); 3.9-3.5 (m, 2H); 3.08 (bm, 2H); 2.86 (m, 2H); 2.72-2.7 (2s, 3H); 2.41-2.4 (2s, 3H); 2.35-2.0 (bm, 2H); 1.65 (bm, 1H); 1.45 (bm, 1H); 1.25 (s, 9H).

### Intermediate 50

### 4-(1-Cyano-1-ethoxycarbonyl-methyl)-4-phenyl-piperidine-1-carboxylic acid tert-butyl ester

A solution of phenyl magnesium bromide (1.0M in THF - 37 mL) was added drop-wise to a mixture of intermediate **1** (6 g) and copper iodide (1.18 g) in anhydrous THF (48 mL) previously cooled to 0°C under a Nitrogen atmosphere. The mixture was allowed to warm to r.t. and stirred at 23°C for 40 minutes. The mixture was cooled to 0°C, treated with 3M hydrochloric acid solution until pH=5 and extracted with AcOEt (3 x 50 mL). The combined organic extracts were washed with a saturated ammonium chloride solution (100 mL), dried and concentrated *in vacuo.* The residue was purified by flash chromatography (CH/AcOEt 85:15) to give the title compound (5.2 g) as a colourless oil.
T.I.c.: CH/AcOEt 8:2, Rf=0.24 (detection with ninhydrine).

### Intermediate 51

### 4-Carboxymethyl-phenyl-piperidine-1-carboxylic acid tert-butyl ester

A mixture of intermediate **50** (5.2 g) in acetic acid (59 mL), conc. sulfuric acid (19.5 mL) and water (19.5 mL) was heated to 140°C for 12 hours. The solution was allowed to cool to r.t., a 2.5M sodium hydroxide solution added, followed by sodium hydroxide pellets until pH 8. Then, di-*tert*-butyl-dicarbonate (5.9 g) was added and the resulting mixture was stirred at r.t. for 2 days. It was cooled to 0°C and treated with 6M hydrochloric acid solution until pH=6-7 and then extracted with AcOEt (3 x 50 mL). The organic phase was washed with brine, dried and concentrated *in vacuo.* The residue was purified by flash chromatography (CH/AcOEt 8:2 ) to give the title compound (2.15 g) as a white solid.

T.I.c.: CH/AcOEt 1:1, Rf=0.18 (detection with ninhydrine).

MS (ES/+): m/z= 342 [M+Na]⁺.

### Intermediate 52

### [1-(3,5-Dichloro-phenyl)-ethyl]-methylamine

Methylamine (2M solution in MeOH - 13 mL) was added to a solution of 3,5-dichloroacetophenone (500 mg) in MeOH (26 mL) under a Nitrogen atmosphere. The mixture was stirred at r.t. for 18 hours, then it was cooled to 0°C and sodium borohydride (98 mg) was added. The mixture was stirred at 0°C for 2 hours, then it was quenched with water and extracted with DCM. The organic layer was dried and concentrated *in vacuo* to give the title compound (340 mg) as an yellow oil.
T.I.c.: CH/AcOEt 1:1, Rf=0.15.
NMR (CDCl₃): δ (ppm) 7.3 (m, 3H); 3.6 (q, 1H); 2.3 (s, 3H); 1.35 (d, 3H).
MS (ES/+): m/z=204 [M+H]⁺.

### Intermediate 53

### [1-(3,5-Dibromo-phenyl)-ethyl]-methylamine

Methylamine (2M solution in MeOH - 138 mL) was added to a solution of 3,5-dibromoacetophenone (7.7 g) in MeOH (138 mL) under a Nitrogen atmosphere. The mixture was stirred at r.t. overnight, then it was cooled to 0°C and sodium borohydride (1.57 g) was added. The mixture was allowed to reach r.t. in 3 hours, then it was concentrated *in vacuo*, the residue was dissolved in Et2O and washed with water and brine. The organic layer was dried and concentrated *in vacuo* to give the title compound (7.8 g) as an orange oil.
T.I.c.: DCM/MeOH 9:1, Rf=0.32.
MS (ES/+): m/z=294 [M+H]⁺.

### Intermediate 54 and Intermediate 55

### [1-(3,5-Dichloro-phenyl)-ethyl]-methylamine (54, Enantiomer 1) and (55, Enantiomer 2)

To a solution of intermediate **52** (759 mg) in dry THF (4 mL), a solution of di-p-toluoyl-D-tartaric acid (1.3 g) in dry THF (4 mL) was added. The solution was stirred at r. t. overnight. The suspension obtained was filtered and the solid residue washed with dry THF (13 mL). The solid (1.8 g) was crystallised from dry THF (15 mL) by heating to reflux for 1 hour, cooling to r.t. and stirring for 2 hours. The suspension was filtered and the solid residue (1 g) washed with dry THF (15 mL) and recristallised as described above twice to give [1-(3,5-dichloro-phenyl)-ethyl]-methylamine di-p-toluoyl-D-tartrate salt (690 mg). The solid was stirred in a mixture of saturated sodium hydrogen carbonate solution (20 mL) and DCM (20 mL). The organic phase was washed with water (20 mL), dried and concentrated *in vacuo* to give the title compound intermediate **55** (230 mg) as colourless oil.

The mother liquors from the precipitation and crystallizations were collected, concentrated *in vacuo*, treated with saturated sodium hydrogen carbonate solution (20 mL) and extracted with DCM (20 mL). The colourless oil thus obtained (550 g) was processed with di-p-toluoyl-L-tartaric acid (1.04 g) in dry THF as described above (one precipitation and three recristallisations) to give [1-(3,5-dichloro-phenyl)-ethyl]-methylamine di-p-toluoyl-L-tartrate salt (820 mg). The solid was stirred in a mixture of saturated sodium hydrogen carbonate solution (20 mL) and DCM (20 mL). The organic phase was washed with water (20 mL), dried and concentrated *in vacuo* to give the title compound intermediate **54** (238 mg) as colourless oil.

### Intermediate 54:

NMR (CDCl₃): δ (ppm) 7.3 (m, 3H); 3.6 (q, 1H); 2.3 (s, 3H); 1.35 (d, 3H).
MS (ES/+): m/z=204 [M+H]⁺.
HPLC (column: Chiral-AGP 15cm x 2mm, 5µm; injection volume=1µL; mobile phase: ammonium phosphate buffer 100mM pH=4.4 / CH₃CN isocratic 96/4 % v/v; flow rate= 0.13 mL/min; detection: λ=210 nm): retention time = 5.2 minutes; purity (a/a %) 100%.

### Intermediate 55:

NMR (CDCl₃): δ (ppm) 7.3 (m, 3H); 3.6 (q, 1H); 2.3 (s, 3H); 1.35 (d, 3H).
MS (ES/+): m/z=204 [M+H]⁺.
HPLC (column: Chiral-AGP 15cm x 2mm, 5µm; injection volume=1µL; mobile phase: ammonium phosphate buffer 100mM pH=4.4 / CH₃CN isocratic 96/4 % v/v; flow rate= 0.13 mL/min; detection: λ=210 nm): retention time = 5.5 minutes; purity (a/a %) 98.5%.

### Intermediate 56 and Intermediate 57

### [1-(3,5-Dibromo-phenyl)-ethyl]-methylamine (56, Enantiomer 1) and (57, Enantiomer 2)

To a solution of intermediate **53** (1.3 g) in dry THF (7 mL), a solution of di-p-toluoyl-D-tartaric acid (1.7 g) in dry THF (7 mL) was added. The solution was stirred at r.t. overnight. The suspension obtained was filtered and the solid residue washed with dry THF (2 x 10 mL). The solid obtained (1.2 g) was crystallised from dry THF (15 mL) by heating to reflux for 1 hour, cooling to r.t. and stirring for 2 hours. The suspension was filtered and the solid residue (0.88 g) washed with dry THF (15 mL) and recristallised as described above twice to give [1-(3,5-dibromo-phenyl)-ethyl]-methylamine di-p-toluoyl-D-tartrate salt (542 mg). The solid was stirred in a mixture of saturated sodium hydrogen carbonate solution (20 mL) and DCM (20mL). The organic phase was washed with water (20 mL), dried and concentrated *in vacuo* to give the title compound intermediate **57** (180mg) as colourless oil.

The two first mother liquors were collected, concentrated *in vacuo* and treated with saturated sodium hydrogen carbonate solution (20 mL) and extracted with DCM (20 mL). The colourless oil thus obtained (880 mg) was processed with di-p-toluoyl-L-tartaric acid (1.16 g) in dry THF as described above (one precipitation and three recristallisations) to give the [1-(3,5-dibromo-phenyl)-ethyl]-methylamine di-p-toluoyl-L-tartrate salt (603 mg). The solid was stirred in a mixture of saturated sodium hydrogen carbonate solution (20 mL) and DCM (20 mL). The organic phase was washed with water (20 mL), dried and concentrated *in vacuo* to give the title compound intermediate **56** (225 mg) as colourless oil.

### Intermediate 56:

NMR (CDCl₃): δ (ppm) 7.6 (t, 1H); 7.4 (d, 2H); 3.6 (q, 1H); 2.3 (s, 3H); 1.3 (d, 3H).
MS (ES/+): m/z=294 [M+H]⁺.
HPLC (column: Chiral-AGP 15cm x 2mm, 5µm; injection volume=1µL; mobile phase: ammonium phosphate buffer 100mM pH=4.4 / acetonitrile isocratic 96/4 % v/v; flow rate= 0.13 mL/min; detection: λ=210 nm): retention time = 7.7 minutes; purity (a/a %) 98.2%.

### Intermediate 57:

NMR (CDCl₃): δ (ppm) 7.6 (t, 1H); 7.4 (d, 2H); 3.6 (q, 1H); 2.3 (s, 3H); 1.3 (d, 3H).
MS (ES/+): m/z=294 [M+H]⁺.
HPLC (column: Chiral-AGP 15cm x 2mm, 5µm; injection volume=1µL; mobile phase: ammonium phosphate buffer 100mM pH=4.4 / acetonitrile isocratic 96/4 % v/v; flow rate= 0.13 mL/min; detection: λ=210 nm): retention time = 9.1 minutes; purity (a/a %) 99.3%.

### Intermediate 58

### 4-{[(3,5-Dibromobenzyl)methylcarbamoyl]methyl}-4-(4-fluoro-phenyl)-piperidine-1-carboxylic acid tert-butyl ester

DIPEA (1.3 mL) and O-(benzotriazol-1-yl)-N,N,N'N'-tetramethyluronium tetrafluoroborate (1.05g) were added to a solution of intermediate **3** (850 mg) in anhydrous DMF (12 mL) under a Nitrogen atmosphere. After stirring for 15 minutes, 3,5-dibromobenzyl-methylamine hydrochloride (794 mg) was added. The mixture was stirred at r.t. for 48 hours, then it was diluted with DCM and washed with 5% sodium hydrogen carbonate solution and brine. The organic layer was dried, concentrated *in vacuo* and the residue was purified by flash chromatography (CH/AcOEt from 7:3 to 1:1) to give the title compound (1.38 g) as a colourless oil.
T.I.c.: CH/AcOEt 1:1, Rf=0.35 (detection with ninhydrine).
MS (ES/+): m/z=621 [M+Na]⁺.

### Intermediate 59

### 4-{[1-(3,5-Dichloro-phenyl)-ethylcarbamoyl]-methyl}-4-(4-fluoro-phenyl)-piperidine-1-carboxylic acid tert-butyl ester

DIPEA (675 µL) and O-(benzotriazol-1-yl)-N,N,N'N'-tetramethyluronium tetrafluoroborate (525 mg) were added to a solution of intermediate **3** (426 mg) in anhydrous DMF (6 mL) under a Nitrogen atmosphere. After stirring for 15 minutes, 1-(3,5-dichloro-phenyl)-ethylamine hydrochloride (283 mg) was added. The mixture was stirred at r.t. for 48 hours, then it was diluted with DCM (14 mL), washed with 5% sodium hydrogen carbonate solution (12 mL), and passed through a phase separation cartridge with polypropylene frit and concentrated *in vacuo*. The residue was purified by flash chromatography (CH/AcOEt 7:3) to give the title compound (343 mg) as a colourless oil.
T.I.c.: CH/AcOEt 1:1, Rf=0.28 (detection with ninhydrine).
MS (ES/+): m/z=531 [M+Na]⁺.

### Intermediate 60

### 4-({[1-(S)-1-(3,5-Bis-trifluoromethyl-phenyl)-ethyl]-methyl-carbamoyl}-methyl)-4-(4-fluoro-phenyl)-piperidine-1-carboxylic acid tert-butyl ester

DIPEA (675 µL) and O-(benzotriazol-1-yl)-N,N,N'N'-tetramethyluronium tetrafluoroborate (525 mg) were added to a solution of intermediate **3** (426 mg) in anhydrous DMF (6 mL) under a Nitrogen atmosphere. After stirring for 15 minutes, [1-(*S*)-(3,5-bis-trifluoromethylphenyl)-ethyl]-methyl-amine (341 mg) was added. The mixture was stirred at r.t. for 48 hours, then it was diluted with DCM (14 mL), washed with 5% sodium hydrogen carbonate solution (12 mL), and passed through a phase separation cartridge with polypropylene frit and concentrated *in vacuo*. The residue was purified by SCX cartridge (load with DCM and elution with MeOH, NH₃ 0.2M in MeOH) to give the title compound (590 mg ) as a yellow oil.
T.I.c.: CH/AcOEt 1:1, Rf=0.59 (detection with ninhydrine).
MS (ES/+): m/z 613 [M+Na]⁺.

### Intermediate 61

### 4-({[1-(3,5-Dibromo-phenyl)-ethyl]-methyl-carbamoyl}-methyl)-4-(4-fluoro-phenyl)-piperidine-1-carboxylic acid tert-butyl ester

DIPEA (675 µL) and O-(benzotriazol-1-yl)-N,N,N'N'-tetramethyluronium tetrafluoroborate (525 mg) were added to a solution of intermediate **3** (426 mg) in anhydrous DMF (6 mL) under a Nitrogen atmosphere. After stirring for 15 minutes, intermediate **53** (369 mg) was added. The mixture was stirred at r.t. for 48 hours, then it was diluted with DCM (14 mL), washed with 5% sodium hydrogen carbonate solution (12 mL), and passed through a phase separation cartridge with polypropylene frit and concentrated *in vacuo*. The residue was purified by SCX Cartridge (load with DCM and elution with MeOH, NH₃ 0.2M in MeOH) to give the title compound (390 mg ) as a yellow oil.
T.I.c.: CH/AcOEt 6:4, Rf=0.3 (detection with ninhydrine).
MS (ES/+): m/z=635 [M+Na]⁺.

### Intermediate 62

### 4-({[1-(3,5-Dibromo-phenyl)-ethyl]-methyl-carbamoyl}-methyl)-4-(4-fluoro-phenyl)-piperidine-1-carboxylic acid tert-butyl ester (enantiomer 1)

DIPEA (236 µL) and O-(benzotriazol-1-yl)-N,N,N'N'-tetramethyluronium tetrafluoroborate (285 mg) were added to a solution of intermediate **3** (230 mg) in anhydrous DMF (5 mL) under a Nitrogen atmosphere. After stirring for 30 minutes intermediate **56** (220 mg) was added. The mixture was stirred at r.t. overnight, then it was taken up with AcOEt (30 mL) and washed with water (4 x 30 mL) and brine (2 x 10 mL). The organic layer was dried, concentrated *in vacuo* and the residue was purified by flash chromatography (CH/AcOEt 9/1 then CH/AcOEt 8/2) to give the title compound (380 mg) as a white foam.
T.I.c.: CH/AcOEt 6:4, Rf=0.3 (detection with ninhydrine).
MS (ES/+): m/z=635 [M+Na]⁺.

### Intermediate 63

### 4-({[1-(3,5-Dibromo-phenyl)-ethyl]-methyl-carbamoyl}-methyl)-4-(4-fluoro-phenyl)-piperidine-1-carboxylic acid tert-butyl ester (enantiomer 2)

DIPEA (195 µL) and O-(benzotriazol-1-yl)-N,N,N'N'-tetramethyluronium tetrafluoroborate (232 mg) were added to a solution of intermediate **3** (188 mg) in anhydrous DMF (5 mL) under a Nitrogen atmosphere. After stirring for 30 minutes intermediate **57** (180 mg) was added. The mixture was stirred at r.t. overnight, then it was taken up with AcOEt (30 mL) and washed with water (4 x 5 mL) and brine (2 x 5 mL). The organic layer was dried, concentrated *in vacuo* and the residue was purified by flash chromatography (CH/AcOEt 7/3) to give the title compound (320 mg) as a white foam.
T.I.c.: CH/AcOEt 6:4, Rf=0.3 (detection with ninhydrine).
MS (ES/+): m/z=635 [M+Na]⁺.

### Intermediate 64

### 4-({[1-(3,5-Dichloro-phenyl)-ethyl]-methyl-carbamoyl}-methyl)-4-(4-fluoro-phenyl)-piperidine-1-carboxylic acid tert-butyl ester (enantiomer 1)

DIPEA (206 µL) and O-(benzotriazol-1-yl)-N,N,N'N'-tetramethyluronium tetrafluoroborate (248 mg) were added to a solution of intermediate **3** (200 mg) in anhydrous DMF (5 mL) under a Nitrogen atmosphere. After stirring for 30 minutes intermediate **54** (124 mg) was added. The mixture was stirred at r.t. overnight, then it was taken up with AcOEt (30 mL) and washed with water (3 x 30 mL) and brine (2 x 10 mL). The organic layer was dried, concentrated *in vacuo* and the residue was purified by flash chromatography (CH/AcOEt 9/1 then CH/AcOEt 8/2) to give the title compound (290 mg) as a white foam.
T.I.c.: CH/AcOEt 6:4, Rf=0.27 (detection with ninhydrine).
MS (ES/+): m/z=545 [M+Na]⁺.

### Intermediate 65

### 4-{[(3,5-Dibromobenzyl)methylcarbamoyl]methyl}-4-phenyl-piperidine-1-carboxylic acid tert-butyl ester

DIPEA (0.5 mL) and O-(benzotriazol-1-yl)-N,N,N'N'-tetramethyluronium tetrafluoroborate (392 mg) were added to a solution of intermediate **51** (300 mg) in anhydrous DMF (4.5 mL) under a Nitrogen atmosphere. After stirring for 30 minutes 3,5-dibromobenzyl-methylamine hydrochloride (296 mg) was added. The mixture was stirred at r.t. 30 hours, then it was diluted with DCM (12 mL) and saturated sodium hydrogen carbonate solution (14 mL). The organic layer was dried, concentrated *in vacuo* and the residue was purified by SCX cartridge (load with MeOH/DCM 1:1 and elution with NH₃ 0.2M in MeOH) to give the title compound (521 mg) as a yellow oil.
T.I.c.: CH/AcOEt 8:2, Rf=0.14 (detection with ninhydrine). MS (ES/+): m/z=603 [M+Na]⁺.

### Intermediate 66

### 4-({[1-(S)-1-(3,5-Bis-trifluoromethyl-phenyl)-ethyl]-methyl-carbamoyl}-methyl)-4-phenyl)-piperidine-1-carboxylic acid tert-butyl ester

DIPEA (0.5 mL) and O-(benzotriazol-1-yl)-N,N,N'N'-tetramethyluronium tetrafluoroborate (392 mg) were added to a solution of intermediate **51** (300 mg) in anhydrous DMF (4.5 mL) under a Nitrogen atmosphere. After stirring for 30 minutes [1-(*S*)-(3,5-bis-trifluoromethyl-phenyl)-ethyl]-methyl-amine (255 mg) was added. The mixture was stirred at r.t. 30 hours, then it was diluted with DCM (12 mL) and saturated sodium hydrogen carbonate solution (14 mL). The organic layer was dried, concentrated *in vacuo* and the residue was purified by SCX cartridge (load with MeOH/DCM 1:1 and elution with NH₃ 0.2M in MeOH) to give the title compound (465 mg) as a yellow oil.
T.I.c.: CH/AcOEt 8:2, Rf=0.12 (detection with ninhydrine).
MS (ES/+): m/z= 595 [M+Na]⁺.

### Intermediate 67

### 4-({[1-(3,5-Dibromo-phenyl)-ethyl]-methyl-carbamoyl}-methyl)-4-phenyl-piperidine-1-carboxylic acid tert-butyl ester

DIPEA (100 µL) and O-(benzotriazol-1-yl)-N,N,N'N'-tetramethyluronium tetrafluoroborate (78.5 mg) were added to a solution of intermediate **51** (60 mg) in anhydrous DMF (0.9 mL) under a Nitrogen atmosphere. After stirring for 30 minutes, intermediate **53** (55 mg) was added. The mixture was stirred at r.t. 30 hours, then it was diluted with DCM (12 mL) and saturated sodium hydrogen carbonate solution (14 mL). The organic layer was dried, concentrated *in vacuo* and the residue was purified by SCX cartridge (load with MeOH/DCM 1:1 and elution with NH₃ 0.2M in MeOH) to give the title compound (107 mg) as a yellow oil.
T.I.c.: CH/AcOEt 8:2, Rf=0.17 (detection with ninhydrine).

### Intermediate 68

### 4-({[1-(3,5-Dibromo-phenyl)-ethyl]-methyl-carbamoyl}-methyl)-4-phenyl-piperidine-1-carboxylic acid tert-butyl ester (enantiomer 1)

DIPEA (200 µL) and O-(benzotriazol-1-yl)-N,N,N'N'-tetramethyluronium tetrafluoroborate (234 mg) were added to a solution of intermediate **51** (180 mg) in anhydrous DMF (8 mL) under a Nitrogen atmosphere. After stirring for 30 minutes intermediate **56** (182 mg) was added. The mixture was stirred at r.t. overnight, then it was diluted with AcOEt and washed with ice/water and brine. The organic layer was dried, concentrated *in vacuo* and the residue was purified by flash chromatography (CH/AcOEt from 8:2 to 6:4) to give the title compound (320 mg) as a colourless oil.
T.I.c.: CH/AcOEt 1:1, Rf=0.53 (detection with ninhydrine).
MS (ES/+): m/z= 617 [M+Na]⁺.

### Intermediate 69

### 4-({[1-(3,5-Dichloro-phenyl)-ethyl]-methyl-carbamoyl}-methyl)-4-(4-fluoro-phenyl)-piperidine-1-carboxylic acid tert-butyl ester

Intermediate **59** ( 493 mg) was dissolved in dry THF (15 mL) under a Nitrogen athmosphere and NaH (60% supsension in mineral oil, 116 mg) was added in small portions. The reaction mixture was stirred under these conditions for 10 minutes then methyl iodide (363 µL) was added, and the reaction was heated up to 50°C and kept at this temperature for 7 hours. Then it was diluted with AcOEt (15 x 2 mL), washed with water (15 mL),and the dried organic phase was concentrated *in vacuo*. The residue was purified by flash chromatography (CH/AcOEt from 8:2 to 1:1) to give the title compound (300 mg) as a white solid.
T.I.c. : CH/AcOEt 1:1, Rf=0.43 (detection with ninhydrine).
MS (ES/+): m/z=523 [M+H]⁺.

### Intermediate 70

### 1,1-Dimethylethyl 4-{2-[{(1R)-1-[3,5-bis(trifluoromethyl)phenyl]ethyl}(methyl)amino]-2-oxoethyl}-4-(4-fluoro-2-methylphenyl)-1-piperidinecarboxylate

DIPEA (80 µl) and O-(benzotriazol-1-yl)-N,N,N'N'-tetramethyluronium tetrafluoroborate (96 mg) were added to a solution of intermediate **28** (80 mg) in anhydrous DMF (6.4 mL) under a Nitrogen atmosphere. After stirring for 30 minutes, [1-(*R*)-(3,5-bis-trifluoromethylphenyl)-ethyl]-methyl-amine (67.8 mg) was added. The mixture was stirred at r.t. for 5 hours, then it was diluted with AcOEt and washed with 5% sodium hydrogen carbonate solution and brine. The organic layer was dried, concentrated *in vacuo* and the residue was purified by flash chromatography (DCM/AcOEt from 95:5 to 9:1) to give the title compound (64.4 mg) as a colourless oil.
NMR (CDCl₃): δ (ppm) 7.77 (s, 1H); 7.56 (s, 2H); 7.27 (m, 1H); 6.83 (d, 2H); 6.00 (q, 1H); 3.72 (d, 2H); 3.17 (t, 2H); 2.81 (d, 2H); 2.53 (m, 2H); 2.50 (s, 3H); 2.25 (s, 3H); 2.05 (t, 2H); 1.45 (s, 9H); 1.32 (d, 3H).
MS (ES/+): m/z=605 [M+H]⁺.

### Intermediate 71

### 1,1-Dimethylethyl 4-{2-[[(3,5-dibromophenyl)methyl](methyl)amino]-2-oxoethyl}-4-(4-fluoro-2-methylphenyl)-1-piperidinecarboxylate

DIPEA (0.24 ml) and O-(benzotriazol-1-yl)-N,N,N'N'-tetramethyluronium tetrafluoroborate (192.6 mg) were added to a solution of intermediate **28** (80 mg) in anhydrous DMF (6.4 mL) under a Nitrogen atmosphere. After stirring for 30 minutes, 3,5-dibromobenzyl-methylamine (139.8 mg) was added. The mixture was stirred at r.t. overnight, then it was diluted with AcOEt and washed with 5% sodium hydrogen carbonate solution and brine. The organic layer was dried, concentrated *in vacuo* and the residue was purified by flash chromatography (DCM/AcOEt 9:1) to give the title compound (31 mg) as a yellow oil.
NMR (d₆-DMSO - 80°C): δ (ppm) 7.64 (s, 1H); 7.30 (m, 1H); 7.24 (s, 2H); 6.9-6.8 (m, 2H); 4.31 (s, 2H); 3.5 (m, 2H); 3.14 (m, 2H); 2.83 (s, 2H); 2.66 (s, 3H); 2.44 (s, 3H); 2.3 (m, 2H); 2.05 (m, 2H); 1.39 (s, 9H).
MS (ES/+): m/z=611 [M+H]⁺.
**Intermediate 72**

### 1,1-Dimethylethyl 4-[2-({[3,5-bis(trifluoromethyl)phenyl]methyl}amino)-2-oxoethyl]-4-(4-fluoro-2-methylphenyl)-1-piperidinecarboxylate

DIPEA (0.2 ml) and O-(benzotriazol-1-yl)-N,N,N'N'-tetramethyluronium tetrafluoroborate (119 mg) were added to a solution of intermediate **28** (100 mg) in anhydrous DMF (8.3 mL) under a Nitrogen atmosphere. After stirring for 30 minutes, {[3,5-bis(trifluoromethyl)phenyl]methyl}amine (104 mg) was added. The mixture was stirred at r.t. for 2 hours, then it was diluted with AcOEt and washed with 5% sodium hydrogen carbonate solution and brine. The organic layer was dried, concentrated *in vacuo* and the residue was purified by flash chromatography (CH, then CH/AcOEt from 9:1 to 1:1) to give the title compound (153 mg) as a yellow oil.
T.I.c.: CH/AcOEt 1:1, Rf=0.26 (detection with ninhydrine).

### Intermediate 73

### 1,1-Dimethylethyl 4-[1-cyano-2-(ethyloxy)-2-oxoethyl]-4-(2-methylphenyl)-1-piperidinecarboxylate

A 0.6M solution of 2-methylphenyl magnesium bromide was prepared by adding drop-wise a solution of 2-bromo-toluene (4.91 mL) in anydrous THF (35 mL) to magnesium turnings (1.1g) and a cristalline of iodine in anydrous THF (28 mL) under Nitrogen athmosphere, and stirring at reflux for 0.5 hours. A solution of 2-methylphenyl magnesium bromide (0.6M in THF, 25.5 mL) was added drop-wise to a mixture of intermediate **1** (2.5 g) and copper iodide (490 mg) in anhydrous THF (20 mL) previously cooled to 0°C under a Nitrogen atmosphere. The mixture was allowed to warm to r.t. and stirred under these conditions for 2 hours. The mixture was cooled to 0°C, treated with 3M hydrochloric acid solution until pH=5 and extracted with AcOEt (2 x 100 mL). The combined organic extracts were washed with a saturated ammonium chloride solution (200 mL), dried and concentrated *in vacuo*. The residue was purified by flash chromatography (CH/AcOEt 9:1 to 8:2) to give the title compound (2.55 g) as a yellow oil.
T.I.c.: CH/AcOEt 8:2, Rf=0.27 (detection with ninhydrine).
MS (ES/+): m/z=409 [M+Na]⁺.

### Intermediate 74

### [1-{[(1,1-Dimethylethyl)oxy]carbonyl}-4-(2-methylphenyl)-4-piperidinyl]acetic acid

A mixture of intermediate **73** (2.5 g) in acetic acid (28 mL), conc. sulfuric acid (9.25 mL) and water (9.25 mL) was heated to 140°C for 5 hours. The solution was allowed to cool to r.t., a 2.5M sodium hydroxide solution added, followed by sodium hydroxide pellets until pH 12. Then, di-*tert*-butyl-dicarbonate (2.0 g) was added and the resulting mixture was stirred at r.t. for 2 days. It was cooled to 0°C and treated with 6M hydrochloric acid solution until pH=6-7 and then extracted with AcOEt (3 x 30 mL). The organic phase was washed with brine, dried and concentrated *in vacuo*. The residue was purified by flash chromatography (CH/AcOEt 7:3) to give the title compound (1.05 g) as a pale yellow foam.
T.I.c.: CH/AcOEt 1:1, Rf=0.36 (detection with ninhydrine).
MS (ES/+): m/z=356 [M+Na]⁺.

### Intermediate 75

### 1,1-Dimethylethyl 4-{2-[[(3,5-dibromophenyl)methyl](methyl)amino]-2-oxoethyl}-4-(2-methylphenyl)-1-piperidinecarboxylate

DIPEA (240 µL) and O-(benzotriazol-1-yl)-N,N,N'N'-tetramethyluronium tetrafluoroborate (188 mg) were added to a solution of intermediate **74** (150 mg) in anhydrous DMF (3 mL) under a Nitrogen atmosphere. After stirring for 15 minutes, 3,5-dibromobenzyl-methylamine hydrochloride (156 mg) was added. The mixture was stirred at r.t. overnight, then it was diluted with AcOEt (10 mL) and washed with water (10 mL) and brine (10 mL). The organic layer was dried, concentrated *in vacuo* and the residue was purified by flash chromatography (CH/AcOEt 8:2) to give the title compound (235 mg) as a colourless oil.
T.I.c.: CH/AcOEt 1:1, Rf=0.51.
MS (ES/+): m/z=617 [M+Na]⁺.

### Intermediate 76

### 1,1-Dimethylethyl 4-{2-[{[3,5-bis(trifluoromethyl)phenyl]methyl}(methyl)amino]-2-oxoethyl}-4-(4-fluorophenyl)-1-piperidinecarboxylate

DIPEA (675 µL) and O-(benzotriazol-1-yl)-N,N,N'N'-tetramethyluronium tetrafluoroborate (525 mg) were added to a solution of intermediate **3** (426 mg) in anhydrous DMF (6 mL) under a Nitrogen atmosphere. After stirring for 15 minutes, {[3,5-bis(trifluoromethyl)phenyl]methyl}methylamine (370 mg) was added. The mixture was stirred at r.t. for 2 days, then it was diluted with DCM (14 mL), washed with 5% sodium hydrogen carbonate solution (12 mL), and passed through a phase separation cartridge with polypropylene frit and concentrated *in vacuo*. The residue was purified by SCX cartridge (load with DCM and elution with MeOH, NH₃ 0.2M in MeOH) to give the title compound (590 mg) as a yellow oil.
T.I.c.: CH/AcOEt 1:1, Rf=0.28 (detection with ninhydrine).

### Intermediate 77

### 1,1-Dimethylethyl 4-(2-{[1-(3,5-dibromophenyl)-1-methylethyl]amino}-2-oxoethyl)-4-(4-fluorophenyl)-1-piperidinecarboxylate

DIPEA (493 µL) and O-(benzotriazol-1-yl)-N,N,N'N'-tetramethyluronium tetrafluoroborate (383 mg) were added to a solution of intermediate **3** (310 mg) in anhydrous DMF (5 mL) under a Nitrogen atmosphere. After stirring for 15 minutes, intermediate **120** (303 mg) was added. The mixture was stirred at r.t. for 2 days, then it was diluted with DCM (12 mL), washed with 5% sodium hydrogen carbonate solution (12 mL), and passed through a phase separation cartridge with polypropylene frit and concentrated *in vacuo*. The residue was purified by flash chromatography (CH/AcOEt from 8:2 to 1:1) to give the title compound (312 mg) as a colourless oil.
T.I.c.: CH/AcOEt 1:1, Rf=0.35 (detection with ninhydrine).
MS (ES/+): m/z=635 [M+Na]⁺.

### Intermediate 78

### 1,1-Dimethylethyl 4-{2-[[1-(3,5-dibromophenyl)-1-methylethyl](methyl)amino]-2-oxoethyl}-4-(4-fluorophenyl)-1-piperidinecarboxylate

Intermediate **77** (502 mg) was dissolved in dry THF (15 mL) under a Nitrogen atmosphere and NaH (60% suspension in mineral oil, 131 mg) was added in small portions. The reaction mixture was stirred under these conditions for 10 minutes then methyl iodide ( 460 µL) was added, and the reaction was heated up to 50°C and kept at this temperature for total 30 hours. Then it was diluted with AcOEt (15 x 2 mL), washed with water (15 mL), the collected organic phases dried and concentrated *in vacuo*. The residue was purified by flash chromatography (CH/AcOEt from 8:2 to 7:3) to give the title compound (314 mg) as a white solid.
T.I.c.: CH/AcOEt 1:1, Rf=0.43 (detection with ninhydrine).
MS (ES/+): m/z=627 [M+H]⁺.

### Intermediate 79

### 4-({[1-(R)-1-(3,5-Bis-trifluoromethyl-phenyl)-ethyl]-methyl-carbamoyl}-methyl)-4-(4-fluoro-phenyl)-piperidine-1-carboxylic acid tert-butyl ester

DIPEA (675 µL) and O-(benzotriazol-1-yl)-N,N,N'N'-tetramethyluronium tetrafluoroborate (525 mg) were added to a solution of intermediate **3** (426 mg) in anhydrous DMF (6 mL) under a Nitrogen atmosphere. After stirring for 15 minutes, [1-(*R*)-(3,5-bis-trifluoromethylphenyl)-ethyl]-methyl-amine hydrochloride (387 mg) was added. The mixture was stirred at r.t. for 2 days, then it was diluted with DCM (14 mL), washed with 5% sodium hydrogen carbonate solution (12 mL), and passed through a phase separation cartridge with polypropylene frit and concentrated *in vacuo*. The residue was purified by SCX cartridge (load with DCM and elution with MeOH, NH₃ 0.2M in MeOH) to give the title compound (700 mg) as a yellow oil.
T.I.c.: CH/AcOEt 1:1, Rf=0.59 (detection with ninhydrine).

### Intermediate 80

### 1,1-Dimethylethyl 4-{2-[{(1R)-1-[3,5-bis(trifluoromethyl)phenyl]ethyl}(methyl)amino]-2-oxoethyl}-4-(4-fluorophenyl)hexahydro-1H-azepine-1-carboxylate

DIPEA (223 µL) and O-(benzotriazol-1-yl)-N,N,N'N'-tetramethyluronium tetrafluoroborate (180 mg) were added to a solution of intermediate **36** (150 mg) in anhydrous DMF (5 mL) under a Nitrogen atmosphere at r. t. After stirring for 30 minutes [1-(*R*)-(3,5-bis-trifluoromethyl-phenyl)-ethyl]-methyl-amine hydrochloride (150 mg) was added. The mixture was stirred at r.t. overnight, then it was taken up with AcOEt (30 mL), washed with water (4 x 5 mL) and brine (2 x 5 mL). The organic layer was dried, concentrated *in vacuo* and the residue was purified by flash chromatography (CH/AcOEt 7:3) to give the title compound (210 mg) as a white foam.
T.I.c.: CH/AcOEt 6:4, Rf=0.3 (detection with ninhydrine).
MS (ES/+): m/z=627 [M+Na]⁺.

### Intermediate 81

### 1,1-Dimethylethyl 4-{2-[[(3,5-dibromophenyl)methyl](methyl)amino]-2-oxoethyl}-4-(4-fluorophenyl)hexahydro-1H-azepine-1-carboxylate

DIPEA (150 □L) and O-(benzotriazol-1-yl)-N,N,N'N'-tetramethyluronium tetrafluoroborate (120 mg) were added to a solution of intermediate **36** (100 mg) in anhydrous DMF (5 mL) under a Nitrogen atmosphere at r.t. After stirring for 30 minutes [(3,5-dibromophenyl)methyl]methylamine hydrochloride (100 mg) was added. The mixture was stirred at r.t. overnight, then it was taken up with AcOEt (30 mL) and washed with water (4 x 5 mL) and brine (2 x 5 mL). The organic layer was dried, concentrated *in vacuo* and the residue was purified by flash chromatography (CH/AcOEt 7:3) to give the title compound (170 mg) as a white foam.
T.I.c.: CH/AcOEt 7:3, Rf=0.3 (detection with ninhydrine).
MS (ES/+): m/z=635 [M+Na]⁺.

### Intermediate 82

### 3-Bromo-5-formylbenzonitrile

A mixture of 3,5-dibromobenzaldehyde (1 g), copper cyanide (0.331 g) and pyridine (0.3 mL) in DMF (30 mL) was heated to 140°C overnight. The solution was allowed to cool to r.t. and dropped into water (30 ml), then exctracted with Et2O (15 mL). The organic phase was dried, concentrated *in vacuo* and the residue was purified by flash chromatography (CH, CH/AcOEt from 95:5 to 80:20) to give the title compound (64 mg) as a yellow oil.
T.I.c.: CH/AcOEt 8:2, Rf=0.52 (detection with 2,4-dinitrophenylhydrazine).
NMR (CDCl₃): δ (ppm) 10.0 (s, 1H); 8.20 (s, 1H); 8.10 (s, 1H); 8.00 (s, 1H).
IR (nujol, cm⁻¹): 2235.70 (C≡N); 1700.14 (C=O).

### Intermediate 83

### 3-Bromo-5-(methylamino)benzonitrile

A solution of methylamine (2M in MeOH - 1.5 mL) was dropped into a solution of intermediate **82** (0.640 g) in anhydrous MeOH (3 mL) under a Nitrogen atmosphere. The mixture was stirred at r. t. for 2 hours, then after cooling at 0 °C, potassium borohydride (0.160 mg) was added stepwise. The solvent was removed *in vacuo*, the residue diluted with water (5 mL) and extracted with AcOEt (2 x 5 mL). The organic phase was dried, concentrated *in vacuo* and the residue was purified by SCX cartridge (load with MeOH and elution with NH₃ 0.25M in MeOH) to give the title compound (48 mg) as a yellow oil.
T.I.c.: DCM/MeOH 8:2, Rf=0.61 (detection with ninhydrine).
MS (ES/+): m/z=225 [M+H]⁺.

### Intermediate 84

### 1,1-Dimethylethyl 4-{2-[[(3-bromo-5-cyanophenyl)methyl](methyl)amino]-2-oxoethyl}-4-(4-fluorophenyl)-1-piperidinecarboxylate

Intermediate 83 was added to a solution of triethylamine (90 µL), O-(7-Azabenzotriazol-1-yl)-N,N,N'N'-tetramethyluroniumhexafluorophosphate (105 mg) and intermediate **3** (71 mg) in anhydrous DCM (3 mL) at r.t. under a Nitrogen atmosphere. After stirring the mixture for 1 hour intermediate **83** (48 mg) was added, the resulting mixture stirred at r.t. overnight, and then diluted with a saturated sodium hydrogen carbonate solution (3 mL). The organic layer was passed through a phase separation cartridge with polypropylene frit and concentrated *in vacuo*. The residue was purified by SCX cartridge (load with MeOH and elution with NH₃ 0.25M in MeOH) to give the title compound (54 mg) as a yellow oil.
T.I.c.: CH/AcOEt 3:7, Rf=0.19 (detection with ninhydrine).
MS (ES/+): m/z=568 [M+Na]⁺.

### Intermediate 85

### 1,1-Dimethylethyl 4-[1-cyano-2-(ethyloxy)-2-oxoethyl]-4-[3-(trifluoromethyl)phenyl]-1-piperidinecarboxylate

A solution of 1-bromo-3-(trifluoromethyl)benzene (2.8 mL) in anhydrous THF (5.5 mL) was dropped into a suspension of magnesium turning (0.55 g) and few crystals of iodine in anhydrous THF (10 mL) under a Nitrogen atmosphere. The mixture was refluxed for 30 minutes, then it was allowed to cool to r.t. and added dropwise to a mixture of intermediate **1** (2 g) and copper iodide (0.77 g) in anhydrous THF (16.5 mL) previously cooled to 0°C under a Nitrogen atmosphere. The mixture was stirred under these conditions for 1 hour and then allowed to warm to r.t. and stirred at 23°C for 2 hours. The mixture was cooled to 0°C, treated with 3M hydrochloric acid solution and extracted with AcOEt (3 x 200 mL). The combined organic extracts were washed with a saturated ammonium chloride solution (200 mL), dried and concentrated *in vacuo*. The residue was purified by flash chromatography (CH/toluene/AcOEt 7:1:2) to give the title compound (2.0 g) as a colourless oil.
MS (ES/+): m/z=463 [M+Na]⁺.

### Intermediate 86

### {1-{[(1,1-Dimethylethyl)oxy]carbonyl}-4-[3-(trifluoromethyl)phenyl]-4-piperidinyl}acetic acid

A mixture of intermediate **85** (1.95 g) in acetic acid (21.6 mL), conc. sulfuric acid (7 mL) and water (7.2 mL) was heated to 140°C overnight. The solution was allowed to cool to 0°C and 2.5 M sodium hydroxide solution was dropped until pH reached 14. Then, di-tert-butyl-dicarbonate (1.4 g) was added and the resulting mixture was stirred at r.t. overnight.

It was extracted with Et2O (2 x 20 mL). Then the aqueous solution was treated with pH 3 buffer and 6M hydrochloric acid solution to pH=4, extracted with AcOEt then washed with brine. The organic phase was dried, concentrated *in vacuo* and the residue was purified by flash chromatography (CH/AcOEt from 95:5 to 1:1) to give the title compound (1 g) as a dark yellow foam.
T.I.c.: AcOEt, Rf=0.7 (detection with ninhydrine).
MS (ES/+): m/z=410 [M+Na]⁺.

### Intermediate 87

### 1,1-Dimethylethyl 4-{2-[[(3,5-dibromophenyl)methyl](methyl)amino]-2-oxoethyl}-4-[3-(trifluoromethyl)phenyl]-1-piperidinecarboxylate

DIPEA (0.202 mL) and O-(benzotriazol-1-yl)-N,N,N'N'-tetramethyluronium tetrafluoroborate (162 mg) were added to a solution of intermediate **86** (150 mg) in anhydrous DMF (6 mL) under a Nitrogen atmosphere. After stirring for 15 minutes, [(3,5-dibromophenyl)methyl]methylamine hydrochloride (134 mg) was added. The mixture was stirred at r.t. for 3 days, then it was diluted with AcOEt and washed with ice cold water.
The organic layer was dried, concentrated *in vacuo* and the residue was purified by flash chromatography (CH/AcOEt 1:1) to give the title compound (270 mg) as a colourless oil.
T.I.c.: CH/AcOEt 1:1, Rf=0.6 (detection with ninhydrine).
MS (ES/+): m/z=671 [M+Na]⁺.

### Intermediate 88

### 1,1-Dimethylethyl 4-[1-cyano-2-(ethyloxy)-2-oxoethyl]-4-(3,4-dimethylphenyl)-1-piperidinecarboxylate

A solution of 4-bromo-1,2-dimethylbenzene (2.75 mL) in anhydrous THF (5.5 mL) was dropped into a suspension of magnesium turning (0.55 g) and few crystals of iodine in anhydrous THF (10 mL) under a Nitrogen atmosphere. The mixture was refluxed for 30 minutes, then it was allowed to cool to r.t. and added dropwise to a mixture of intermediate **1** (2 g) and copper iodide (0.77 g) in anhydrous THF (16.5 mL) previously cooled to 0°C under a Nitrogen atmosphere. The mixture was stirred under these conditions for 1 hour and then allowed to warm to r.t. and stirred at 23°C for 2 hours. The mixture was cooled to 0°C, treated with 3M hydrochloric acid solution and extracted with AcOEt (3 x 200 mL). The combined organic extracts were washed with a saturated ammonium chloride solution (200 mL), dried and concentrated *in vacuo*. The residue was purified by flash chromatography (CH/toluene/AcOEt 7:1:2) to give the title compound (2.7 g).
MS (ES/+): m/z=423 [M+Na]⁺.

### Intermediate 89

### [1-{[(1,1-Dimethylethyl)oxy]carbonyl}-4-(3,4-dimethylphenyl)-4-piperidinyl]acetic acid

A mixture of intermediate **88** (2.65 g) in acetic acid (29.4 mL), conc. sulfuric acid (9.5 mL) and water (9.8 mL) was heated to 140°C overnight. The solution was allowed to cool to 0°C and 2.5M sodium hydroxide solution was dropped until pH reached 14. Then, di-tert-butyl-dicarbonate (2.1 g) was added and the resulting mixture was stirred at r.t. overnight. It was extracted with Et2O (2 x 20 mL). Then the aqueous solution was treated with pH 3 buffer and 6M hydrochloric acid solution to pH=5, extracted with AcOEt then washed with brine solution. The organic phase was dried, concentrated *in vacuo* and the residue was purified by flash chromatography (CH/AcOEt from 95:5 to 1:1) to give the title compound (1.4 g) as brownish oil.
T.I.c.: AcOEt, Rf=0.8 (detection with ninhydrine).
MS (ES/+): m/z=348 [M+H]⁺.

### Intermediate 90

### 1,1-Dimethylethyl 4-{2-[[(3,5-dibromophenyl)methyl](methyl)amino]-2-oxoethyl}-4-(3,4-dimethylphenyl)-1-piperidinecarboxylate

DIPEA (0.226 mL) and O-(benzotriazol-1-yl)-N,N,N'N'-tetramethyluronium tetrafluoroborate (180 mg) were added to a solution of intermediate **89** (150 mg) in anhydrous DMF (6 mL) under a Nitrogen atmosphere. After stirring for 15 minutes, [(3,5-dibromophenyl)methyl]methylamine hydrochloride (150 mg) was added. The mixture was stirred at r.t. for 3 days, then it was diluted with AcOEt and washed with ice-cold water.
The organic layer was dried, concentrated *in vacuo* and the residue was purified by flash chromatography (CH/AcOEt 1:1) to give the title compound (262 mg) as a colourless oil.
T.I.c.: CH/AcOEt 1:1, Rf=0.5 (detection with ninhydrine).
MS (ES/+): m/z=631 [M+Na]⁺.

### Intermediate 91

### 1,1-Dimethylethyl 4-[1-cyano-2-(ethyloxy)-2-oxethyl]-4-(3-fluorophenyl)-1-piperidinecarboxylate

A solution of 1-bromo-3-fluorobenzene (2.24 mL) in anhydrous THF (5.5 mL) was dropped into a suspension of magnesium turning (0.55 g) and few crystals of iodine in anhydrous THF (10 mL) under a Nitrogen atmosphere. The mixture was refluxed for 30 minutes, then it was allowed to cool to r.t. and added dropwise to a mixture of intermediate 1 (2 g) and copper iodide (0.776 g) in anhydrous THF (16.5 mL) previously cooled to 0°C under a Nitrogen atmosphere. The mixture was stirred under these conditions for 1 hour and then allowed to warm to r.t. and stirred at 23°C for 2 hours. The mixture was cooled to 0°C, treated with 3M hydrochloric acid solution and extracted with AcOEt (3 x 200 mL). The combined organic extracts were washed with a saturated ammonium chloride solution (200 mL), dried and concentrated *in vacuo*. The residue was purified by flash chromatography (CH/AcOEt from 9:1 to 6:4) to give the title compound (1.86 g).
MS (ES/+): m/z=413 [M+Na]⁺.

### Intermediate 92

### [1-{[(1,1-Dimethylethyl)oxy]carbony}-4-(3-fluorophenyl)-4-piperidinyl]acetic acid

A mixture of intermediate **91** (1.86 g) in acetic acid (3.6 mL), conc. sulfuric acid (1.2 mL) and water (1.2 mL) was heated to 140°C for 13 hours. The solution was allowed to cool to 0°C and 2.5M sodium hydroxide solution was dropped until pH reached 14. Then, di-tert-butyl-dicarbonate (2.01 g) was added and the resulting mixture was stirred at r.t. overnight. It was extracted with Et2O (2 x 20 mL). Then the aqueous solution was treated with pH 3 buffer and 6M hydrochloric acid solution to pH=4, extracted with AcOEt then washed with brine solution. The organic phase was dried, concentrated *in vacuo* and the residue was purified by flash chromatography (CH/AcOEt 6:4) to give the title compound (1.13 g) as an off-white oil.
T.I.c.: CH/AcOEt 1:1, Rf=0.4 (detection with ninhydrine).
MS (ES/+): m/z=360 [M+Na]⁺.

### Intermediate 93

### 1,1-Dimethylethyl 4-{2-[[1-(3,5-dichlorophenyl)ethyl](methyl)amino]-2-oxoethyl}-4-(3-fluorophenyl)-1-piperldinecarboxylate (enantiomer 1)

DIPEA (87 µL) and O-(benzotriazol-1-yl)-N,N,N'N'-tetramethyluronium tetrafluoroborate (102 mg) were added to a solution of intermediate **92** (92 mg) in anhydrous DMF (3 mL) under a Nitrogen atmosphere. After stirring for 15 minutes intermediate **54** (50 mg) was added. The mixture was stirred at r.t. for 2 days, then it was diluted with DCM (12 mL), washed with 5% sodium hydrogen carbonate solution (12 mL), and passed through a phase separation cartridge with polypropylene frit and concentrated *in vacuo*. The residue was purified by flash chromatography (CH/AcOEt from 8:2 to 1:1) to give the title compound (135 mg) as a colourless oil. T.I.c.: CH/AcOEt 1:1, Rf=0.73 (detection with ninhydrine).
MS (ES/+): m/z=545 [M+Na]⁺.

### Intermediate 94

### 1,1-Dimethylethyl 4-[1-cyano-2-(ethyloxy)-2-oxoethyl]-4-(3,4-difluorophenyl)-1-piperidinecarboxylate

A solution of 4-bromo-1,2-difluorobenzene (2.304 mL) in anhydrous THF (5.5 mL) was dropped into a suspension of magnesium turning (0.55 g) and few crystals of iodine in anhydrous THF (10 mL) under a Nitrogen atmosphere. The mixture was refluxed for 30 minutes, then it was allowed to cool to r.t. and added dropwise to a mixture of intermediate 1 (2 g) and copper iodide (0.776 g) in anhydrous THF (16.5 mL) previously cooled to 0°C under a Nitrogen atmosphere. The mixture was stirred under these conditions for 1 hour and then allowed to warm to r.t. and stirred at 23°C for 2 hours. The mixture was cooled to 0°C, treated with 3M hydrochloric acid solution and extracted with AcOEt (3 x 200 mL). The combined organic extracts were washed with a saturated ammonium chloride solution (200 mL), dried and concentrated *in vacuo*. The residue was purified by flash chromatography (CH/AcOEt from 9:1 to 6:4) to give the title compound (2.33 g).
MS (ES/+): m/z=431 [M+Na]⁺.

### Intermediate 95

### [1-{[(1,1-Dimethylethyl)oxy]carbonyl}-4-(3,4-difiuorophenyl)-4-piperidinyl]acetic acid

A mixture of intermediate **91** (2.33 g) in acetic acid (4.5 mL), conc. sulfuric acid (1.5 mL) and water (1.5 mL) was heated to 140°C for 13 hours. The solution was allowed to cool to 0°C and 2.5M sodium hydroxide solution was dropped until pH reached 14. Then, di-tert-butyl-dicarbonate (2.407 g) was added and the resulting mixture was stirred at r.t. overnight. It was extracted with Et2O (2 x 20 mL). Then the aqueous solution was treated with pH 3 buffer and 6M hydrochloric acid solution to pH=4, extracted with AcOEt then washed with brine solution. The organic phase was dried, concentrated *in vacuo* and the residue was purified by flash chromatography (CH/AcOEt 6:4) to give the title compound (1.6 g) as brownish oil.
T.I.c.: CH/AcOEt 1:1, Rf=0.4 (detection with ninhydrine).
MS (ES/+): m/z=378 [M+Na]⁺.

### Intermediate 96

### 1,1-Dimethylethyl 4-{2-[[1-(3,5-dichlorophenyl)ethyl](methyl)amino]-2-oxoethyl}-4-(3,4-difluorophenyl)-1-piperidinecarboxylate (enantiomer 1)

DIPEA (87 µL) and O-(benzotriazol-1-yl)-N,N,N'N'-tetramethyluronium tetrafluoroborate (102 mg) were added to a solution of intermediate **95** (87 mg) in anhydrous DMF (3 mL) under a Nitrogen atmosphere. After stirring for 15 minutes, intemediate **54** (50 mg) was added. The mixture was stirred at r.t. for 2 days, then it was diluted with DCM (12 mL), washed with 5% sodium hydrogen carbonate solution (12 mL), and passed through a phase separation cartridge with polypropylene frit and concentrated *in vacuo*. The residue was purified by flash chromatography (CH/AcOEt from 8:2 to 1:1) to give the title compound (135 mg) as a colourless oil.
T.I.c.: CH/AcOEt 1:1, Rf=0.69 (detection with ninhydrine).
MS (ES/+): m/z=563 [M+Na]⁺.

### Intermediate 97

### 1,1-Dimethylethyl 4-{2-[[1-(3,5-dibromophenyl)ethyl](methyl)amino]-2-oxoethyl}-4-(3,4-difluorophenyl)-1-piperidinecarboxylate (enantiomer 1)

DIPEA (117 µL) and O-(benzotriazol-1-yl)-N,N,N'N'-tetramethyluronium tetrafluoroborate (130 mg) were added to a solution of intermediate **95** (120 mg) in anhydrous DMF (4 mL) under a Nitrogen atmosphere. After stirring for 30 minutes intermediate **56** (100 mg) was added. The mixture was stirred at r.t. overnight, then it was taken up with AcOEt (20 mL) and washed with water (4 x 20 mL). The organic layer was dried, concentrated *in vacuo* and the residue was purified by flash chromatography (CH/AcOEt 85/15, then 70/30) to give the title compound (197 mg) as a white foam.
T.I.c.: CH/AcOEt 6:4, Rf=0.48 (detection with ninhydrine). MS (ES/+): m/z=653 [M+Na]⁺.

### Intermediate 98

### 1,1-Dimethylethyl 4-{2-[[1-(3,5-dibromophenyl)ethyl](methyl)amino]-2-xoethyl}-4-(3,4-difluorophenyl)-1-piperidinecarboxylate (enantiomer 2)

DIPEA (117 µL) and O-(benzotriazot-1-yl)-N,N,N'N'-tetramethyluronium tetrafluoroborate (130 mg) were added to a solution of intermediate **95** (120 mg) in anhydrous DMF (4 mL) under a Nitrogen atmosphere. After stirring for 30 minutes intermediate **57** (100 mg) was added. The mixture was stirred at r.t. overnight, then it was taken up with AcOEt (20 mL) and washed with water (4 x 20 mL). The organic layer was dried, concentrated *in vacuo* and the residue was purified by flash chromatography (CH/AcOEt 85/15, then 70/30) to give the title compound (188 mg) as a white foam.
T.I.c.: CH/AcOEt 6:4, Rf=0.48 (detection with ninhydrine).
MS (ES/+): m/z=653 [M+Na]⁺.

### Intermediate 99

### 1,1-Dimethylethyl 4-{2-[[1-(3,5-dibromophenyl)ethyl](methyl)amino]-2-xoethyl}-4-(3-fluorophenyl)-1-piperidinecarboxylate (enantiomer 1)

DIPEA (124 µL) and O-(benzotriazol-1-yl)-N,N,N'N'-tetramethyluronium tetrafluoroborate (137 mg) were added to a solution of intermediate **92** (120 mg) in anhydrous DMF (4 mL) under a Nitrogen atmosphere. After stirring for 30 minutes intermediate **56** (106 mg) was added. The mixture was stirred at r.t. overnight, then it was taken up with AcOEt (20 mL) and washed with water (4 x 20 mL). The organic layer was dried, concentrated *in vacuo* and the residue was purified by flash chromatography (CH/AcOEt 85/15, then 70/30) to give the title compound (219 mg) as a white foam.
T.I.c.: CH/AcOEt 6:4, Rf=0.48 (detection with ninhydrine).
MS (ES/+): m/z=635 [M+Na]⁺.

### Intermediate 100

### 1,1-Dimethylethyl 4-[1-cyano-2-(ethyloxyl)-2-oxoethyl]-4-(4-fluoro-3-methylphenyl)-1-piperidinecarboxylate

A solution of 4-fluoro 3-methylphenyl magnesium bromide (1M in THF, 20.38 mL) was added drop-wise to a mixture of intermediate 1 (2 g) and copper iodide (0.77 g) in anhydrous THF (17 mL) previously cooled to 0°C under a Nitrogen atmosphere. The mixture was stirred under these conditions for 1 hour and then allowed to warm to r.t. and stirred at 23°C for 2 hours. The mixture was cooled to 0°C, treated with saturated ammonium chloride solution and few drops of conc. NH₄OH solution and extracted with AcOEt (3 x 200 mL). The combined organic extracts were dried and concentrated *in vacuo*. The residue was purified by flash chromatography (CH/AcOEt 8:2) to give the title compound (2.13 g) as a yellow oil.
T.I.c.: CH/AcOEt 8:2, Rf=0.3 (detection with ninhydrine).
MS (ES/+): m/z=427 [M+Na]⁺.

### Intermediate 101

### [1-{[(1,1-Dimethylethyl)oxy]carbonyl}-4-(4-fluoro-3-methylphenyl)-4-piperidinyl]acetic acid

A mixture of intermediate **100** (2.16 g) in acetic acid (4.5 mL), conc. sulfuric acid (1.5 mL) and water (1.5 mL) was heated to 140°C overnight. The solution was cooled to 0°C and a 2.5M sodium hydroxide solution was dropped until pH reached 14. Then, di-tert-butyl-dicarbonate (1.75 g) was added and the resulting mixture was stirred at r.t. overnight. It was treated with a saturated sodium hydrogen carbonate solution and then extracted with Et2O. Aqueous solution was treated with hydrochloric acid 2.5 M to pH 4 and extracted with AcOEt. The organic phase was dried, concentrated *in vacuo* and the residue was purified by flash chromatography (CH/AcOEt 7:3) to give the title compound (450 mg) as a white solid.
T.I.c.: AcOEt, Rf=0.7 (detection with ninhydrine).
MS (ES/-): m/z=350 [M-H]⁻.

### Intermediate 102

### 1,1-Dimethylethyl 4-{2-[[1-(3,5-dibromophenyl)ethyl](methyl)amino]-2-oxoethyl}-4-(4-fluoro-3-methylphenyl)-1-piperidinecarboxylate (enantiomer 1)

DIPEA (109 µL) and O-(benzotriazol-1-yl)-N,N,N'N'-tetramethyluronium tetrafluoroborate (120 mg) were added to a solution of intermediate **101** (110 mg) in anhydrous DMF (4 mL) under a Nitrogen atmosphere. After stirring for 30 minutes intermediate **56** (94 mg) was added. The mixture was stirred at r.t. overnight, then it was taken up with AcOEt (20 mL) and washed with water (4 x 20 mL). The organic layer was dried, concentrated *in vacuo* and the residue was purified by flash chromatography (CH/AcOEt 85/15, 70/30) to give the title compound (160 mg) as a white foam.
T.I.c.: CH/AcOEt 6:4, Rf=0.45 (detection with ninhydrine).
MS (ES/+): m/z=649 [M+Na]⁺.

### Intermediate 103

### 1,1-Dimethylethyl 4-(3-chlorophenyl)-4-[1-cyano-2-(ethyloxy)-2-oxoethyl]-1-piperidinecarboxylate

A solution of 1-bromo-3-chlorobenzene (2.39 mL) in anhydrous THF (11.5 mL) was dropped into a suspension of magnesium turning (0.55 g) and few crystals of iodine in anhydrous THF (5 mL) under a Nitrogen atmosphere. The mixture refluxed for 30 minutes, then it was allowed to cool to r.t. and added drop-wise to a mixture of intermediate **1** (2 g) and copper iodide (0.77 g) in anhydrous THF (17 mL) previously cooled to 0°C under a Nitrogen atmosphere. The mixture was stirred under these conditions for 1 hour and then allowed to warm to r.t. and stirred at 23°C for 2 hours. The mixture was cooled to 0°C, treated with saturated ammonium chloride solution and few drops of conc. NH₄OH solution and extracted with AcOEt (3 x 200 mL). The combined organic extracts were concentrated *in vacuo*. The residue was purified by flash chromatography (CH/AcOEt 8:2) to give the title compound (2.06 g) as a yellow oil.
T.I.c.: CH/AcOEt 8:2, Rf=0.2 (detection with ninhydrine).
MS (ES/+): m/z=429 [M+Na]⁺.

### Intermediate 104

### (4-(3-Chlorophenyl)-1-{[(1,1-dimethylethyl)oxy]carbonyl}-4-piperidinyl)acetic acid

A mixture of intermediate **103** (2.06 g) in acetic acid (4.5 mL), conc. sulfuric acid (1.5 mL) and water (1.5 mL) was heated to 140°C for 10 hours. The solution was allowed to cool to 0°C and a 2.5M sodium hydroxide solution was dropped until pH reached 14. Then, di-tert-butyl-dicarbonate (1.66 g) was added and the resulting mixture was stirred at r.t. overnight. It was treated with saturated sodium hydrogen carbonate solution and then extracted with Et2O. The aqueous phase was treated with 2.5M hydrochloric acid to pH 4 and extracted with AcOEt. The organic phase was dried, concentrated *in vacuo* and the residue was purified by flash chromatography (CH/AcOEt 7:3) to give the title compound (380 mg) as a white solid.
T.I.c.: AcOEt, Rf=0.64 (detection with ninhydrine).
MS (ES/-): m/z=352 [M-H]⁻.

### Intermediate 105

### 1,1-Dimethylethyl 4-(3-chlorophenyl)-4-{2-[[1-(3,5-dibromophenyl)ethyl](methyl)amino]-2-oxoethyl}-1-piperidinecarboxylate (enantiomer 1)

DIPEA (90 µL) and O-(benzotriazol-1-yl)-N,N,N'N'-tetramethyluronium tetrafluoroborate (98 mg) were added to a solution of intermediate **104** (90 mg) in anhydrous DMF (4 mL) under a Nitrogen atmosphere. After stirring for 30 minutes intermediate **56** (76 mg) was added. The mixture was stirred at r.t. overnight, then it was taken up with AcOEt (20 mL) and washed with water (4 x 20 mL). The organic layer was dried, concentrated *in vacuo* and the residue was purified by flash chromatography (CH/AcOEt 85:1.5, then 70:30) to give the title compound (147 mg) as a white foam.
T.I.c.: CH/AcOEt 6:4, Rf=0.49 (detection with ninhydrine).
MS (ES/+): m/z=651 [M+Na]⁺.

### Intermediate 106

### 1,1-Dimethylethyl 4-(3-chlorophenyl)-4-{2-[[1-(3,5-dichlorophenyl)ethy](methyl)amino]-2-xoethyl}-1-piperidinecarboxylate (enantiomer 1)

DIPEA (80 µL) and O-(benzotriazol-1-yl)-N,N,N'N'-tetramethyluronium tetrafluoroborate (96 mg) were added to a solution of intermediate **104** (80 mg) in anhydrous DMF (3 mL) under a Nitrogen atmosphere. After stirring for 20 minutes intermediate **54** (52 mg) was added. The mixture was stirred at r.t. overnight, then it was diluted with AcOEt and washed with ice/water and brine. The organic layer was dried, concentrated *in vacuo* and the residue was purified by flash chromatography (CH/AcOEt 8:2) to give the title compound (100 mg) as a colourless oil.
T.I.c.: CH/AcOEt 6:4, Rf=0.53 (detection with ninhydrine).
MS (ES/+): m/z= 561 [M+Na]⁺.

### Intermediate 107

### 1,1-Dimethylethyl 4-(3-chlorophenyl)-4-{2-[[(3,5-dibromophenyl)methyl]methyl)amino]-2-oxoethyl}-1-piperidinecarboxylate

DIPEA (148 µL) and O-(benzotriazol-1-yl)-N,N,N'N'-tetramethyluronium tetrafluoroborate (118 mg) were added to a solution of intermediate **104** (100 mg) in anhydrous DMF (4 mL) under a Nitrogen atmosphere. After stirring for 15 minutes, [(3,5-dibromophenyl)methyl]methylamine hydrochloride (98 mg) was added. The mixture was stirred at r.t. overnight, then it was diluted with AcOEt and washed with ice cold water.

The organic layer was dried, concentrated *in vacuo* and the residue was purified by flash chromatography (CH/AcOEt from 95:5 to 70:30) to give the title compound (170 mg) as a pale yellow foam.
T.I.c.: CH/AcOEt 7:3, Rf=0.25 (detection with ninhydrine).
MS (ES/+): m/z=637 [M+Na]⁺.

### Intermediate 108

### 1,1-Dimethylethyl 4-{2-[[1-(3,5-dichlorophenyl)ethyl](methyl)amino]-2-xoethyl}-4-(4-fluoro-3-methylphenyl)-1-piperidinecarboxylate (enantiomer 1)

DIPEA (104 µL) and O-(benzotriazol-1-yl)-N,N,N'N'-tetramethyluronium tetrafluoroborate (145 mg) were added to a solution of intermediate **101** (105 mg) in anhydrous DMF (3 mL) under a Nitrogen atmosphere. After stirring for 20 minutes intermediate **54** (68 mg) was added. The mixture was stirred at r.t. overnight, then it was diluted with AcOEt and washed with ice/water and brine. The organic layer was dried, concentrated *in vacuo* and the residue was purified by flash chromatography (CH/AcOEt 8:2) to give the title compound (103 mg) as a colourtess oil.
T.I.c.: CH/AcOEt 6:4, Rf=0.43 (detection with ninhydrine).
MS (ES/+): 559 [M+Na]⁺.

### Intermediate 109

### 1,1-Dimethylethyl 4-{2-[[(3,5-dibromophenyl)methyl](methyl)amino]-2-oxoethyl}-4-(4-fluoro-3-methylphenyl)-1-piperidinecarboxylate

DIPEA (149 µL) and Q-(benzotriazol-1-yl)-N,N,N'N'-tetramethyluronium tetrafluoroborate (119 mg) were added to a solution of intermediate 101 (100 mg) in anhydrous DMF (4 mL) under a Nitrogen atmosphere. After stirring for 15 minutes, [(3,5-dibromophenyl)methyl]methylamine hydrochloride (99 mg) was added. The mixture was stirred at r.t. overnight, then it was diluted with AcOEt and washed with ice-cold water. The organic layer was dried, concentrated *in vacuo* and the residue was purified by flash chromatography (CH/AcOEt from 95:5 to 70:30) to give the title compound (167 mg) as a yellow foam.
T.I.c.: CH/AcOEt 7:3, Rf=0.13 (detection with ninhydrine).
MS (ES/+): m/z=635 [M+Na]⁺.

### Intermediate 110

### 1,1-Dimethylethyl 4-{2-[[(3,5-dibromophenyl)methyl](methyl)amino]-2-oxoethyl}-4-(3-fluorophenyl)-1-piperdinecarboxylate

DIPEA (238 µL) and O-(benzotriazol-1-yl)-N,N,N'N'-tetramethyluronium tetrafluoroborate (186 mg) were added to a solution of intermediate **92** (150 mg) in anhydrous DMF (4 mL) under a Nitrogen atmosphere. After stirring for 15 minutes, [(3,5-dibromophenyl)methyl]methylamine hydrochloride (140 mg) was added. The mixture was stirred at r.t. for 2 days, then it was diluted with DCM (12 mL), washed with 5% sodium hydrogen carbonate solution (12 mL), and passed through a phase separation cartridge with polypropylene frit and concentrated *in vacuo*. The residue was purified by flash chromatography (CH/AcOEt from 8:2 to 6:4) to give the title compound (270 mg) as a colourtess oil.
T.I.c.: CH/AcOEt 1:1, Rf=0.38 (detection with ninhydrine).
MS (ES/+): m/z=621 [M+Na]⁺.

### Intermediate 111

### 1,1-Dimethylethyl 4-{2-[[(3,5-dibromophenyl)methyl](methyl)amino]-2-oxoethyl}-4 (3,4-difluorophenyl)-1-piperidinecarboxylate

DIPEA (226 µL) and O-(benzotriazol-1-yl)-N,N,N'N'-tetramethyluronium tetrafluoroborate (176 mg) were added to a solution of intermediate **95** (150 mg) in anhydrous DMF (4 mL) under a Nitrogen atmosphere. After stirring for 15 minutes, [(3,5-dibromophenyl)methyl]methylamine hydrochloride (133 mg) was added. The mixture was stirred at r.t. for 2 days, then it was diluted with DCM (12 mL), washed with 5% sodium hydrogen carbonate solution (12 mL), and passed through a phase separation cartridge with polypropylene frit and concentrated *in vacuo*. The residue was purified by flash chromatography (CH/AcOEt from 8:2 to 6:4) to give the title compound (240 mg) as a colourless oil.
T.I.c.: CH/AcOEt 1:1, Rf=0.33 (detection with ninhydrine).
MS (ES/+): m/z=639 [M+Na]⁺.

### Intermediate 112

### 1,1-Dimethylethyl 4-(2,2-dimethyl-4,6-dioxo-1,3-dioxan-5-ylidene)-1-piperidinecarboxylate

A round bottom flask was charged with *tert*-butyl-4-oxo-1-piperidine carboxylate (5.0 g), 2,2-dimethyl-1,3-dioxane-4,6-diorie (Meldrum's acid) (3.65 g), ammonium acetate (0.100 g) and acetic acid (0.300 mL) in anhydrous toluene (20 mL) over molecular sieves (3A, 3.65 g). The mixture was stirred for 3 days at r.t. then sieves were filtered off and washed with AcOEt (300 mL). The organic solution was dried and concentrated *in vacuo* to a residue, which was purified by flash chromatography (DCM, DCM/MeOH 99:1) to give the title compound (2.6 g) as a white solid.
T.I.c.: DCM/MeOH 99:1, Rf=0.4 (detection with ninhydrine).
NMR (CDCl₃): δ (ppm) 3.6 (t, 4H); 3.1 (t, 4H); 1.7 (s, 6H); 1.45 (s, 9H).
MS (ES/-): m/z=324 [M-H]⁻.

### Intermediate 113

### 1,1-Dimethylethyl 4-(2,2-dimethyl-4,6-dioxo-1,3-dioxan-5-yl)-4-(4-fluorophenyl)-1-piperidinecarboxylate

A solution of 4-fluorophenyl magnesium bromide (1M in THF - 2.7 mL) was added drop-wise to a mixture of intermediate **112** (0.30 g) and copper iodide (0.10 g) in anhydrous THF (3 mL) previously cooled to 0°C under a Nitrogen atmosphere. The mixture was stirred under these conditions for 1 hour and then allowed to warm to r.t. and stirred at 23°C for 2 hours. The mixture was cooled to 0°C, treated with ammonium chloride saturated solution and few drops of conc. NH₄OH solution and extracted with AcOEt (3 x 20 mL). The combined organic extracts were dried and concentrated *in vacuo*. The residue was purified by flash chromatography (DCM/MeOH 99:1) to give the title compound (0.17 g) as a off-white solid.
T.I.c.: CH/AcOEt 1:1, Rf=0.4 (detection with ninhydrine).
NMR (CDCl₃): δ (ppm) 7.3-7.2 (m, 2H); 7.1-7.0 (t, 2H); 3.85-3.65 (m, 2H); 3.45 (s, 1H); 3.15-3.00 (td, 2H); 2.60-2.35 (m, 2H); 2.30-2.10 (td, 2H); 1.60 (s, 6H); 1.4 (s, 9H).
MS (ES/-): m/z=420 [M-H]⁻.

### Intermediate 114

### 1,1-Dimethylethyl 4-(4-cyanophenyl)-4-(2,2-dimethyl-4,6-dioxo-1,3-dioxan-5-yl)-1-piperidinecarboxylate

A solution of *t*-BuLi (1.5M in pentane - 113 µL) was added drop-wise to 4-bromobenzonitrile (29.4 mg) in anhydrous THF (1.25 mL) previously cooled to -80°C under a Nitrogen atmosphere. Afer stirring at -80°C for 10 minutes, copper iodide (15.4 mg) and intermediate 112 (50 mg) in anhydrous THF (1.25 mL) were added drop-wise to the reaction mixture. The resulting mixture was stirred at -80°C for 1 hour and then allowed to warm to r.t. and stirred at 23°C for 2 hours. The mixture was cooled to 0°C, treated with ammonium chloride saturated solution and few drops of conc. NH₄OH solution and extracted with AcOEt (3 x 20 mL). The combined organic extracts were dried and concentrated *in vacuo*. The residue was purified by flash chromatography (CH/AcOEt from 3:7 to 1:9, then AcOEt) to give the title compound (20 mg) as a white solid.
T.I.c.: CH/AcOEt 3:7, Rf=0.12 (detection with ninhydrine).
MS (ES/-): m/z=427 [M-H]⁻.

### Intermediate 115

### (4-(4-Cyanophenyl)-1-{[(1,1-dimethylethyl)oxy]carbonyl}-4-piperidinyl)acetic acid

A mixture of intermediate **114** (25 mg) in 3-pentanone (4 mL), and water (2 mL) was heated to 102°C for 14 hours. The solution was allowed to cool to r.t and the organic phase was separated. The aqueous phase was acidified to pH=4.5 and extracted with AcOEt (10 mL).

The combined organic phases were dried and concentrated *in vacuo* to give the title compound (25 mg) as a yellow oil used as crude in the next step without further purification.
T.I.c.: DCM/MeOH 9:1, Rf=0.54 (detection with ninhydrine).
MS (ES/-): m/z=343 [M-H]⁻

### Intermediate 116

### 1,1-Dimethylethyl 4-(4-cyanophenyl)-4-{2-[[1-(3,5-dibromophenyl)ethyl](methyl)amino]-2-oxoethyl}-1-piperidinecarboxylate (enantiomer 1)

DIPEA (30 µL) and O-(benzotriazol-1-yi)-N,N,N'N'-tetramethyluronium tetrafluoroborate (35 mg) were added to a solution of intermediate **115** (28 mg) in anhydrous DMF (2 mL) under a Nitrogen atmosphere. After stirring for 15 minutes, intermediate **56** (22 mg) was added. The mixture was stirred at r.t. for 2 days, then it was diluted with DCM (12 mL), washed with 5% sodium hydrogen carbonate solution (12 mL), and passed through a phase separation cartridge with polypropylene frit and concentrated *in vacuo*. The residue was purified by flash chromatography (CH/AcOEt from 8:2 to 1:1) to give the title compound (17 mg) as a colourless oil.
T.I.c.: CH/AcOEt 1:1, Rf=0.38 (detection with ninhydrine).
\MS (ES/+): m/z=642 [M+Na]⁺.

### Intermediate 117

### Methyl (3,5-dibromophenyl)acetate

Trimethylsilyldiazomethane (2M in hexane - 9.25 mL) was added to a solution of 3,5-dibromophenylacetic acid (2.72 g) and anhydrous MeOH (0.41 mL) in anhydrous toluene (25 mL) previously cooled to 0°C under Nitrogen atmosphere. The solution was stirred at 0°C for 20 minutes, then it was concentrated *in vacuo* to give the title compound (2.9 g) as a yellow oil.
T.I.c.: CH/AcOEt 7:3, Rf=0.80 (UV detection).

### Intermediate 118

### Methyl 2-(3,5-dibromophenyl)-2-methylpropanoate

Sodium bis(trimethylsilyl)amide (1M in THF - 24 mL) was added over 15 minutes to a solution of intermediate **117** (2.84 g) in anhydrous THF (28 mL) previously cooled to - 10°C under Nitrogen atmosphere. The orange solution was stirred at 0°C for 20 minutes, then iodomethane (3.5 mL) was dropped over 5 minutes. The resulting brown solution was stirred at room temperature for 1.5 hours becoming an orange suspension. The mixture was quenched with 5% hydrochloric acid solution (50 mL) and extracted with Et20 (2 x 100 mL). The combined organic extracts were washed with 20% sodium thiosulphate solution (100 mL) and brine (100 mL), dried and concentrated *in vacuo*. The residue was purified by flash chromatography (CH, then CH/AcOEt 98/2) to give the title compound (2.6 g) as a yellow oil.
T.I.c.: CH/AcOEt 9:1, Rf=0.67 (UV detection).

### Intermediate 119

### 2-(3,5-dibromphenyl)-2-methylpropanoic acid

Potassium hydroxide (1.7 g) was added to a solution of intermediate **118** in MeOH (25 mL). The resulting yellow mixture was stirred at 70°C for 2 hours, then it was concentrated *in vacuo*. The residue was dissolved in water (30 mL) and washed with Et2O (30 mL). The aqueous layer was then acidified at 0°C until pH=1 with 10% hydrochloric acid solution.

The mixture was extracted with AcOEt (2 x 100 mL). The combined organic extracts were washed with brine (100 mL), dried and concentrated *in vacuo* to give the title compound (2.23 g) as a white solid.
T.I.c.: CH/AcOEt 7:3, Rf=0.19 (UV detection).

### Intermediate 120

### [1-(3,5-dibromophenyl)-1-methylethy]amine hydrochloride

Diphenylphosphorylazide (0.5 mL) was added to a solution of intermediate **119** (0.6 g) and TEA (0.65 mL) in anhydrous toluene (20 mL) under a Nitrogen atmosphere. The resulting pale yellow mixture was stirred at 23°C for 6 hours, then it was washed with a saturated potassium carbonate solution (20 mL). The organic layer was dried and concentrated *in vacuo* to give a yellow oil (0.79 g). This material was treated with 5M hydrochloric acid solution (30 mL) and the mixture was heated to reflux for 18 hours. The mixture was cooled to r.t., basified with 10% sodium hydroxide solution until pH=13 and extracted with AcOEt (2 x 40 mL). The combined organic extracts were dried and concentrated *in vacuo* to give a yellow oil (0.79 g).
This material was dissolved in anhydrous Et2O (6 mL) and treated at 0°C with hydrochloric acid (1M in Et2O - 2.2 mL). The suspension was stirred at 0°C for 20 minutes, then the precipitated formed was filtered and washed with pentane (4 x 4 mL) to give the title compound (0.49 g) as a white solid.
T.I.c.: CH/AcOEt 7:3, Rf=0.04 (detection with ninhydrine).
NMR (d₆-DMSO): δ (ppm) 8.70 (bs, 3H); 7.80 (s, 1H); 7.70 (s, 2H); 1.6 (s, 6H). MS (ES/+): m/z=277 [MH-HCl-NH₃]⁺.

### Example 1

### N-(3,5-Dichlorobenzyl)-2-[4-(4-fluorophenyl)-piperidin-4-yl]-N-methyl-acetamide hydrochloride

TFA (0.5 mL) was added to a solution of intermediate **4** (80 mg) in DCM (2 mL) under a Nitrogen atmosphere. The resulting solution was stirred at r.t. for 30 minutes, then a saturated potassium hydroxide solution was added until pH=9. The aqueous layer was extracted with DCM (2 x 15 mL). The combined organic extracts were dried, concentrated *in vacuo* and the residue was purified by flash chromatography (AcOEt/MeOH 1:1 containing 0.5% of conc. ammonium hydroxide solution) to give the N-(3,5-dichlorobenzyl)-2-[4-(4-fluorophenyl)-piperidin-4-yl]-N-methyl-acetamide (40 mg) as a pale yellow oil (T.I.c.: AcOEt/MeOH 1:1 containing 0.5% of conc. NH₄OH, Rf=0.03).

This compound (40 mg) was dissolved in anhydrous Et2O (1 mL) and treated with hydrochloric acid (1M in Et2O - 0.195 mL) at 0°C under a Nitrogen atmosphere. The mixture was stirred at 0°C for 30 minutes, then it was concentrated *in vacuo* and the residue was triturated with pentane (2 x 1 mL) to give the title compound (40 mg) as a white solid.
NMR (d₆-DMSO - 70°C): δ (ppm) 8.48 (bs, 2H); 7.52-7.47 (2bs, 1H); 7.41 (m, 2H); 7.15-7.11 (2t, 2H); 7.02-7.0 (2s, 2H); 4.29-4.19 (s, 2H); 3.21 (m, 2H); 2.88 (m, 2H); 2.79-2.75 (2s, 3H); 2.54 (s, 3H); 2.4-2.2 (m, 4H).

### Example 2

### N-(3,5-Dichlorobenzyl)-2-[3-fluoro-4-(4-fluorophenyl)-piperidin-4-yl]-N-methylacetamide hydrochloride (anti isomer)

TFA (0.5 mL) was added to a solution of intermediate **9** (60 mg) in DCM (2 mL) under a Nitrogen atmosphere. The resulting solution was stirred at r.t. for 30 minutes, then a saturated potassium hydroxide solution was added until pH=9. The aqueous layer was extracted with DCM (2 x 15 mL). The combined organic extracts were dried, concentrated *in vacuo* and the residue was purified by flash chromatography (AcOEt/MeOH 8:2) to give the N-(3,5-dichlorobenzyl)-2-[3-fluoro-4-(4-fluorophenyl)-piperidin-4-yl]-N-methylacetamide (45 mg) as a pale yellow oil (T.I.c.: AcOEt/MeOH 8:2, Rf=0.1).
This compound was dissolved in anhydrous Et2O (1 mL) and treated with hydrochloric acid (1M in Et2O - 0.21 mL) at 0°C under a Nitrogen atmosphere. The mixture was stirred at 0°C for 30 minutes, then it was concentrated *in vacuo* and the residue was triturated with pentane (2 x 1 mL) to give the title compound (42 mg) as a white solid.
NMR (d₆-DMSO - 70°C): δ (ppm) 8.95 (bs, 2H); 7.46 (m, 2H); 7.39 (bs, 1H); 7.13 (t, 2H); - 6.99 (bs, 2H); 5.86 (bd, 1H); 4.3 (m, 2H); 3.48 (m, 1H); 3.18 (m, 1H); 2.99 (m, 1H); 2.94 (d, 1 H); 2.76 (d, 1 H); 2.75 (m, 1 H); 2.69 (m, 1H); 2.66 (s, 3H); 2.27 (bm, 1 H).

### Example 6

### N-(3,5-Bis-trifluoromethyl)-benzyl-2-[(4-fluoro-2-methyl-phenyl)-piperidin-4-yl]-N-methyl-acetamide

TFA (1.9 mL) was added to a solution of intermediate **30** (144 mg) in anhydrous DCM (2.5 mL) under a Nitrogen atmosphere. The solution was stirred at 23°C for 3 hours, then it was diluted with DCM and washed with saturated potassium carbonate solution and brine.

The organic layer was dried and concentrated *in vacuo* to a residue which was purified by flash chromatography (from AcOEt/MeOH 1:1 to AcOEt/MeOH 3:7 containing 3% of conc.

NH₄OH, then MeOH containing 4% of conc. NH₄OH) to give the title compound (94 mg) as colourless oil. , T.I.c.: AcOEt/MeOH 1:1 containing 1% of conc. NH₄OH, Rf=0.08 (detection with ninhydrine).
NMR (d₆-DMSO): δ (ppm) 7.98 (bs, 1H); 7.74 (bs, 2H); 7.17 (dd, 1H); 6.84 (dd, 1H); 6.7 (dt, 1H); 4.46 (s, 2H); 2.83 (s, 2H); 2.79 (bt, 2H); 2.62 (s, 3H); 2.56 (bt, 2H); 2.41 (s, 3H); 2.15 (m, 2H); 1.97 (m, 2H).
MS (ES/+): m/z=491 [M+H]⁺.

### Example 7

### N-(3,5-Dichlorobenzyl)-2-[4-(4-fluoro-2-methyl-phenyl)-pineridin-4-yl]-N-methylacetamide

TFA (0.4 mL) was added to a solution of intermediate **29** (30 mg) in anhydrous DCM (0.5 mL) under a Nitrogen atmosphere. The solution was stirred at 23°C for 1 hour, then it was diluted with DCM and washed with saturated potassium carbonate solution and brine. The organic layer was dried and concentrated *in vacuo* to a residue which was purified by flash chromatography (from AcOEt/MeOH 1:1 to AcOEt/MeOH 3:7 containing 3% of conc. NH₄OH) to give the title compound (23.5 mg) as colourless oil. T.I.c.: AcOEt/MeOH 3:7 containing 3% of conc. NH₄OH, Rf=0.12 (detection with ninhydrine).
NMR (d₆-DMSO): δ (ppm) 7.45 (s, 1H); 7.25 (dd, 1H); 7.02 (s, 2H); 7.0-6.8 (m, 2H); 4.28 (s, 2H); 2.81-2.78 (s + m, 4H); 2.55-2.57 (m, 5H); 2.43 (s, 3H); 2.17 (m, 2H); 1.99 (m, 2H). MS (ES/+): m/z=423 [M+H]⁺.

### Example 8

### N-(3,5-Dichlorobenzyl)-2-[4-(4-fluoro-2-methyl-phenyl)-piperidin-4-yl]-N-methylacetamide hydrochloride

The compound of Example 7 (19 mg) was dissolved in Et2O (0.2 mL), cooled to 0°C and treated with hydrochloric acid (1M in Et2O - 54 µL). The mixture was stirred at 0°C for 10 minutes, then it was concentrated *in vacuo* and the residue was triturated with pentane to give the title compound (19.3 mg) as a white solid. NMR (d₆-DMSO): δ (ppm) 8.46 (bd, 2H); 7.4 (s, 1H); 73 (m, 1H); 7.02 (s, 2H); 6.94 (m, 2H); 4.31 (s, 2H); 3.23-2.9 (m, 4H); 2.88 (m, 2H); 2.63 (s, 3H); 2.5 (s, 3); 2.5-2.3 (m, 4H).

### Example 9

### N-(3,5-Bis-trifluoromethyl-benzyl)-2-[4-(4-fluorophenyl)-azepin-4-yl]-N-methylacetamide hydrochloride

TFA (0.5 mL) was added to a solution of intermediate **37** (16 mg) in DCM (2 mL) under a Nitrogen atmosphere. The resulting solution was stirred at r.t. for 30 minutes, then a saturated potassium hydroxide solution was added until pH=9. The aqueous layer was extracted with DCM (2 x 15 mL). The combined organic extracts were dried, concentrated *in vacuo* and the residue was purified by flash chromatography (DCM/MeOH 8:2) to give N-(3,5-bis-trifluoromethyl-benzyl)-2-[4-(4-fluorophenyl)-azepin-4-yl]-N-methyl-acetamide (12 mg - T.I.c.: DCM/MeOH 8:2 containing 0.5% of conc. NH₄OH, Rf=0.4).
This compound (12 mg) was dissolved in anhydrous Et2O (1 mL) and treated with hydrochloric acid (1M in Et2O - 0.05 mL) at 0°C under a Nitrogen atmosphere. The mixture was stirred at 0°C for 30 minutes, then it was concentrated *in vacuo* and the residue was triturated with pentane (2 x 1 mL) to give the title compound (10 mg) as a white solid.
NMR (d₆-DMSO): δ (ppm) 8.41 (bs, 2H); 7.92 (s, 1H); 7.74 (s, 2H); 7.33 (dd, 2H); 7.01 (bt, 2H); 4.5 (m, 2H); 3.27 (dd, 1H); 3.07 (m, 2H); 2.91 (dd, 1H); 2.72 (bs, 3H); 2.81-2.65 (m, 2H); 2.56 (m, 1H); 2.42-2.29 (m, 2H); 2.05 (dd, 1H); 1.86 (bm, 1H); 1.6 (bm, 1 H).

### Example 10

### N-(3,5-Bis-trifluoromethyl-benzyl)-2-[4-(4-fluoro-2-methyl-phenyl)-azepin-4-yl]-N-methyl-acetamide

TFA (0.5 mL) was added to a solution of intermediate **40** (30 mg) in DCM (2 mL) under a Nitrogen atmosphere. The resulting solution was stirred at r.t. for 30 minutes, then a saturated potassium hydroxide solution was added until pH=9. The aqueous layer was extracted with DCM (2 x 15 mL). The combined organic extracts were dried, concentrated *in vacuo* and the residue was purified by flash chromatography (DCM/MeOH 8:2) to give the title compound (17 mg) as a pale yellow oil.
T.I.c.: DCM/MeOH 8:2, Rf=0.5.
NMR (d₆-DMSO): δ (ppm) 8.06-8.0 (2bs, 1H); 7.8-7.76 (2bs, 2H); 7.23-7.15 (2m, 1H); 6.91 (m, 1H); 6.76 (m, 1H); 4.69 (bs, 1H); 4.53 (m, 1H); 3.0-2.3 (m, 11H); 2.43 (s, 3H); 2.01 (m, 2H); 1.66 (m, 1H); 1.41 (m, 1H).

### Example 11

### N-(3,5-Dichlorobenzyl)-2-[4-(4-fluoro-2-methyl-phenyl)-azepin-4-yl]-N-methylacetamide

TFA (0.5 mL) was added to a solution of intermediate **41** (30 mg) in DCM (2 mL) under a Nitrogen atmosphere. The resulting solution was stirred at r.t. for 30 minutes, then a saturated potassium hydroxide solution was added until pH=9. The aqueous layer was extracted with DCM (2 x 15 mL). The combined organic extracts were dried, concentrated *in vacuo* and the residue was purified by flash chromatography (DCM/MeOH 8:2) to give the title compound (20 mg) as a pale yellow oil.
T.I.c.: DCM/MeOH 8:2, Rf=0.5.
NMR (d₆-DMSO): δ (ppm) 7.53-7.47 (2t, 1H); 7.24-7.2 (2dd, 1H); 7.1 (d, 2H); 6.88 (m, 2H); 4.46-4.34 (s + m, 2H); 3.0-2.3 (m, 11H); 2.44 (s, 3H); 2.01 (m, 2H); 1.67 (bm, 1H); 1.39 (bm, 1 H).

### Example 12

### N-(3,5-Bis-trifluoromethyl-benzyl)-2-[3-fluoro-4-(4-fluoro-2-methyl-phenyl)-azepin-4-yl]-N-methyl-acetamide (anti isomer)

TFA (0.5 mL) was added to a solution of intermediate **48** (35 mg) in DCM (2 mL) under a Nitrogen atmosphere. The resulting solution was stirred at r.t: for 30 minutes, then a saturated potassium hydroxide solution was added until pH=9. The aqueous layer was extracted with DCM (4 x 15 mL). The combined organic extracts were dried, concentrated *in vacuo* and the residue was purified by flash chromatography (AcOEt/MeOH 8:2) to give the title compound (25.1 mg) as a pale yellow oil.
T.I.c.: AcOEt/MeOH 8:2, Rf=0.14.
NMR (d₆-DMSO): δ (ppm) 8.06-7.97 (2bs, 1H); 7.76 (bs, 2H); 7.17-7.13 (2dd, 1H); 6.89 (dd, 1H); 6.78 (dt, 1H); 5.53-5.4 (2bd, 1H); 4.76-4.53 (2m, 2H); 3.25-2.85 (m, 4H); 2.68 (m, 2H); 2.91-2.72 (2s, 3H); 2.44-2.28 (2s, 3H); 2.2 (m, 2H); 1.57 (bm, 1H); 1.3 (bm, 1H).

### Example 13

### N-(3,5-Dichlorobenzyl)-2-[3-fluoro-4-(4-fluoro-2-methyl-phenyl)-azepin4-yl]-N-methyl-acetamide (anti isomer)

TFA (0.5 mL) was added to a solution of intermediate **49** (36.1 mg) in DCM (2 mL) under a Nitrogen atmosphere. The resulting solution was stirred at r.t. for 30 minutes, then a saturated potassium hydroxide solution was added until pH=9. The aqueous layer was extracted with DCM (4 x 15 mL). The combined organic extracts were dried, concentrated *in vacuo* and the residue was purified by flash chromatography (AcOEt/MeOH 8:2) to give the title compound (20.5 mg) as a pale yellow oil.
T.I.c.: AcOEt/MeOH 8:2, Rf=0.08.
NMR (d₆-DMSO): δ (ppm) 7.54-7.46 (2t, 1H); 7.16 (m, 1H); 7.11-7.07 (2d, 2H); 6.94 (dd, 1H); 6.87 (m, 1 H); 5.6-5.49 (2bd, 1H); 4.55-4.35 (2m, 2H); 3.25-2.88 (m, 2H); 3.05 (m, 2H); 2.85-2.69 (2s, 3H); 2.7 (m, 1H); 2.65-2.35 (m, 1H); 2.46 (2s, 3H); 2.35-2.1 (m, 2H) 1.62-1.5 (2bm, 1H); 1.33-1.23 (bm, 1H).

### Example 14

### N-(3,5-Dichlorobenzyl)-2-[3-fluoro-4-(4-fluoro-2-methyl-phenyl)-azepin-4-yl]-N-methyl-acetamide (14a enantiomer1)and (14b enantiomer2)

The compound of example **13** (16 mg) was separated into the enantiomers via preparative HPLC (Column: Chiralpack AD 25 x 2 cm; mobile phase: n-hexane/EtOH 8:2; flux=7.5 mUmin; λ 225 nm). Thus, example **14a** (6 mg) and example **14b** (5.8 mg) were obtained.

### Example 14a:

Chiral HPLC: Column Chiralpack AD 25cm x 4.6mm x 5µm, mobile phase n-hexane/EtOH 8:2, flux=1 mUmin, λ 225 nm; retention time 12.2 minutes. Ratio **14a/14b**=100:0.

### Example 14b:

Chiral HPLC: Column Chiralpack AD 25cm x 4.6mm x 5µ, mobile phase n-hexane/EtOH 8:2, flux=1 mL/min, λ 225 nm; retention time 14.05 minutes. Ratio **14a/14b**=0:100.

### Example 15

### N-(3,5-Bis-trifluoromethyl-benzyl)-2-[3-fluoro-4-(4-fluoro-2-methyl-phenyl)-azepin-4-yl]-N-methyl-acetamide (15a enantiomer 1)-and (15b enantiomer 2)

The compound of Example **12** (21 mg) was separated into the enantiomers via preparative HPLC (Column: Chiralpack AD 25 x 2 cm; mobile phase: n-hexane/EtOH 92:8; flux=7.5 mL/min; λ 225 nm). Thus, example **15a** (4.9 mg) and example **15b** (6.6 mg) were obtained.

### Example 15a:

Chiral HPLC: Column Chiralpack AD 25cm x 4.6mm x 5µm, mobile phase n-hexane/EtOH 85:15, flux=1 mL/min, λ 225 nm; retention time 7.37 minutes. Ratio **15a/15b**=6:4.

### Example 15b:

Chiral HPLC: Column Chiralpack AD 25cm x 4.6mm x 5µm, mobile phase n-hexane/EtOH 92:8, flux=1 mL/min, λ 225 nm; retention time 8.04 minutes. Ratio **15a/15b**=2:98.

### Example 16

### N-(3,5-Dibromobenzyl)-2-[4-(4-fluorophenyl)-piperidin-4-yl]-N-methyl-acetamide

TFA (12 mL) was added to a solution of intermediate **58** (1.38 g) in DCM (48 mL) previously cooled to 0°C under a Nitrogen atmosphere. The resulting solution was stirred in these conditions for 1 hour, then a 10% sodium hydroxide solution was added until pH=12. The aqueous layer was extracted with DCM (2 x 15 mL). The combined organic extracts were dried, concentrated *in vacuo* and the residue was purified by flash chromatography (AcOEt/MeOH 9:1 to 7:3 containing 0.5% of conc. NH₄OH) to give the title compound (800 mg) as a white foam.
T.I.c.: DCM/MeOH 8:2 containing 0.5% of conc. NH₄OH, Rf=0.07.
NMR (d₆-acetone): δ (ppm) 7.52 (s, 1H); 7.29 (dd, 2H); 7.21 (s, 2H); 6.89 (t, 2H); 4.24 (s, 2H); 2.77 (dd, 2H); 2.64-2.54 (m, 4H); 2.08-1.9 (mm, 4H); 2.62 (s, 3H).
MS (ES/+): m/z=499 [M+H]⁺.

### Example 17

### N-(3,5-Dibromo-benzyl)-2-[4-(4-fluoro-phenyl)-1-methyl-piperldin-4-yl]-N-methylacetamide

Sodium triacethoxyborohydride (966 mg) was added to a solution of Example **16** (760 mg) in anhydrous CH₃CN (30 mL) with formaldehyde in water (37% w/w, 460 µL) under a Nitrogen atmosphere. The mixture was stirred at r.t. for 2 hours, then the solvent was removed *in vacuo* and the residue was dissolved in 5% sodium hydrogen carbonate solution (10 mL) and extracted with DCM (2 x 15mL). The organic layer was dried, concentrated *in vacuo* and the residue was purified by flash chromatography (DCM, DCM/MeOH from 9:1 to 1:1) to give the title compound (512 mg) as a white solid. T.I.c.: DCM/MeOH 8:2 containing 0.5% of conc. NH₄OH, Rf=0.62 (detection with ninhydrine).
MS (ES/+): m/z=513 [M+H]⁺.

### Example 18

### N-(3,5-Dibromo-benzyl)-2-[4-(4-fluoro-phenyl)-1-methyl-piperidin-4-yl]-N-methylacetamide hydrochloride

The compound of Example **17** (512 mg) was dissolved in Et2O (15 mL), cooled to 0°C and treated with hydrochloric acid (1 M in Et2O - 1.1 mL). The mixture was stirred at 0°C for 30 minutes, then it was filtered under N₂ athmosphere and the precipitate was triturated with pentane to give the title compound (360 mg) as a white solid.
NMR (d₆-DMSO): δ (ppm) 10.0-9.6 (m, 1 H); 7.70 (s, 1H); 7.43 (7.36) (dd, 2H); 7.24 (7.21) (s, 2H): 7.18-7.06 (m (t), 2H); 4.27 (s, 2H); 3.38 (s, 3H); 3.31 (m, 2H); 2.79 (q, 2H); 2.62 (bs, 4H); 2.53 (s, 3); 2.05-1.90 (t/t, 1/1 H).

### Example 19

### N-(3,5-Dibromobenzyl)-2-(4-phenyl-piperidin-4-yl)-N-methyl-acetamide

TFA (6 mL) was added to a solution of intermediate **65** (518 mg) in anhydrous DCM (24 mL) under a Nitrogen atmosphere. The solution was cooled at -4°C overnight, then it was treated with 10% sodium hydroxide solution until pH= 8-9 and extracted with DCM. The organic layer was dried and concentrated *in vacuo* to give the title compound (335 mg) as colourless oil.
T.I.c.: DCM/MeOH 8:2, containing 2.5% of conc. NH₄OH, Rf=0.39 (detection with ninhydrine).
MS (ES/+): m/z= 481 [M+H]⁺.

### Example 20

### N-(3,5-Dibromo-benzyl)-2-(4-phenyl-1-methyl-piperidin-4-yl)-N-methyl-acetamide

Sodium triacethoxyborohydride (99 mg) was added to a solution of Example **19** (100 mg) in anhydrous CH₃CN (5.6 mL) with formaldehyde in water (37% w/w, 47 µL) under a Nitrogen atmosphere. The mixture was stirred at r.t. 3.5 hours, then it was diluted with water (5 mL), AcOEt (10 mL) and washed with brine (10 mL). The organic layer was dried, concentrated *in vacuo* and the residue was purified by flash chromatography (DCM, DCM/MeOH from 9:1 to 1:1) to give the title compound (93.5 mg) as a white solid.
T.I.c.: DCM/MeOH 8:2, Rf=0.12 (detection with ninhydrine).
NMR (d₆-DMSO ): δ (ppm) 7.70 (s, 1H); 7.314-7.10 (m, 7H); 4.28 (s, 2H); 2.63 (s, 3H); 2.5-2.1 (m, 8H); 2.33 (s, 2H); 2.07 (s, 3H).

### Example 21

### N-[1-(3,5-Dichloro-phenyl)-ethyl]-2-[4-(4-fluoro-phenyl)-piperidin-4-yl]-N-methylacetamide

TFA (3 mL) was added to a solution of intermediate **69** (300 mg) in DCM (12 mL) previously cooled to 0°C under a Nitrogen atmosphere. The resulting solution was stirred in these conditions for 2 hours, then a 10% sodium hydroxide solution was added until pH=12. The aqueous layer was extracted with DCM (2 x 15 mL) which was passed through a phase separation cartridge with polypropylene frit and concentrated in vacuo.The residue was purified by SCX cartridge (load with DCM and elution with MeOH, NH₃ 0.2M in MeOH) to give the title compound (118 mg) as a white foam.
T.I.c.: DCM/MeOH 8:2 containing 1 % of conc. NH₄OH, Rf=0.07.
MS (ES/+): m/z=423 [M+H]⁺.

### Example 22

### N-[1-(3,5-Dichloro-phenyl)-ethyl]-2-[4-(4-fluoro-phenyl)-1-methyl-piperidin-4-yl]-N-mothyl-acetamide

Sodium triacethoxyborohydride (126 mg) was added to a solution of Example **21** (100 mg) in anhydrous CH₃CN (5 mL) with a solution of formaldehyde in water (37% w/w, 60 µL) under a Nitrogen atmosphere. The mixture was stirred at r.t. for 2 hours, then the solvent was removed *in vacuo* and the residue was dissolved in 5% sodium hydrogen carbonate solution (5 mL) and extracted with DCM (2 x 8 mL), which was passed through a phase separation cartridge with polypropylene frit and concentrated *in vacuo.* The residue was purified by flash chromatography (DCM, DCM/MeOH from 95:5 to 1:1) to give the title compound (68 mg) as a white solid.
T.I.c.: DCM/MeOH 8:2 containing 1% of conc. NH₄OH, Rf=0.38 (detection with ninhydrine).
NMR (d₆-DMSO): δ (ppm) 7.24 (dd, 2H); 7.16 (t, 1H); 6.93 (t, 2H); 6.87 (s, 2H); 5.77 (q, 1 H); 2.58-2.02 (m, 10H); 2.17 (s, 3H); 2.02 (s, 3H); 1.14 (d, 3H).
MS (ES/+): m/z=437 [M+H]⁺.

### Example 23

### N-[1-(S)-1-(3,5-Bis-trifluoromethyl-phenyl)-ethyl]-2-[4-(4-fluoro-phenyl)-piperidin-4-yl]-N-methyl-acetamide

TFA (6 mL) was added to a solution of intermediate **60** (590 mg) in DCM (24 mL) previously cooled to 0°C under a Nitrogen atmosphere. The resulting solution was stirred in these conditions for 2 hours, then a 10% sodium hydroxide solution was added until pH=12. The aqueous layer was extracted with DCM (2 x 15 mL) which was passed through a phase separation cartridge with polypropylene frit and concentrated *in vacuo*.

The residue was purified by SCX Cartridge (load with DCM and elution with MeOH, NH₃ 0.2M in MeOH) to give the title compound (517 mg) as a white foam.
T.I.c.: DCM/MeOH 8:2 containing 0.5% of conc. NH₄OH, Rf=0.15.
MS (ES/+): m/z=491 [M+H]⁺.

### Example 24

### N-[1-(S)-1-(3,5-Bis-trifluoromethyl-phenyl)-ethyl]-2-[4-(4-fluoro-phenyl)-methyl-piperidin-4-yl]-N-methyl-acetamide

Sodium triacethoxyborohydride (126 mg) was added to a solution of Example **23** (100 mg) in anhydrous CH₃CN (5 mL) with a solution of formaldehyde in water (37% w/w, 60 µL) under a Nitrogen atmosphere. The mixture was stirred at r.t. for 2 hours, then the solvent was removed *in vacuo* and the residue was dissolved in 5% sodium hydrogen carbonate solution (5 mL) and extracted with DCM (2 x 8 mL), which was passed through a phase separation cartridge with polypropylene frit and concentrated *in vacuo*. The residue was purified by flash chromatography (DCM, DCM/MeOH from 95:5 to 1:1) to give the title compound (59 mg) as a white solid.
T.I.c.: DCM/MeOH 8:2 containing 0.5% of conc. NH₄OH, Rf=0.42 (detection with ninhydrine).
NMR (d₆-DMSO): δ (ppm) 7.97 (s, 1H); 7.67 (s, 2H); 7.30 (dd, 2H); 7.94 (t, 2H); 5.70 (q, 1H); 2.62-2.03 (m, 10H); 2.34 (s, 3H); 2.05 (s, 3H); 1.26 (d, 3H).
MS (ES/+): m/z=505 [M+H]⁺.

### Example 25

### N-[1-(S)-1-(3,5-Bis-trifluoromethyl-phenyl)-ethyl]-2-[4-(4-fluoro-phenyl)-methyl-piperidin-4-yl]-N-methyl-acetamide hydrochloride

The compound of Example **24** (20 mg) was dissolved in Et2O (1 mL), cooled to 0°C and treated with hydrochloric acid (1M in Et2O - 44 µL). The mixture was stirred at 0°C for 30 minutes, then the solvent was removed *in vacuo* and the residue was triturated with pentane to give the title compound (20 mg) as a white solid.
MS (ES/+): m/z=505 [MH-HCl]⁺.

### Example 26

### N-[1-(3,5-Dibromo-phenyl)-ethyl]-2-[4-(4-fluoro-phenyl)-piperidin-4-yl]-N-methylacetamide

TFA (6 mL) was added to a solution of intermediate **61** (390 mg) in DCM (24 mL) previously cooled to 0°C under a Nitrogen atmosphere. The resulting solution was stirred in these conditions for 2 hours, then a 10% sodium hydroxide solution was added until pH=12. The aqueous layer was extracted with DCM (2 x 15 mL) which was passed through a phase separation cartridge with polypropylene frit and concentrated *in vacuo*.The residue was purified by SCX cartridge (load with DCM and elution with MeOH, NH₃ 0.2M in MeOH) to give the title compound (290 mg) as a white foam.
T.I.c.: DCM/MeOH 8:2 containing 1% of conc. NH₄OH, Rf=0.15.
MS (ES/+): m/z=513 [M+H]⁺.

### Example 27

### N-[1-(3,5-Dibromo-phenyl)-ethyl]-2-[4-(4-fluoro-phenyl)-1-methyl-piperidin-4-yl]-N-methyl-acetamide

Sodium triacethoxyborohydride (126 mg) was added to a solution of Example **26** (100 mg) in anhydrous CH₃CN (5 mL) with a solution of formaldehyde in water (37% w/w, 60 µL) under a Nitrogen atmosphere. The mixture was stirred at r.t. for 2 hours, then the solvent was removed *in vacuo* and the residue was dissolved in 5% sodium hydrogen carbonate solution (5 mL) and extracted with DCM (2 x 8 mL), which was passed through a phase separation cartridge with polypropylene frit and concentrated *in vacuo*. The residue was purified by flash chromatography (DCM, DCM/MeOH from 95:5 to 1:1) to give the title compound (46 mg) as a white solid. T.I.c.: DCM/MeOH 8:2 containing 1% of conc. NH₄OH, Rf=0.65 (detection with ninhydrine).
NMR (CDCl₃): δ (ppm) 7.53 (t, 1H); 7.31 (dd, 2H); 7.15 (s, 2H); 7.00 (t, 2H); 5.84 (q, 1H); 2.64-2.07 (m, 10H); 2.24 (s, 3H); 2.07 (s, 3H); 1.19 (d, 3H).
MS (ES/+): m/z=527 [M+H]⁺.

### Example 28

### N-[1-(S)-1-(3,5-Bis-trifluoromethyl-phenyl)-ethyl]-2-(4-phenyl-piperidin-4-yl)-N-methyl-acetamide

TFA (6 mL) was added to a solution of intermediate **66** (463 mg) in anhydrous DCM (24 mL) under a Nitrogen atmosphere. The solution was cooled at -4°C and keeped at this temperature overnight, then it was treated with 10% sodium hydroxide solution until pH= 8-9 and extracted with DCM. The organic layer was dried and concentrated *in vacuo* to give the title compound (348.2 mg) as colourless oil.
T.I.c.: DCM/MeOH 8:2 containing 2.5% of conc. NH₄OH, Rf=0.39 (detection with ninhydrine).
MS (ES/+): m/z= 473 [M+H]⁺.

### Example 29

### N-[1-(S)-1-(3,5- Bis-trifluoromethyl-phenyl)-ethyl]-2-(4-phenyl-1-methyl-piperidin-4-yl)-N-methyl-acetamide

Sodium triacethoxyborohydride (152 mg) was added to a solution of Example **28** (100 mg) in anhydrous CH₃CN (5.7 mL) with a solution of formaldehyde in water (37% w/w, 70 µL) under a Nitrogen atmosphere. The mixture was stirred at r.t. 5 hours, then it was diluted with water (5 mL) and washed with 5% sodium hydrogen carbonate solution (10 mL) and extracted with DCM. The organic layer was dried, concentrated *in vacuo* and the residue was purified by flash chromatography (DCM, DCM/MeOH from 9:1 to 6:4) to give the title compound (82.8 mg) as a white solid.
T.I.c.: DCM/MeOH 8:2, Rf=0.17 (detection with ninhydrine).
NMR (CDCl₃): δ (ppm) 7.74 (s, 1H); 7.54 (s, 2H); 7.32-7.10 (m, 5H); 5.98 (q, 1H); 2.70-2.10 (m, 10H); 2.23 (s, 3H); 1.99 (s, 3H); 1.26 (d, 3H).

### Example 30

### N-[1-(3,5-Dibromo-phenyl)-ethyl]-2-(4-phenyl-piperidin-4-yl)-N-methyl-acetamide

TFA (2.5 mL) was added to a solution of intermediate **67** (105 mg) in anhydrous DCM (10 mL) under a Nitrogen atmosphere. The solution was cooled at -4°C and keeped at this temperature overnight, then it was treated with 10% sodium hydroxide solution until pH= 8-9 and extracted with DCM. The organic layer was dried and concentrated *in vacuo* to give the title compound (78.8 mg) as colourless oil.
T.I.c.: DCM/MeOH 8:2 containing 2.5% of conc. NH₄OH: Rf=0.39 (detection with ninhydrine).
MS (ES/+): m/z= 495 [M+H]⁺.

### Example 31

### N-[1-(3,5-Dibromo-phenyl)-ethyl]-2-(4-phenyl-1-methyl-piperidin-4-yl)-N-methylacetamide

Sodium triacethoxyborohydride (102 mg) was added to a solution of Example **30** (70 mg) in anhydrous CH₃CN (3.8 mL) with a solution of formaldehyde in water (37% w/w, 48 µL) under a Nitrogen atmosphere. The mixture was stirred at r.t. 5 hours, then it was diluted with water (5 mL) and washed with 5% sodium hydrogen carbonate solution (10 mL) and extracted with DCM. The organic layer was dried, concentrated *in vacuo* and the residue was purified by flash chromatography (DCM, DCM/MeOH from 9:1 to 6:4) to give the title compound (60.1 mg) as a white solid.
T.I.c.: DCM/MeOH 8:2, Rf=0.12 (detection with ninhydrine).
NMR (CDCl₃ ): δ (ppm) 7.51 (s, 1H); 7.36-7.18 (m, 5H); 7.14 (s, 2H); 5.83 (q, 1H); 2.70-2.10 (m, 10H); 2.23 (s, 3H); 1.96 (s, 3H); 1.16 (d, 3H).

### Example 32

### N-[1-(3,5-Dibromo-phenyl)-ethyl]-2-[4-(4-fluoro-phenyl)-piperidin-4-yl]-N-methylacetamide (enantiomer 1)

TFA (2.8 mL) was added to a solution of intermediate **62** (370 mg) in anhydrous DCM (12 mL) previously cooled at 0°C, under a Nitrogen atmosphere. The solution was stirred at 0°C for 2 hours, then it was treated at 0°C with a 10% sodium hydroxide solution until pH=10. The aqueous layer was extracted with DCM (2 x 10 mL). The combined organic extracts were dried, concentrated *in vacuo* to give the title compound as a white foam (370 mg) used as crude in the next step without further purification.
T.I.c.: DCM/MeOH 8:2, containing 1% of conc. NH₄OH, Rf=0.15 (detection with ninhydrine).
MS (ES/+): m/z=513 [M+H]⁺.

### Example 33

### N-[1-(3,5-Dibromo-phenyl)-ethyl]-2-[4-(4-fluoro-phenyl)-1-methyl-piperidin-4-yl]-N-methyl-acetamide (enantiomer 1)

A solution of formaldehyde in water (37% w/w; 110 µL) was added to a stirred solution of Example **32** in CH₃CN (8 mL) under a Nitrogen atmosphere at 23°C. After 10 minutes sodium triacetoxyborohydride (230 mg) was added. The mixture was stirred for further 2 hours then it was quenched with a 5% sodium hydrogen carbonate solution (10 mL) and extracted with EtOAc (3 x 30 mL). The combined organic phases were dried and concentrated *in vacuo*, and the residue was purified by flash chromatography (DCM/MeOH from 9:1 to 8:2, then from 8:2 containing 3% of conc. NH₄OH to 7:3 containing 3% of conc. NH₄OH) to give the title compound (260 mg) as a white solid. T.I.c.: DCM/MeOH 8:2 containing 1% of conc. NH₄OH, Rf=0.65 (detection with ninhydrine).
MS (ES/+): m/z = 527 [M+H]⁺.

### Example 34

### (-)-N-[1-(3,5-Dibromo-phenyl)-ethyl]-2-[4-(4-fluoro-phenyl)-1-methyl-piperidin-4-yl]-N-methyl-acetamide hydrochloride

The compound of Example **33** (246 mg) was dissolved in dry Et2O (8 mL), cooled to 0°C and treated with hydrochloric acid (1 M in Et2O - 514 µL). The mixture was stirred at 0°C for 1.5 hours, then it was filtered under a nitrogen atmopsphere in a dry box. The solid was washed with Et2O (2 x 4 mL), and then pentane (3 x 8 mL) to give the title compound (200 mg) as a white solid.
NMR (d₆-DMSO): δ (ppm) 10.02 (bs, 1H); 7.71 (s, 1H); 7.37 (dd, 2H); 7.20 (s, 2H); 7.13 (m, 2H); 5,58 (q, 1H); 3.35 (bm, 2H); 2.96 (d, 1H); 2.9 (d, 1H); 2.82 (dd, 1H); 2.79 (d, 3H); 2.64 (dd, 1H); 2.7-2.5 (m, 2H); 2.25 (s, 3H); 2.07 (dd, 1H); 1.92 (m, 1H); 1.18 (d, 3H).
MS (ES/+): m/z=527 [MH-HCl]⁺.
[α]_{D}: - 49.1 (0.5% in MeOH).
HPLC (column: XTerra MSC18 50x4.6mm, 5 µm; mobile phase: H₂O+0.1% TFA/CH₃CN+0.1 TFA from 90/10 to 10/90 in 12 min.; flow rate=1 mL/min; detection: λ=200-400 nm, ES+: 100-900): retention time = 8.56 minutes; purity (a/a %) >99%.

### Example 35

### N-[1-(3,5-Dibromo-phenyl)-ethyl]-2-[4-(4-fluoro-phenyl)-piperidin-4-yl]-N-methylacetamide (enantiomer 2)

TFA (1.5 mL) was added to a solution of intermediate **63** (310 mg) in anhydrous DCM (6 mL) previously cooled at 0°C, under a Nitrogen atmosphere. The solution was stirred at 0°C for 2 hours, then it was concentrated *in vacuo* at 0°C. The residue was taken up with a saturated solution of potassium carbonate (10 mL) and diluited with EtOAc (50 mL). The organic phase was separated and the aqueous layer was extracted with EtOAc (2 x 50 mL). The collected organic phases were dried and concentrated *in vacuo* to give the title compound as a white foam (300 mg) used as crude in the next step without further purification.
T.I.c.: DCM/MeOH 8:2, containing 1% of conc. NH₄OH, Rf=0.15 (detection with ninhydrine).
MS (ES/+): m/z=513 [M+H]⁺.

### Example 36

### N-[1-(3,5-Dibromo-phenyl)-ethyl]-2-[4-(4-fluoro-phenyl)-1-methyl-piperidin-4-yl]-N-methyl-acetamide (enantiomer 2)

A solution of formaldehyde in water (37% w/w; 92 µL) was added to a stirred solution of Example **35** in CH₃CN (6 mL) under a nitrogen atmosphere at 23°C. After 30 minutes sodium triacetoxyborohydride (181 mg) was added. The mixture was stirred for further 2 hours then it was quenched with a saturated solution of sodium hydrogen carbonate (5 mL) and extracted with EtOAc (3 x 50 mL). The combined organic phases were dried and concentrated *in vacuo*, and the residue was purified by flash chromatography (DCM/MeOH from 9:1 to 8:2, then from 8:2 containing 3% of conc. NH₄OH to 7:3 containing 3% of conc. NH₄OH) to give the title compound (225 mg) as a white solid. T.I.c.: DCM/MeOH 8:2 containing 1% of conc. NH₄OH, Rf=0.65 (detection with ninhydrine).
MS (ES/+): m/z=527 [M+H]⁺.

### Example 37

### (+)-N-[1-(3,5-Dibromo-phenyl)-ethyl]-2-[4-(4-fluoro-phenyl)-1-methyl-piperidin-4-yl]-N-methyl-acetamide hydrochloride (enantiomer 2)

The compound of Example **36** (220 mg) was dissolved in dry Et2O (8 mL), cooled to 0°C and treated with hydrochloric acid (1M in Et2O - 460 µL). The mixture was stirred at 0°C for 30 minutes, then it was filtered under a nitrogen atmopsphere in a dry box. The solid was washed with Et2O (2 x 5 mL), Et2O/pentane 1:1 (2 x 6 mL) and pentane (2 x 3 mL) to give the title compound (190 mg) as a white solid.
NMR (d₆-DMSO): δ (ppm) 10.02 (bs, 1H); 7.71 (s, 1H); 7.37 (dd, 2H); 7.20 (s, 2H); 7.13 (m, 2H); 5,58 (q, 1 H); 3.35 (bm, 2H); 2.96 (d, 1H); 2.9 (d, 1 H); 2.82 (dd, 1H); 2.79 (d, 3H); 2.64 (dd, 1H); 2.7-2.5 (m, 2H); 2.25 (s, 3H); 2.07 (dd, 1H); 1.92 (m, 1H); 1.18 (d, 3H).
MS (ES/+): m/z=527 [MH-HCl]⁺.
[α]_{D}: +50.6 (0.5% in MeOH).
HPLC (column: XTerra MSC18 50x4.6mm, 5µm; mobile phase: H₂O+0.1% TFA/CH₃CN+0.1 TFA from 90/10 to 10/90 in 12 min.; flow rate=1 mL/min; detection: λ=200-400 nm, ES+: 100-900): retention time = 8.56 minutes; purity (a/a %) >99%.

### Example 38

### N-[1-(3,5-Dibromo-phenyl)-ethyl]-2-(4-phenyl-piperidin-4-yl)-N-methyl-acetamide (enantiomer 1)

TFA (3 mL) was added to a solution of intermediate **68** (320 mg) in DCM (12 mL) previously cooled to 0°C under a Nitrogen atmosphere. The resulting solution was stirred in these conditions for 1 hour, then a 10% sodium hydroxide solution was added until pH=12. The aqueous layer was extracted with DCM (2 x 10 mL). The combined organic extracts were dried, concentrated *in vacuo* to give the title compound (215 mg) as a white foam.
T.I.c.: DCM/MeOH 8:2 containing 0.5% of conc. NH₄OH, Rf=0.09.
MS (ES/+): m/z=495 [M+H]⁺.

### Example 39

### N-[1-(3,5-Dibromo-phenyl)-ethyl]-2-(1-methyl-4-phenyl-piperidin-4-yl)-N-methylacetamide (enantiomer 1)

Sodium triacethoxyborohydride (276 mg) was added to a solution of Example **38** (215 mg) in anhydrous CH₃CN (10 mL) with a solution of formaldehyde in water (37% w/w, 130 µL) under a Nitrogen atmosphere. The mixture was stirred at r.t. for 1 hour, then the solvent was removed *in vacuo* and the residue was dissolved in 5% sodium hydrogen carbonate solution (5 mL) and extracted with DCM (2x10 mL). The organic layer was dried, concentrated *in vacuo* and the residue was purified by flash chromatography (DCM, DCM/MeOH from 9:1 to 8:2 containing 0.5% of conc. NH₄OH) to give the title compound (162 mg) as a white solid.
T.I.c.: DCM/MeOH 8:2 containing 0.5% of conc. NH₄OH, Rf=0.43 (detection with ninhydrine).
MS (ES/+): m/z=509 [M+H]⁺.

### Example 40

### N-[1-(3,5-Dibromo-phenyl)-ethyl]-2-(1-methyl-4-phenyl-piperidin-4-yl)-N-methylacetamide hydrochloride (enantiomer 1)

The compound of Example **39** (160 mg) was dissolved in Et2O (6 mL), cooled to 0°C and treated with hydrochloric acid (1 M in Et2O - 350 µL). The mixture was stirred at 0°C for 30 minutes, then the solvent was removed *in vacuo* and the residue was triturated with pentane to give the title compound (160 mg) as a white solid. NMR (d₆-DMSO): δ (ppm) 9.75 (bs, 1H) 7.69 (s, 1H); 7.45-7.08 (m, 7H); 5.56 (q, 1H); 3.3 (b, 2H); 3.0-1.8 (m, 14); 1.12 (d, 3H).

MS (ES/+): m/z=505 [MH-HCl]⁺.

### Example 41

### N-[1-(3,5-Dichloro-phenyl)-ethyl]-2-[4-(4-fluoro-phenyl)-piperidin-4-yl]-N-methylacetamide (enantiomer 1)

TFA (3 mL) was added to a solution of intermediate **64** (270 mg) in anhydrous DCM (12 mL) previously cooled at 0°C, under a Nitrogen atmosphere. The solution was stirred at 0°C for 2 hours, then it was treated at 0°C with a 10% sodium hydroxide solution until pH=10. The aqueous layer was extracted with DCM (2 x 10 mL). The combined organic extracts were dried, concentrated *in vacuo* to give the title compound as a colourless oil (227 mg) used as crude in the next step without further purification. T.I.c.: DCM/MeOH 8:2, containing 1% of conc. NH₄OH, Rf=0.07 (detection with ninhydrine).
MS (ES/+): m/z=423 [M+H]⁺.

### Example 42

### N-[1-(3,5-Dichloro-phenyl)-ethyl]-2-[4-(4-fluoro-phenyl)-1-methyl-piperidin-4-yl]-N-methyl-acetamide (enantiomer 1)

A solution of formaldehyde in water (37% w/w; 80 µL) was added to a stirred solution of Example **41** (227 mg) in CH₃CN (8 mL) under a Nitrogen atmosphere at 23°C. After 10 minutes sodium triacetoxyborohydride (170 mg) was added. The mixture was stirred for further 2 hours then it was quenched with a 5% sodium hydrogen carbonate solution (15 mL) and extracted with EtOAc (3 x 20 mL). The combined organic phases were dried and concentrated *in vacuo*, and the residue was purified by flash chromatography (DCM/MeOH from 95:5 to 80:20) to give the title compound (190 mg) as a white solid.

T.I.c.: DCM/MeOH 8:2 containing 1% of conc. NH₄OH, Rf=0.38 (detection with ninhydrine).
MS (ES/+): m/z=437 [M+H]⁺.

### Example 43

### N-[1-(3,5-Dichloro-phenyl)-ethyl]-2-[4-(4-fluoro-phenyl)-1-methyl-piperidin-4-yl]-N-methyl-acetamide hydrochloride (enantiomer 1)

The compound of Example **42** (185 mg) was dissolved in dry Et2O (6 mL), cooled to 0°C and treated with hydrochloric acid (1M in Et2O - 465 µL). The mixture was stirred at 0°C for 1.5 hours, then it was filtered under a Nitrogen atmopsphere in a dry box. The solid was washed with Et2O (2 x 4 mL), and then pentane (3 x 8 mL) to give the title compound (150 mg) as a white solid.
NMR (d₆-DMSO): δ (ppm) 10.13 (bs, 1H); 7.43 (s, 1H); 7.36 (dd, 2H); 7.10 (t, 2H); 6.95 (s, 2H); 5,54 (q, 1H); 2.89 (d, 1H); 2.83 (d, 1H); 2.57 (s, 3H); 2.9-2.5 (m, 4H); 2.23 (s, 3H); 2.1-1.8 (m, 4H); 1.14 (d, 3H).
MS (ES/+): m/z=437 [MH-HCl]⁺.
HPLC (column: XTerra MSC18 50x4.6mm, 5µm; mobile phase: H₂O+0.1% TFA/CH₃CN+0.1 TFA from 90/10 to 10/90 in 12 min.; flow rate=1 mL/min; detection: λ=200-400 nm, ES+: 100-900): retention time = 8.4 minutes; purity (a/a %) >99%.

### Example 44

### N-[(3,5-Dichlorophenyl)methyl]-2-{4-(4-fluoro-2-methylphenyl)-1-[2-(methyloxy)ethyl]-4-piperidinyl}-N-methylacetamide

2-Bromoethyl methyl ether (2 µl) and DIPEA (13 µl) were added to a stirred solution of Example **8** (8.6 mg) in CH₃CN (0.5 mL) under a Nitrogen atmosphere. The mixture was heated to 80 °C for 4 hours, DIPEA (13 µL) was added and heated for additional 7 hours, then left to stirr at 23°C for 16 hours. The solvent was removed in vacuo, DCM (10 mL) and a 5% sodium hydrogen carbonate solution (10 mL) were added, and the aqueous extracted with DCM (2 x 10 mL). The combined organic phases were dried and concentrated *in vacuo*, and the residue was purified by flash chromatography (AcOEt/MeOH from 9:1 to 4:6) to give the title compound (5.7 mg) as a white solid.
T.I.c.: AcOEt/MeOH 6:4 containing, Rf=0.1 (detection with ninhydrine).
NMR (d₆-DMSO): δ (ppm) 7.45 (s, 1H); 7.26 (dd, 1H); 7.02 (s, 2H); 6.92-6.80 (m, 2H); 4.32 (s, 2H); 3.38 (m, 2H); 3.20 (s, 3H); 2.79 (s, 2H); 2.57 (s, 3H); 2.44 (s, 3H); 2.60-2.00 (bm, 10H).
MS (ES/+): m/z=481 [M+H]⁺.

### Example 45

### N-[(3,5-Dichlorophenyl)methyl]-2-{4-(4-fluoro-2-methylphenyl)-1-[2-(methyloxy)ethyl]-4-piperidinyl}-N-methylacetamide hydrochloride

The compound of Example **44** (2.8 mg) was dissolved in dry Et2O (0.2 mL), cooled to 0°C and treated with hydrochloric acid (1 M in Et2O - 6.5 µL). The mixture was stirred at 0°C for 5 minutes, then the solvent was removed *in vacuo* and the residue was triturated with Et2O (2 x 0.2 mL), then with pentane (1 x 0.5 mL) to give the title compound (3 mg) as a white solid.
MS (ES/+): m/z=481 [MH-HCl]⁺.

### Example 46

### N-{(1R)-1-[3,5-Bis(trifluoromethyl)phenyl]ethyl}-2-[4-(4-fluoro-2-methylphenyl)-4-piperidinyl]-N-methylacetamide

TFA (0.75 mL) was added to a solution of intermediate 70 (52.1 mg) in DCM (2.25 mL) under a Nitrogen atmosphere. The resulting solution was stirred at r.t. for 30 minutes, then 5% sodium hydrogen carbonate solution was added. The aqueous layer was extracted with AcOEt (2 x 5 mL). The combined organic extracts were dried, concentrated *in vacuo* and the residue was purified by flash chromatography (AcOEt, AcOEt/MeOH from 9:1 to 1:9, then MeOH) to give the title compound (34 mg) as a colourless oil.
NMR (d₆-DMSO - 60°C): δ (ppm) 7.92 (bs, 1H); 7.68 (s, 2H); 7.26 (m, 1H); 6.9 (m, 1 H); 6.8 (m, 1H); 5.71 (m, 1H); 2.81 (bs, 5H); 2.60 (m, 2H); 2.45-2.35 (m, 2H); 2.42 (s, 3H); 2.0 (m, 2H); 2.19 (m, 2H); 1.34 (d, 3H).
MS (ES/+): m/z=505 [M+H]⁺.

### Example 47

### N-[(3,5-Dibromophenyl)methyl]-2-[4-(4-fluoro-2-methylphenyl)-4-piperidinyl]-N-methylacetamide

TFA (1 mL) was added to a solution of intermediate 71 (26.5 mg) in DCM (4 mL) under a Nitrogen atmosphere. The resulting solution was stirred at r.t. for 30 minutes, then a 5% sodium hydrogen carbonate solution was added. The aqueous layer was extracted with AcOEt (2 x 5 mL). The combined organic extracts were dried, concentrated *in vacuo* and the residue was purified by flash chromatography (AcOEt, AcOEt/MeOH from 9:1 to 1:9, then MeOH) to give the title compound (12.5 mg) as a colourless oil.
NMR (CDCl₃): δ (ppm) 7.55 (bs, 1H); 7.14 (s, 2H); 7.27 (m, 1H); 6.9-6.7 (m, 2H); 5.5-5.3 (b, 1H); 4.32 (s, 2H); 3.06 (m, 2H); 2.82 (bs, 3H); 2.73 (s, 2H); 2.85 (m, 2H); 2.56 (m, 2H);
2.48 (s, 3H); 2.14 (m, 2H).
MS (ES/+): m/z=511 [M+H]⁺

### Example 48

### N-{[3,5-Bis(trifluoromethyl)phenyl]methyl}-2-[4-(4-fluoro-2-methylphenyl)-1-methyl-4-piperidinyl]-N-methylacetamide

A solution of formaldehyde in water (37% w/w: 9 µL) was added to a stirred solution of Example 6 (30 mg) in CH₃CN (1.7 mL) under a Nitrogen atmosphere at 23°C. After 20 minutes sodium triacetoxyborohydride (19.4 mg) was added. The mixture was stirred for further 3 hours then it was quenched with water and extracted with AcOEt (3 x 10 mL). The combined organic phases were dried and concentrated *in vacuo,* and the residue was purified by flash chromatography (DCM, DCM/MeOH from 95:5 to 70:30) to give the title compound (19 mg) as a colourless oil.
NMR (d₆-DMSO): δ (ppm) 7.98 (s, 1H); 7.73 (s, 2H); 7.19 (t, 1H); 6.90 (d, 1H); 6.76 (t, 1H); 4.45 (s, 2H); 3.15-2.43 (m, 6H); 3.15-2.43 (m, 4H); 2.62 (s, 3H); 2.5 (s, 3H); 2.43 (s, 3H).
MS (ES/+): m/z=505 [M+H]⁺.

### Example 49

### N-{[3,5-Bis(trifluoromethyl)phenyl]methyl}-2-[4-(4-fluoro-2-methylphenyl)-1-methyl-4-piperidinyl]-N-methylacetamide hydrochloride

The Example **48** (15 mg) was dissolved in anhydrous Et2O (1 mL) and treated with hydrochloric acid (1M in Et2O - 0.327 mL) at 0°C under a Nitrogen atmosphere. The mixture was stirred at 0°C for 30 minutes, then it was concentrated *in vacuo* and the residue was triturated with pentane (3 x 1 mL) to give the title compound (10.3 mg) as a white solid.
MS (ES/+): m/z=505 [MH-HCl]⁺.

### Example 50

### N-[(3,5-Dichlorophenyl)methyl]-2-[4-(4-fluoro-2-methylphenyl)-1-methyl-4-piperidinyl]-N-methylacetamide

A solution of formaldehyde in water (37% w/w; 10.2 µL) was added to a stirred solution of Example **7** (29 mg) in CH₃CN (2 ml) under a Nitrogen atmosphere at 23°C. After 30 minutes sodium triacetoxyborohydride (22 mg) was added. The mixture was stirred for further 2 hours then it was quenched with water and extracted with AcOEt (2 x 10 mL).

The combined organic phases were dried and concentrated *in vacuo*, and the residue was purified by flash chromatography (DCM, DCM/MeOH from 9:1 to 1:1) to give the title compound (16 mg) as a colourless oil.
NMR (d₆-DMSO): δ (ppm) 7.45 (s, 1H); 7.26 (dd, 1H); 7.01 (s, 2H); 6.93/6.84 (m, 2H); 4.28 (s, 2H); 2.84/2.26 (m, 6H); 2.57 (s, 3H); 2.5 (s, 3H); 2.45 (s, 3H); 2.84-2.26 (m, 4H).
MS (ES/+): m/z=437 [M+H]⁺.

### Examale 51

### N-{[3,5-Bis(trifluoromethyl)phenyl]methyl}-2-[4-(4-fluoro-2-methylphenyl)-4-piperidinyl]acetamide

TFA (0.5 mL) was added to a solution of intermediate **72** (153 mg) in DCM (2 mL) under a Nitrogen atmosphere. The resulting solution was stirred at r.t. for 30 minutes, then a satured potassium carbonate solution was added. The aqueous layer was extracted with AcOEt (2 x 10 mL). The combined organic extracts were dried, concentrated *in vacuo* and the residue was purified by flash chromatography (DCM, DCM/MeOH from 9:1 to 1:9, then MeOH) to give the title compound (113 mg) as a colourless oil.
NMR (d₆-DMSO): δ (ppm) 8.20 (t, 1H); 7.93 (s, 1H); 7.74 (s, 2H); 7.15 (dd, 1H); 6.79 (dd, 1H); 6.75 (td, 1H); 4.22 (d, 2H); 2.74 (m, 2H); 2.56 (s, 2H); 2.54 (bm, 2H); 2.42 (s, 3H); 2.09 (bm, 2H); 1.94 (bm, 2H).
MS (ES/+): m/z= 477 [M+H]⁺

### Example 52

### N-{[3,5-Bis(trifluoromethyl)phenyl]methyl}-2-[4-(4-fluoro-2-methylphenyl)-1-methyl-4-piperidinyl]acetamide

A solution of formaldehyde in water (37% w/w; 9.1 µL) was added to a stirred solution of Example **51** (29 mg) in CH₃CN (1.6 mL) under a Nitrogen atmosphere at 23°C. After 30 minutes sodium triacetoxyborohydride (19 mg) was added. The mixture was stirred for further 1 hour then it was quenched with water and extracted with AcOEt (2 x 10 mL). The combined organic phases were dried and concentrated *in vacuo*, and the residue was purified by flash chromatography (DCM, DCM/MeOH from 9:1 to 1:1) to give the title compound (15 mg) as a colourless oil.
NMR (CDCl₃): δ (ppm) 7.77 (s, 1H); 7.53 (s, 2H); 7.17 (dd, 1H); 6.81 (dd, 1H); 6.71 (t, 1H); 5.00 (m, 1H); 4.23 (d, 2H); 2.72-2.12 (m, 10H); 2.5 (s, 3H); 2.26 (s, 3H).
MS (ES/+): m/z= 491 [M+H]⁺.

### Example 53

### N-[(3,5-Dibromophenyl)methyl]-2-[4-(4-fluoro-2-methylphenyl)-1-methyl-4-piperidinyl]-N-methylacetamide

A solution of formaldehyde in water (37% w/w; 0.5 mL) was added to a stirred solution of Example **47** (210 mg) in DCM (10 mL) under a Nitrogen atmosphere at 23°C. After 5 minutes sodium triacetoxyborohydride (130 mg) was added. The mixture was stirred for 18 hours then it was quenched with water (5 mL) and the layers separated. The organic layer was washed with brine (10 mL), dried and concentrated *in vacuo* and the residue was triturated with Et2O (10 mL). The material was dissolved in DCM (10 mL), washed with a saturated potassium carbonate solution (2 x 10 mL), dried and concentrated in *vacuo* to yield the title compound (84 mg) as a white foam.
NMR (d₆-DMSO): δ (ppm) 7.69 (s, 1H); 7.23 (m, 3H); 6.89 (bd, 1H); 6.84 (bt, 1H); 4.30 (s, 2H); 2.79 (bs, 2H); 2.6-2.0 (m, 8H); 2.44 (s, 3H); 2.54 (s, 3H); 2.07 (s, 3H).
MS (ES/+): m/z=527 [M+H]⁺

### Example 54

### N-[(3,5-Dibromophenyl)methyl]-N-methyl-2-[4-(2-methylphenyl)-4-piperidinyl]acetamide

TFA (4 mL) was added to a solution of intermediate 75 (230 mg) in DCM (16 mL) under a Nitrogen atmosphere. The resulting solution was stirred at -20°C overnight, then a 10% potassium hydroxide solution was added until pH=9. The aqueous layer was extracted with DCM (2 x 20 mL). The combined organic extracts were dried, concentrated *in vacuo* and the residue was purified by flash chromatography (AcOEt/MeOH from 8:2 to1:1 containing 0.5% of conc. ammonium hydroxide solution) to give the title compound (156 mg) as a colourless oil.
T.I.c.: DCM/MeOH 8:2 containing 0.5% of conc. NH₄OH, Rf=0.09.
NMR (CDCl₃): δ (ppm) 7.7-7.5 (m, 1H); 7.27 (dd, 1H); 7.2-6.96 (m, 5H); 4.27 (s, 2H);3.04-2.94 (m, 2H); 2.78 (s, 2H); 2.78 (m, 2H); 2.68 (s, 3H); 2.38 (s, 3H); 2.55 (m, 2H); 2.06 (m, 2H).
MS (ES/+): m/z=495 [M+H]⁺.

### Example 55

### N-[(3,5-Dibromophenyl)methyl]-N-methyl-2-[1-methyl-4-(2-methylphenyl)-4-piperldinyl]acetamide

Sodium triacethoxyborohydride (50 mg) was added to a solution of Example 54 (50 mg) in anhydrous CH₃CN (6 mL) with formaldehyde in water (37% w/w, 25 µL) under a Nitrogen atmosphere. The mixture was stirred at r.t. for 2 hours, then the solvent was removed in *vacuo* and the residue was dissolved in 5% sodium hydrogen carbonate solution (10 mL) and extracted with DCM (2 x 15mL). The organic layer was dried, concentrated *in vacuo* and the residue was purified by flash chromatography (DCM, DCM/MeOH from 9:1 to 1:1) to give the title compound (14 mg) as a white solid. T.I.c.: DCM/MeOH 8:2 containing 0.5% of conc. NH₄OH, Rf=0.72 (detection with ninhydrine).
NMR (CDCl₃): δ (ppm) 7.7-7.5 (m, 1H); 7.32 (dd, 1H); 7.2-6.96 (m, 5H); 4.25 (s, 2H);2.8-2.5 (bs, 6H); 2.26 (s, 3H); 2.29 (s, 3H); 2.19 (s, 3H); 2.3 (bs, 2H); 2.2-2.1 (bs, 2H).
MS (ES/+): m/z=509 [M+H]⁺.

### Example 56

### N-[(3,5-Dichlorophenyl)methyl]-2-[4-(4-fluorophenyl)-1-methyl-4-piperidinyl]-N-methylacetamide

A solution of formaldehyde in water (37% w/w; 100 µL) was added to a stirred solution of Example **1** (20.1 mg) in DCM (1 mL) under a Nitrogen atmosphere at 23°C. After 5 minutes sodium triacetoxyborohydride (14.3 mg) was added. The mixture was stirred for 18 hours then it was diluted with DCM (5 mL), washed with saturated potassium carbonate solution (5 mL) and brine (5 mL). The organic phase was dried and concentrated *in vacuo*, and the residue was resubmit to reaction with a solution of formaldehyde in water (37% w/w; 100 µL) and triacetoxyborohydride (14.3 mg) in DCM (1 mL) under a Nitrogen atmosphere at 23°C. The mixture was stirred for 18 hours then it was diluted with DCM (5 mL), washed with saturated potassium carbonate solution (5 mL) and brine (5 mL). The organic phase was dried and concentrated *in vacuo* to give the title compound (12.7 mg) as a yellow oil.
MS (ES/+): m/z=423 [M+H]⁺

### Example 57

### N-{[3,5-Bis(trifluoromethyl)phenyl]methyl}-2-[4-(4-fluorophenyl)-4-piperidinyl]-N-methylacetamide

TFA (6 mL) was added to a solution of intermediate **76** (590 mg) in DCM (24 mL) previously cooled to 0°C under a Nitrogen atmosphere. The resulting solution was stirred under these conditions for 2 hours, then a 10% sodium hydroxide solution was added until pH=12. The aqueous layer was extracted with DCM (2 x 15 mL) which was passed through a phase separation cartridge with polypropylene frit and concentrated *in vacuo.*

The residue was purified by SCX cartridge (load with DCM and elution with MeOH, NH₃ 0.2M in MeOH) to give the title compound (348 mg) as a white foam.
T.I.c.: DCM/MeOH 8:2 containing 0.5% of conc. NH₄OH, Rf=0.07.
MS (ES/+): m/z=477 [M+H]⁺.

### Example 58

### N-{[3,5-Bis(trifluoromethyl)phenyl]methyl}-2-[4-(4-fluorophenyl)-1-methyl-4-piperidinyl]-N-methylacetamide

Sodium triacethoxyborohydride (126 mg) was added to a solution of Example **57** (100 mg) in anhydrous CH₃CN (5 mL) with a solution of formaldehyde in water (37% w/w, 60 µL) under a Nitrogen atmosphere. The mixture was stirred at r.t. for 2 hours, then the solvent was removed *in vacuo* and the residue was dissolved in 5% sodium hydrogen carbonate solution (5 mL) and extracted with DCM (2 x 8 mL), which was passed through a phase separation cartridge with polypropylene frit and concentrated *in vacuo*. The residue was purified by flash chromatography (DCM, DCM/MeOH from 95:5 to 1:1) to give the title compound (32 mg) as a white solid. T.I.c.: DCM/MeOH 8:2 containing 0.5% of conc. NH₄OH, Rf=0.61 (detection with ninhydrine).
NMR (d₆-DMSO): δ (ppm) 7.97 (s, 1H); 7.73 (s, 2H); 7.27 (dd, 2H); 6.89 (t, 2H); 4.41 (s, 2H); 2.65-2.10 (m, 10H); 2.50 (s, 3H); 2.05 (s, 3H).
MS (ES/+): m/z=491 [M+H]⁺.

### Example 59

### N-[1-(3,5-Dibromophenyl)-1-methylethyl]-2-[4-(4-fluoroohenyl)-4-piperidinyl]-N-methylacetamide

TFA (3 mL) was added to a solution of intermediate **78** (265 mg) in DCM (12 mL) previously cooled to 0°C under a Nitrogen atmosphere. The resulting solution was stirred under these conditions for 2 hours, then a 10% sodium hydroxide solution was added until pH=12. The aqueous layer was extracted with DCM (2 x 15 mL) which was passed through a phase separation cartridge with polypropylene frit and concentrated *in vacuo.*
The residue was purified by SCX cartridge (load with DCM and elution with MeOH, NH₃ 0.2M in MeOH) to give the title compound (118 mg) as a white foam.
T.I.c.: DCM/MeOH 8:2 containing 0.5% of conc. NH₄OH, Rf=0.11.
MS (ES/+): m/z=527 [M+H]⁺.

### Example 60

### N-[1-(3,5-Dibromophenyl)-1-methylethyl]-2-[4-(4-fluorophenyl)-1-methyl-4-piperidinyl]-N-methylacetamide

Sodium triacethoxyborohydride (126 mg) was added to a solution of Example **59** (105 mg) in anhydrous CH₃CN (5 mL) with a solution of formaldehyde in water (37% w/w, 60 µL) under a Nitrogen atmosphere. The mixture was stirred at r.t. for 2 hours, then the solvent was removed *in vacuo* and the residue was dissolved in 5% sodium hydrogen carbonate solution (5 mL) and extracted with DCM (2 x 8 mL), which was passed through a phase separation cartridge with polypropylene frit and concentrated *in vacuo.* The residue was purified by flash chromatography (DCM, DCM/MeOH from 95:5 to 1:1) to give the title compound (51 mg) as a white solid.
T.I.c.: DCM/MeOH 8:2 containing 0.5% of conc. NH₄OH, Rf=0.50 (detection with ninhydrine).
NMR (d₆-DMSO): δ (ppm) 7.52 (s, 1H); 7.32 (dd, 2H); 7.21 (s, 2H); 7.11 (t, 2H); 2.60 (s, 3H); 2.35 (bs, 2H); 2.2-2.0 (m, 6H); 1.91 (m, 2H); 2.49 (s, 3H); 1.30 (s, 6H).
MS (ES/+): m/z=541 [M+H]⁺.

### Example 61

### N-[1-(R)-1-(3,5-Bis-trifluoromethyl-phenyl)-ethyl]-2-[4-(4-fluoro-phenyl)-piperidin-4-yl]-N-methyl-acetamide

TFA (6 mL) was added to a solution of intermediate **79** (700 mg) in DCM (24 mL) previously cooled to 0°C under a Nitrogen atmosphere. The resulting solution was stirred under these conditions for 2 hours, then a 10% sodium hydroxide solution was added until pH=12. The aqueous layer was extracted with DCM (2 x 15 mL) which was passed through a phase separation cartridge with polypropylene frit and concentrated *in vacuo.*
The residue was purified by SCX cartridge (load with DCM and elution with MeOH, NH₃ 0.2M in MeOH) to give the title compound (469 mg) as a white foam.
T.I.c.: DCM/MeOH 8:2 containing 0.5% of conc. NH₄OH, Rf=0.15. MS (ES/+): m/z=491 [M+H]⁺.

### Example 62

### N-[1-(R)-1-(3,5-Bis-trifluoromethyl-phenyl)-ethyl]-2-[4-(4-fluoro-phenyl)-1-methyl-piperidin-4-yl]-N-methyl-acetamide

Sodium triacethoxyborohydride (126 mg) was added to a solution of Example **61** (100 mg) in anhydrous CH₃CN (5 mL) with a solution of formaldehyde in water (37% w/w, 60 µL) under a Nitrogen atmosphere. The mixture was stirred at r.t. for 2 hours, then the solvent was removed *in vacuo* and the residue was dissolved in 5% sodium hydrogen carbonate solution (5 mL) and extracted with DCM (2 x 8 mL), which was passed through a phase separation cartridge with polypropylene frit and concentrated *in vacuo.* The residue was purified by flash chromatography (DCM, DCM/MeOH from 95:5 to 8:2) to give the title compound (44 mg) as a white solid.
T.I.c.: OCM/MeOH 8:2 containing 0.5% of conc. NH₄OH, Rf=0.49 (detection with ninhydrine).
NMR (COCl₃): δ (ppm) 7.73 (s, 1H); 7.52 (s, 2H); 7.26 (dd, 2H); 6.93 (t, 2H); 5.95 (q, 1H); 2.76 (bs, 2H); 2.56-2.20 (m, 6H); 2.65 (s, 2H); 2.32 (s, 3H); 2.15 (s, 3H); 1.32 (d, 3H).
MS (ES/+): m/z=505 [M+H]⁺.

### Example 63

### 2-[1-(Cyclopropylmethyl)-4-(4-fluorophenyl)-4-piperidinyl]-N-[(3,5-dibromophenyl)methyl]-N-methylacetamide

Sodium triacethoxyborohydride (32 mg) was added to a solution of Example **16** (50 mg) in anhydrous CH₃CN (3 mL) with cyclopropylcarboxyaldehyde (15 µL) under a Nitrogen atmosphere. The mixture was stirred at r.t. for 1.5 h, then the solvent was removed in *vacuo* and the residue was dissolved in 5% sodium hydrogen carbonate solution (5 mL) and extracted with DCM (2 x 8 mL), which was passed through a phase separation cartridge with polypropylene frit and concentrated *in vacuo.* The residue was purified by flash chromatography (DCM, DCM/MeOH 95:5) to give the title compound (24 mg) as a white solid.
T.I.c.: DCM/MeOH 8:2 containing 1% of conc. NH₄OH, Rf=0.94 (detection with ninhydrine).
NMR (CDCl₃): δ (ppm) 7.56 (s, 1 H);7.30 (dd, 2H); 7.16 (s, 2H); 7.00 (t, 2H); 4.30 (s, 2H); 2.80 (bs, 2H); 2.65 (s, 3H); 2.36 (bs, 2H); 2.5-2.1 (bs, 8H); 0.88 (bs, 1H); 0.52 (d, 2H).; 0.08 (d, 2H).
MS (ES/+): m/z=553 [M+H]⁺.

### Example 64

### 2-[4-{2-[[(3,5-Dibromophenyl)methyl](methyl)amino]-2-oxoethyl}-4-(4-fluorophenyl)-1-piperidinyl]-N,N-dimethylacetamide

2-Chloro-N,N-dimethylacetamide (17 µL) was added to a solution of Example **16** (50 mg) in anhydrous CH₃CN (3 mL) with DIPEA (17 µL) under a Nitrogen atmosphere. The mixture was stirred at r.t. for 52 h, then 5% sodium hydrogen carbonate solution (5 mL) was added and extracted with AcOEt (2 x 8 mL). The organic phase was then separated, dried and concentrated *in vacuo.* The residue was purified by flash chromatography (DCM, DCM/MeOH from 95:5 to 9:1) to give the title compound (16 mg) as a white foam.

T.I.c.: DCM/MeOH 8:2 containing 1% of conc. NH₄OH, Rf=0.72 (detection with ninhydrine).
NMR (CDCl₃): δ (ppm) 7.52 (s,1H);7.24 (dd, 2H); 7.13 (s, 2H); 6.95 (t, 2H); 4.30 (s, 2H); 3.12 (s, 2H); 3.06(s, 3H); 2.94 (s, 3H); 2.72 (s, 2H);2.64 (s, 2H); 2.36 (bs, 4H);2.36 (s, 3H);2.18 (t, 2H).
MS (ES/+): m/z=584 [M+H]⁺.

### Example 65

### N-[(3,5-Dibromophenyl)methyl]-2-[1-ethyl-4-(4-fluorophenyl)-4-piperidinyl]-N-methylacetamide

Sodium triacethoxyborohydride (32 mg) was added to a solution of Example **16** (50 mg) in anhydrous CH₃CN (3 mL) with acetaldehyde (11 µL) under a Nitrogen atmosphere. The mixture was stirred at r.t. for 1.5 hour, then the solvent was removed *in vacuo* and the residue was dissolved in 5% sodium hydrogen carbonate solution (5 mL) and extracted with DCM (2 x 8 mL), which was passed through a phase separation cartridge with polypropylene frit and concentrated *in vacuo.* The residue was purified by preparative HPLC (column: X Terra MS C18 50x4.6mm, 5µm; mobile phase: A: H₂O+0.1% TFA; B: CH₃CN+0.1% TFA; gradient: 30% (B) for 3 min, from 30% (B) to 70% (B) in 10 min.; flow rate=17 mL/min; detection: A=200-400 nm); the solution recovered was concentrated *in vacuo*, and the residue was dissolved in 5% sodium hydrogen carbonate solution (5 mL) and extracted with DCM (2 x 8 mL), which was passed through a phase separation cartridge with polypropylene frit and concentrated *in vacuo*, to give the title compound (12 mg) as white foam.
T.I.c.: DCM/MeOH 8:2 containing 1% of conc. NH₄OH, Rf=0.24 (detection with ninhydrine).
NMR (d₆-DMSO): □ (ppm) 7.72 (s, 1H); 7.36 (dd, 2H); 7.26 (s, 2H); 7.04 (t, 2H); 4.27 (s, 2H); 2.67 (s, 2H); 2.45-2.55 (bm, 2H); 2.49 (s, 3H); 2.23 (q, 2H); 2.1-2.3 (bm, 2H); 2.3-2.0 (bm, 4H); 0.96 (t, 3H).
MS (ES/+): m/z=527 [M+H]⁺.

### Example 66

### N-{(1R)-1-[3,5-Bis(trifluoromethyl)phenyl]ethyl}-2-[4-(4-fluorophenyl)hexahydro-1H-azepin-4-yl]-N-methylacetamide

TFA (1.5 mL) was added to a solution of intermediate **80** (200 mg) in anhydrous DCM (6 mL) previously cooled at 0°C, under a Nitrogen atmosphere. The solution was stirred at 0°C for 2 hours, then it was concentrated *in vacuo* at 0°C. The residue was taken up with a saturated potassium carbonate solution (10 mL) and diluited with AcOEt (50 mL). The organic phase was separated and the aqueous layer was extracted with AcOEt (2 x 50 mL). The collected organic phases were dried and concentrated *in vacuo* to give the title compound (180 mg) as a white foam.
T.I.c.: DCM/MeOH 8:2, containing 0.3% of conc. NH₄OH, Rf=0.15 (detection with ninhydrine).
MS (ES/+): m/z=505 [M+H]⁺.

### Example 67

### N-{(1R)-1-[3,5-Bis(trifluoromethyl)phenyl]ethyl}-2-[4-(4-fluorophenyl)-1-methylhexahvdro-1H-azepin-4-yl]-N-methylacetamide

A solution of formaldehyde in water (37% w/w; 40 µL) was added to a stirred solution of Example **66** (90 mg) in CH₃CN (3 mL) under a Nitrogen atmosphere at 23°C. After 30 minutes sodium triacetoxyborohydride (40 mg) was added. The mixture was stirred for further 2 hours, then it was quenched with a saturated sodium hydrogen carbonate solution (5 mL) and extracted with AcOEt (3 x 50 mL). The combined organic phases were dried and concentrated *in vacuo*, and the residue was purified by flash chromatography (DCM/MeOH from 9:1 to 8:2, then from 8:2 containing 0.3% of conc. NH₄OH to 7:3 containing 0.3% of conc. NH₄OH) to give the title compound (75 mg) as a white solid foam.
T.I.c.: DCM/MeOH 8:2 containing 1% of conc. NH₄OH, Rf=0.65 (detection with ninhydrine).
NMR (CDCl₃): δ (ppm) 7.7 (s, 1H), 7.51 (s, 2H), 7.27 (dd, 2H), 6.92 (t, 2H), 5.9 (q, 1H), 2.65 (m, 3H), 2.4 (m, 3H), 2.25 (bm, 2H), 2.3 (s, 2H), 2.2 (s, 3H), 2.15 (s, 3H), 1.7 (m, 2H), 1.35 (d, 3H).
MS (ES/+): m/z=519 [M+H]⁺.

### Example 68

### N-[(3,5-Dibromophenyl)methyl]-2-[4-(4-fluorophenyl)hexahydro-1H-azepin-4-yl]-N-methylacetamide

TFA (1.5 mL) was added to a solution of intermediate **81** (160 mg) in anhydrous DCM (6 mL) previously cooled at 0°C, under a Nitrogen atmosphere. The solution was stirred at 0°C for 2 hours, then it was concentrated *in vacuo* at 0°C. The residue was taken up with a 10% hydrogen sodium carbonate solution (10 mL) and diluited with DCM (10 mL). The organic phase was separated and the aqueous layer was extracted with DCM (3 x 10 mL). The collected organic phases were washed with a 5% hydrogen sodium carbonate solution (10 mL) and brine (5 mL), dried and concentrated *in vacuo* to give the title compound (110 mg) as a white foam.
T.I.c.: DCM/MeOH 6:4, containing 0.5% of conc. NH₄OH, Rf=0.12 (detection with ninhydrine).
MS (ES/+): m/z=513 [M+H]⁺.

### Example 69

### N-[(3,5-Dibromophenyl)methyl]-2-[4-(4-fluorophenyl)-1-methylhexahydro-1H-azepin-4-yl]-N-methylacetamide

A solution of formaldehyde in water (37% w/w; 30 µL) was added to a stirred solution of Example **68** (102.6 mg) in CH₃CN (5 mL) under a Nitrogen atmosphere at 23°C. After 10 minutes sodium triacetoxyborohydride (63.6 mg) was added. The mixture was stirred for 3 hours, then a solution of formaldehyde in water (37% w/w; 30 µL) and sodium triacetoxyborohydride (63.6 mg) in CH₃CN (5 mL) were added. After stirring for 1 hour, the mixture was quenched with a saturated sodium hydrogen carbonate solution (0.5 mL) and solvent evaporated *in vacuo.* The residue was taken up with AcOEt (20 mL), washed with a saturated sodium hydrogen carbonate solution (10 mL) and the aqueous phase extracted woth AcOEt (2 x 10 mL). The combined organic phases were dried and concentrated *in vacuo*, and the residue was purified by flash chromatography (DCM/MeOH from 9:1 to 8:2, then 6:4 containing 0.5% of conc. NH₄OH) to give the title compound (78.7 mg) as a white foam.
T.I.c.: DCM/MeOH 6:4 containing 1% of conc. NH₄OH, Rf=0.36 (detection with ninhydrine).
NMR (CDCl₃): δ (ppm) 7.56 (s, 1H); 7.28-7.04 (m, 2H); 7.17 (s, 2H); 6.99 (t, 2H), 4.33 (s, 2H), 2.52 (s, 3H), 2.42 (s, 3H), 2.81-1.68 (bm, 12H).
MS (ES/+): m/z=527 [M+H]⁺.

### Example 70

### N-[1-(3,5-Dibromo-phenyl)-ethyl]-2-[4-(4-fluoro-phenyl)-piperidin-4-yl]-N-methylacetamide hydrochloride (enantiomer 1)

The compound of Example **32** (261 mg) was dissolved in dry Et2O (5 mL), cooled to 0°C and treated with hydrochloric acid (1 M in Et2O - 560 µL). The mixture was stirred at 0°C for 1 hour, then the solvent was removed *in vacuo* and the residue was triturated with pentane (5 x 1 mL) to give the title compound (135 mg) as a white solid.
NMR (d₆-DMSO): δ (ppm) 8.65-8.55 (bm, 2H); 7.72 (s, 1H); 7.43 (dd, 2H); 7.24 (s, 2H); 5.60 (q, 1H); 3.22 (bm, 2H); 2.88 (bm, 2H); 2.78 (d, 1H); 2.73 (d, 1H); 2.2-2.4 (bm, 4H); 2.21 (s, 3H); 1.21 (d, 3H).
MS (ES/+): m/z=513 [MH-HCl]⁺.

### Example 71

### N-[(3-Bromo-5-cyanophenyl)methyl]-2-[4-(4-fluorophenyl)-4-piperidinyl]-N-methylacetamide

TFA (0.5 mL) was added to a solution of intermediate **84** (54 mg) in DCM (2 mL) previously cooled at 0°C under a Nitrogen atmosphere. The resulting solution was stirred at r.t. for 2 hours, then a saturated sodium hydrogen carbonate solution was added. The organic layer was passed through a phase separation cartridge with polypropylene frit and concentrated *in vacuo*, the residue was purified by SCX cartridge (load with MeOH and elution with NH3 0.25M in MeOH) to give the title compound (39 mg) as a white foam.
NMR (CDCl₃): δ (ppm) 8.02 (s, 1H); 7.56 (s, 1H); 7.46 (s,1H); 7.35 (dd, 2H); 7.05 (t, 2H); 4.30 (s, 2H); 6.0-5.0 (vbs, 1 H); 3.0 (t, 2H); 2.8 (t, 2H); 2.6 (s, 2H); 2.4 (m, 5H); 2.1 (m, 2H).
IR (nujol, cm-1): 1634.96 (C=O), 2232.28 (C≡N).

### Example 72

### N-[(3-Bromo-5-cyanophenyl)methyl]-2-[4-(4-fluorophenyl)-1-methyl-4-piperidinyl]-N-methylacetamide

Sodium triacethoxyborohydride (25 mg) was added to a solution of Example **71** (31 mg) in anhydrous CH₃CN (3.8 mL) with a solution of formaldehyde in water (37% w/w, 50 µL) under a Nitrogen atmosphere. The mixture was stirred at r.t. 3 hours, then it was diluted with saturated sodium hydrogen carbonate solution and DCM and the organic layer passed through a phase separation cartridge with polypropylene frit and concentrated *in vacuo*. The residue was purified by SCX cartridge (load with MeOH and elution with NH₃ 0.25M in MeOH) to give the title compound (17 mg) as a white solid.
NMR (CDCl₃): δ (ppm) 8.04 (s, 1H); 7.58 (s, 1H); 7.48 (s, 1H); 7.36 (dd, 2H); 7.04 (dd, 2H); 4.31 (s,2H); 2.68 (s, 2H); 2.51 (s, 3H); 2.4-2.6 (bm, 2H); 2.0-2.3 (bm, 2H); 2.0-2.3 (bm, 4H); 2.11 (s, 3H).
MS (ES/+): m/z=458 [M+H]⁺.

### Example 73

### N-[(3.5-Dibromophenyl)methyl]-N-methyl-2-{4-[3-(trifluoromethyl)phenyl]-4-piperidinyl}acetamide

TFA (3 mL) was added to a solution of intermediate **87** (270 mg) in DCM (12 mL) previously cooled at 0°C under a Nitrogen atmosphere. The resulting solution was stirred at 0°C for 1 hour, then a 10% potassium hydroxide solution was added until pH=9. The aqueous layer was extracted with DCM (2 x 20 mL). The combined organic extracts were dried, concentrated *in vacuo* to give the title compound (205 mg) as a colourless oil.
MS (ES/+): m/z=549 [M+H]⁺.

### Example 74

### N-[(3,5-Dibromophenyl)methyl]-N-methyl-2-{1-methyl-4-[3-(trifluoromethyl)phenyl]-4-piperidinyl}acetamide

Sodium triacethoxyborohydride (210 mg) was added to a solution of Example **73** (205 mg) in anhydrous CH₃CN (7.5 mL) with formaldehyde in water (37% w/w, 110 µL) under a Nitrogen atmosphere. The mixture was stirred at r.t. overnight, then the solvent was removed *in vacuo* and the residue was dissolved in 5% sodium hydrogen carbonate solution (15 mL) and extracted with DCM (2 x 15mL). The organic layer was dried, concentrated *in vacuo* and the residue was purified by flash chromatography (DCM, DCM/MeOH from 9:1 to 8:2 containing 0.5% of conc. NH₄OH to give the title compound (152 mg) as a white solid.
T.I.c.: DCM/MeOH 8:2 containing 0.5% of conc. NH₄OH, Rf=0.67 (detection with ninhydrine).
NMR (d₆-DMSO): δ (ppm) 7.7-7.4 (m, 5H); 7.15 (s, 2H); 4.19 (s, 2H); 2.73 (s, 2H); 2.52 (bm, 2H); 2.06 (s, 3H); 2.5-2.0 (m, 8H).
MS (ES/+): m/z=563 [M+H]⁺.

### Example 75

### N-[(3,5-Dibromophenyl)methyl]-2-[4-(3,4-dimethylphenyl)-piperidinyl]-N-methylacetamide

TFA (3 mL) was added to a solution of intermediate **90** (262 mg) in DCM (12 mL) previously cooled at 0°C under a Nitrogen atmosphere. The resulting solution was stirred at 0°C for 1 hour, then a 10% potassium hydroxide solution was added until pH=9. The aqueous layer was extracted with DCM (2 x 20 mL). The combined organic extracts were dried, concentrated *in vacuo* to give the title compound (215 mg) as a colourless oil
MS (ES/+): m/z=509 [M+H]⁺.

### Example 76

### N-[(3,5-Dibromophenyl)methyl]-2-[4-(3,4-dimethylphenyl)-1-methyl-4-piperidinyl]-N-methylacetamide

A solution of formaldehyde in water (37% w/w; 94 µL) was added to a stirred solution of Example **75** (215 mg) in CH₃CN (8.5 mL) under a Nitrogen atmosphere at 23°C. After 30 minutes sodium triacetoxyborohydride (179 mg) was added. The mixture was stirred for 24 hours then a solution of formaldehyde in water (37% w/w; 31 µL) and sodium triacetoxyborohydride (60 mg) were added. The mixture was stirred at 23°C for 5 hours then the solvent was removed *in vacuo* and the residue was dissolved in a saturated sodium hydrogen carbonate solution (5 mL) and extracted with DCM. The combined organic phases were dried and concentrated *in vacuo*, and the residue was purified by flash chromatography (DCM/MeOH from 95:5 to 8:2), to give the title compound (139 mg) as a vitreous solid.
NMR (CDCl₃): δ (ppm) 7.51 (s, 1H); 7.17 (s, 2H); 7.2-6.9 (m, 3H); 4.24 (s, 2H); 2.58 (s, 3H); 2.65-2.0 (m, 19H).
MS (ES/+): m/z=523 [M+H]⁺.

### Example 77

### N-[1-(3,5-Dichlorophenyl)ethyl]-2-[4-(3-fluorophenyl)-4-piperidinyl]-N-methylacetamide (enantiomer 1)

TFA (1.5 mL) was added to a solution of intermediate **93** (135 mg) in DCM (6 mL) previously cooled to 0°C under a Nitrogen atmosphere. The resulting solution was stirred in these conditions for 2 hours, then a 10% sodium hydroxide solution was added until pH=12. The aqueous layer was extracted with DCM (2 x 8 mL) which was passed through a phase separation cartridge with polypropylene frit and concentrated *in vacuo*. to give the title compound (116 mg) as a white foam.
T.I.c.: DCM/MeOH 8:2 containing 0.5% of conc. NH₄OH, Rf=0.46.
MS (ES/+): m/z=423 [M+H]⁺.

### Example 78

### N-[1-(3,5-Dichlorochenyl)ethyl]-2-[4-(3-fluorophenyl)-1-methyl-4-pioeridinyl]-N-methylacetamide (enantiomer 1)

Sodium triacethoxyborohydride (167 mg) was added to a solution of Example **77** (116 mg) in anhydrous CH₃CN (5 mL) with a solution of formaldehyde in water (37% w/w, 78 µL) under a Nitrogen atmosphere. The mixture was stirred at r.t. for 2 hours, then the solvent was removed *in vacuo* and the residue was dissolved in 5% sodium hydrogen carbonate solution (5 mL) and extracted with DCM (2 x 8 mL), which was passed through a phase separation cartridge with polypropylene frit and concentrated *in vacuo.* The residue was purified by flash chromatography (DCM, DCM/MeOH from 95:5 to 1:1) to give the title compound (80 mg) as a white solid.
T.I.c.: DCM/MeOH 8:2 containing 0.5% of conc. NH₄OH, Rf=0.61 (detection with ninhydrine).
NMR (d₆-DMSO): δ (ppm) 7.44 (s,1H);7.28 (q, 1H); 7.17-7.12 (m, 2H); 7.00-6.97 (s, 2H); 7.00-6.97 (m, 1H); 5.56 (q, 1 H); 2.60 (s, 3H); 2.7-2.0 (m, 8H); 2.2 (s, 3H); 2.1 (s, 2H); 1.2 (d, 3H).
MS (ES/+): m/z=437 [M+H]⁺.

### Example 79

### N-[1-(3,5-Dichlorophenyl)ethyl]-2-[4-(3-fluorophenyl)-1-methyl-4-piperidinyl]-N-methylacetamide hydrochloride (enantiomer 1)

Example **78** (69 mg) was dissolved in anhydrous Et2O (1.5 mL) and treated with hydrochloric acid (1M in Et2O - 174 µL) at 0°C under a Nitrogen atmosphere. The mixture was stirred at 0°C for 30 minutes, then it was concentrated *in vacuo* and the residue was triturated with pentane (2 x 1 mL) to give the title compound (60 mg) as a white solid.
MS (ES/+): m/z=437 [MH-HCl]⁺.

### Example 80

### N-[1-(3,5-Dichlorophenyl)ethyl]-2-[4-(3,4-difluorophenyl)-4-piperidinyl]-N-methylacetamide (enantiomer 1)

TFA (1.5 mL) was added to a solution of intermediate **96** (105 mg) in DCM (6 mL) previously cooled to 0°C under a Nitrogen atmosphere. The resulting solution was stirred under these conditions for 2 hours, then a 10% sodium hydroxide solution was added until pH=12. The aqueous layer was extracted with DCM (2 x 8 mL) which was passed through a phase separation cartridge with polypropylene frit and concentrated *in vacuo* to give the title compound (84 mg) as a white foam.
T.I.c.: DCM/MeOH 8:2 containing 0.5% of conc. NH4OH, Rf=0.34.
MS (ES/+): m/z=441 [M+H]⁺.

### Example 81

### N-[1-(3,5-Dichlorophenyl)ethyl]-2-[4-(3,4-difluorophenyl)-1-methyl-4-piperidinyl]-N-methylacetamide (enantiomer 1)

Sodium triacethoxyborohydride (126 mg) was added to a solution of Example **80** (84 mg) in anhydrous CH₃CN (5 mL) with a solution of formaldehyde in water (37% w/w, 60 µL) under a Nitrogen atmosphere. The mixture was stirred at r.t. for 2 hours, then the solvent was removed *in vacuo* and the residue was dissolved in 5% sodium hydrogen carbonate solution (5 mL) and extracted with DCM (2 x 8 mL), which was passed through a phase separation cartridge with polypropylene frit and concentrated *in vacuo.* The residue was purified by flash chromatography (DCM, DCM/MeOH from 95:5 to 1:1) to give the title compound (50 mg) as a white solid.
T.I.c.: DCM/MeOH 8:2 containing 0.5% of conc. NH₄OH, Rf=0.72 (detection with ninhydrine).
NMR (d₆-DMSO): δ (ppm) 7.44 (s,1H); 7.37 (m, 1 H); 7.27 (q, 1H); 7.16 (m, 1H); 6.98 (s, 2H); 5.56 (q, 1H); 2.60 (s, 3H); 2.7-2.0 (m, 8H); 2.2 (s, 3H); 2.1 (s, 2H); 1.2 (d, 3H).
MS (ES/+): m/z=455 [M+H]⁺.

### Example 82

### N-[1-(3,5-Dichlorophenyl)ethyl]-2-[4-(3,4-difluorophenyl)-1-methyl-4-piperidinyl]-N-methylacetamide hydrochloride (enantiomer 1)

Example **81** (45 mg) was dissolved in anhydrous Et2O (1.2 mL) and treated with hydrochloric acid (1M in Et2O -110 µL) at 0°C under a Nitrogen atmosphere. The mixture was stirred at 0°C for 30 minutes, then it was concentrated *in vacuo* and the residue was triturated with pentane (2 x 1 mL) to give the title compound (43 mg) as a white solid.
MS (ES/+): m/z=455 [MH-HCl]⁺.

### Example 83

### N-[1-(3,5-Dibromophenyl)ethyl]-2-[4-(3,4-difluorophenyl)-4-piperidinyl]-N-methylacetamide (enantiomer 1)

TFA (1 mL) was added to a solution of intermediate **97** (197 mg) in anhydrous DCM (4 mL) previously cooled at 0°C, under a Nitrogen atmosphere. The solution was stirred at 0°C for 2 hours, then it was treated at 0°C with a 10% sodium hydroxide solution until pH=10. The aqueous layer was extracted with DCM (3 x 8 mL). The combined organic extracts were dried, concentrated *in vacuo* to give the title compound (183 mg) as a colourless oil.
T.I.c.: DCM/MeOH 8:2, containing 1% of conc. NH₄OH, Rf=0.09 (detection with ninhydrine).
MS (ES/+): m/z=531 [M+H]⁺.

### Example 84

### N-[1-(3,5-Dibromophenyl)ethyl]-2-[4-(3,4-difluorophenyl)-1-methyl-4-piperidinyl]-N-methylacetamide (enantiomer 1)

A solution of formaldehyde in water (37% w/w; 51 µL) was added to a stirred solution of Example **83** (183 mg) in CH₃CN (4 mL) under a Nitrogen atmosphere at 23°C. After 10 minutes sodium triacetoxyborohydride (109 mg) was added. The mixture was stirred for further 2 hours then it was quenched with a 5% sodium hydrogen carbonate solution (8 mL). Solvent was evaporated under *vacuo* and the aqueous phase was extracted with DCM (3 x 10 mL). The combined organic phases were dried and concentrated *in vacuo,* and the residue was purified by flash chromatography (DCM/MeOH 8:2) to give the title compound (153 mg) as a white solid.
T.I.c.: DCM/MeOH 8:2, Rf=0.44 (detection with ninhydrine).
MS (ES/+): m/z = 545 [M+H]⁺.

### Example 85

### N-[1-(3,5-Dibromophenyl)ethyl]-2-[4-(3,4-difluorophenyl)-1-methyl-4-piperidinyl]-N-methylacetamide hydrochloride (enantiomer 1)

The compound of Example **84** (153 mg) was dissolved in dry Et2O (4 mL), cooled to 0°C and treated with hydrochloric acid (1M in Et2O - 310 µL). The mixture was stirred at 0°C for 1.5 hours, then Et2O was evaporated under Nitrogen flux and the residue was triturated in pentane (3 x 4 mL) and filtered to give the title compound (153 mg) as a white solid. NMR (d₆-DMSO): δ (ppm) 9.94/9.79 (2bs, 1H); 7.69 (s, 1H); 7.53-7.25 (m, 3H); 7.19 (2s, 2H); 5,56 (q, 1H); 3.4-1.8 (m, 10H); 2.60 (s, 3H); 2.31 (s, 3H); 1.18 (d, 3H).
MS (ES/+): m/z=545 [MH-HCl]⁺.

### Example 86

### N-[1-(3,5-Dibromophenyl)ethyl]-2-[4-(3,4-difluorophenyl)-4-piperidinyl]-N-methylacetamide (enantiomer 2)

TFA (1 mL) was added to a solution of intermediate **98** (188 mg) in anhydrous DCM (4 mL) previously cooled at 0°C, under a Nitrogen atmosphere. The solution was stirred at 0°C for 2 hours, then it was treated at 0°C with a 10% sodium hydroxide solution until pH=10. The aqueous layer was extracted with DCM (3 x 8 mL). The combined organic extracts were dried, concentrated *in vacuo* to give the title compound (172 mg) as a colourless oil.
T.I.c.: DCM/MeOH 8:2, containing 1% of conc. NH₄OH, Rf=0.09 (detection with ninhydrine).
MS (ES/+): m/z=531 [M+H]⁺.

### Example 87

### N-[1-(3,5-Dibromophenyl)ethyl]-2-[4-(3,4-difluorophenyl)-1-methyl-4-piperidinyl]-N-methylacetamide (enantiomer 2)

A solution of formaldehyde in water (37% w/w; 51 µL) was added to a stirred solution of Example **86** (172 mg) in CH₃CN (4 mL) under a Nitrogen atmosphere at 23°C. After 10 minutes sodium triacetoxyborohydride (103 mg) was added. The mixture was stirred for further 2 hours then it was quenched with a 5% sodium hydrogen carbonate solution (8 mL). Solvent was evaporated under *vacuo* and the aqueous phase was extracted with DCM (3 x 10 mL). The combined organic phases were dried and concentrated *in vacuo,* and the residue was purified by flash chromatography (DCM/MeOH 8:2) to give the title compound (142 mg) as a white solid.
T.I.c.: DCM/MeOH 8:2, Rf=0.44 (detection with ninhydrine).
MS (ES/+): m/z = 545 [M+H]⁺.

### Example 88

### N-[1-(3,5-Dibromophenyl)ethyl]-2-[4-(3,4-difluorophenyl)-1-methyl-4-piperidinyl]-N-methylacetamide hydrochloride (enantiomer 2)

The compound of Example **87** (142 mg) was dissolved in dry Et2O (4 mL), cooled to 0°C and treated with hydrochloric acid (1M in Et2O - 290 µL). The mixture was stirred at 0°C for 1.5 hours, then Et2O was evaporated under Nitrogen flux and the residue was triturated in pentane (3 x 4 mL) and filtered to give the title compound (142 mg) as a white solid.
NMR (d₆-DMSO): δ (ppm) 9.84/9.69 (2bs, 1H); 7.69 (s, 1H); 7.53-7.25 (m, 3H); 7.19 (2s, 2H); 5,56 (q, 1H); 3.35-1.85 (m, 10H); 2.61 (s, 3H); 2.31 (s, 3H); 1.18 (d, 3H).
MS (ES/+): m/z=545 [MH-HCl]⁺.

### Example 89

### N-[1-(3,5-Dibromophenyl)ethyl]-2-[4-(3-fluorophenyl)-4-piperidinyl]-N-methylacetamide (enantiomer 1)

TFA (1 mL) was added to a solution of intermediate **99** (217 mg) in anhydrous DCM (4 mL) previously cooled at 0°C, under a Nitrogen atmosphere. The solution was stirred at 0°C for 2 hours, then it was treated at 0°C with a 10% sodium hydroxide solution until pH=10. The aqueous layer was extracted with DCM (3 x 8 mL). The combined organic extracts were dried, concentrated *in vacuo* to give the title compound (200 mg) as a colourless oil.
T.I.c.: DCM/MeOH 8:2, containing 1% of conc. NH₄OH, Rf=0.08 (detection with ninhydrine).
MS (ES/+): m/z=513 [M+H]⁺.

### Example 90

### N-[1-(3,5-Dibromophenyl)ethyl]-2-[4-(3-fluorophenyl)-1-methyl-4-piperidinyl]-N-methylacetamide (enantiomer 1)

A solution of formaldehyde in water (37% w/w; 60 µL) was added to a stirred solution of Example **89** (200 mg) in CH₃CN (4 mL) under a Nitrogen atmosphere at 23°C. After 10 minutes sodium triacetoxyborohydride (124 mg) was added. The mixture was stirred for further 2 hours then it was quenched with a 5% sodium hydrogen carbonate solution (8 mL). Solvent was evaporated under *vacuo* and the aqueous phase was extracted with DCM (3 x 10 mL). The combined organic phases were dried and concentrated *in vacuo,* and the residue was purified by flash chromatography (DCM/MeOH 8:2) to give the title compound (152 mg) as a white solid.
T.I.c.: DCM/MeOH 8:2, Rf=0.46 (detection with ninhydrine).
MS (ES/+): m/z = 527 [M+H]⁺.

### Example 91

### N-[1-(3,5-Dibromophenyl)ethyl]-2-[4-(3-fluorophenyl)-1-methyl-4-piperidinyl]-N-methylacetamide hydrochloride (enantiomer 1)

The compound of Example **90** (152 mg) was dissolved in dry Et2O (4 mL), cooled to 0°C and treated with hydrochloric acid (1M in Et2O - 310 µL). The mixture was stirred at 0°C for 1.5 hours, then Et2O was evaporated under Nitrogen flux and the residue was triturated in pentane (3 x 4 mL) and filtered to give the title compound (146 mg) as a white solid.
NMR (d₆-DMSO): δ (ppm) 9.79/9.69 (2bs, 1H); 7.69 (s, 1H); 7.53-7.06 (m, 4H); 7.19 (bs, 2H); 5,56 (q, 1H); 3.5-1.8 (m, 10H); 2.62 (s, 3H); 2.29 (s, 3H); 1.15 (d, 3H).
MS (ES/+): m/z=527 [MH-HCl]⁺.

### Example 92

### N-[1-(3,5-Dibromophenyl)ethyl]-2-[4-(4-fluoro-3-methylphenyl)-4-piperidinyl]-N-methylacetamide (enantiomer 1)

TFA (0.8 mL) was added to a solution of intermediate **102** (160 mg) in anhydrous DCM (4 mL) previously cooled at 0°C, under a Nitrogen atmosphere. The solution was stirred at 0°C for 2 hours, then it was treated at 0°C with a 10% sodium hydroxide solution until pH=10. The aqueous layer was extracted with DCM (3 x 8 mL). The combined organic extracts were dried, concentrated *in vacuo* to give the title compound (139 mg) as a colourless oil.
T.I.c.: DCM/MeOH 8:2, containing 1% of conc. NH₄OH, Rf=0.06 (detection with ninhydrine).
MS (ES/+): m/z=527 [M+H]⁺.

### Example 93

### N-[1-(3,5-Dibromophenyl)ethyl]-2-[4-(4-fluoro-3-methylphenyl)-1-methyl-4-piperidinyl]-N-methylacetamide (enantiomer 1)

A solution of formaldehyde in water (37% w/w; 40 µL) was added to a stirred solution of Example **92** (139 mg) in CH₃CN (4 mL) under a Nitrogen atmosphere at 23°C. After 10 minutes sodium triacetoxyborohydride (84 mg) was added. The mixture was stirred for further 2 hours then it was quenched with a 5% sodium hydrogen carbonate solution (8 mL). Solvent was evaporated under *vacuo* and the aqueous phase was extracted with DCM (3 x 10 mL). The combined organic phases were dried and concentrated *in vacuo,* and the residue was purified by flash chromatography (DCM/MeOH 8:2) to give the title compound (125 mg) as a white solid.
T.I.c.: DCM/MeOH 8:2, Rf=0.38 (detection with ninhydrine).
MS (ES/+): m/z = 541 [M+H]⁺.

### Example 94

### N-[1-(3,5-Dibromophenyl)ethyl]-2-[4-(4-fluoro-3-methylphenyl)-1-methyl-4-piperidinyl]-N-methylacetamide hydrochloride (enantiomer 1)

The compound of Example **93** (125 mg) was dissolved in dry Et2O (4 mL), cooled to 0°C and treated with hydrochloric acid (1 M in Et2O - 260 µL). The mixture was stirred at 0°C for 1.5 hours, then Et2O was evaporated under Nitrogen flux and the residue was triturated in pentane (3 x 4 mL) and filtered to give the title compound (112 mg) as a white solid.
NMR (d₆-DMSO): 8 (ppm) 9.80/9.71 (2bs, 1H); 7.69 (s, 1H); 7.4-7.0 (m, 3H); 7.20 (2s, 2H); 5,57 (q, 1H); 3.4-1.7 (m, 10H); 2.61 (s, 3H); 2.19 (s, 6H); 1.15 (d, 3H).
MS (ES/+): m/z=541 [MH-HCl]⁺.

### Example 95

### 2-[4-(3-Chlorophenyl)-4-piperidinyl]-N-[1-(3,5-dibromophenyl)ethyl]-N-methylacetamide (enantiomer 1)

TFA (0.7 mL) was added to a solution of intermediate **105** (147 mg) in anhydrous DCM (4 mL) previously cooled at 0°C, under a Nitrogen atmosphere. The solution was stirred at 0°C for 2 hours, then it was treated at 0°C with a 10% sodium hydroxide solution until pH=10. The aqueous layer was extracted with DCM (3 x 8 mL). The combined organic extracts were dried, concentrated *in vacuo* to give the title compound (129 mg) as a colourless oil.
T.I.c.: DCM/MeOH 8:2, containing 1% of conc. NH₄OH, Rf=0.07 (detection with ninhydrine).
MS (ES/+): m/z=529 [M+H]⁺.

### Example 96

### 2-[4-(3-Chlorophenyl)-1-methyl-4-piperidinyl]-N-[1-(3,5-dibromophenyl)ethyl]-N-methylacetamide (enantiomer 1)

A solution of formaldehyde in water (37% w/w: 36 µL) was added to a stirred solution of Example **95** (129 mg) in CH₃CN (4 mL) under a Nitrogen atmosphere at 23°C. After 10 minutes sodium triacetoxyborohydride (78 mg) was added. The mixture was stirred for further 2 hours then it was quenched with a 5% sodium hydrogen carbonate solution (8 mL). Acetonitrile was evaporated under *vacuo* and the aqueous phase was extracted with DCM (3 x 10 mL). The combined organic phases were dried and concentrated *in vacuo,* and the residue was purified by flash chromatography (DCM/MeOH 8:2) to give the title compound (120 mg) as a white solid.
T.I.c.: DCM/MeOH 8:2, Rf=0.50 (detection with ninhydrine).
MS (ES/+): m/z = 543 [M+H]⁺.

### Example 97

### 2-[4-(3-Chlorophenyl)-1-methyl-4-piperidinyl]-N-[1-(3,5-dibromophenyl)ethyl]-N-methylacetamide

The compound of Example **96** (120 mg) was dissolved in dry Et2O (4 mL), cooled to 0°C and treated with hydrochloric acid (1M in Et2O - 250 µL). The mixture was stirred at 0°C for 1.5 hours, then Et2O was evaporated under Nitrogen flux and the residue was triturated in pentane (3 x 4 mL) and filtered to give the title compound (100 mg) as a white solid.
NMR (d₆-DMSO): δ (ppm) 9.64 (bm, 1H); 7.69 (s, 1H); 7.46-7.31 (m, 4H); 7.19 (s, 2H); 5,55 (q, 1H); 3.5-1.7 (m, 10H); 2.64 (s, 3H); 2.29 (s, 3H); 1.15 (d, 3H).
MS (ES/+): m/z=543 [MH-HCl]⁺.

### Example 98

### 2-[4-(3-Chlorophenyl)-4-piperidinyl]-N-[1-(3,5-dichlorophenyl)ethyl]-N-methylacetamide (enantiomer 1)

TFA (2.3 mL) was added to a solution of intermediate **106** (100 mg) in anhydrous DCM (10 mL) previously cooled to 0°C under a Nitrogen atmosphere. The solution was stirred at 0°C for 1 hour, then it was concentrated *in vacuo*. The residue was dissolved in DCM (10 mL) and washed with saturated sodium carbonate solution (10 mL). The organic layer was dried and concentrated in vacuo to give the title compound (90 mg) as a white solid.
T.I.c.: DCM/MeOH 7:3, Rf=0.29 (detection with ninhydrine).
MS (ES/+): m/z=439 [M+H]⁺.

### Example 99

### 2-[4-(3-Chlorophenyl)-1-methyl-4-piperidinyl]-N-[1-(3,5-dichlorophenyl)ethyl]-N-methylacetamide (enantiomer 1)

A solution of formaldehyde in water (37% w/w; 33 µL) was added to a stirred solution of Example **98** (95 mg) in CH₃CN (3.5 mL) under a Nitrogen atmosphere at 23°C. After 10 minutes sodium triacetoxyborohydride (73 mg) was added. The mixture was stirred for further 2 hours then it was quenched with a 5% sodium hydrogen carbonate solution (5 mL) and extracted with AcOEt (3 x 10 mL). The combined organic phases were dried and concentrated *in vacuo,* and the residue was purified by flash chromatography (DCM/MeOH 8:2) to give the title compound (60 mg) as a white solid.
T.I.c.: DCM/MeOH 7:3, Rf=0.48 (detection with ninhydrine).
MS (ES/+): m/z = 453 [M+H]⁺.

### Example 100

### 2-[4-(3-Chlorophenyl)-1-methyl-4-piperidinyl]-N-[1-(3,5-dichlorophenyl)ethyl]-N-methylacetamide hydrochloride (enantiomer 1)

The compound of Example 99 (60 mg) was dissolved in dry Et2O (2.6 mL), cooled to 0°C and treated with hydrochloric acid (1 M in Et2O -146 µL). The mixture was stirred at 0°C for 1.5 hours, then the solvent was removed *in vacuo* and the residue was triturated with pentane (3 x 3 ml) to give the title compound (60.4 mg) as a white solid.
NMR (d₆-DMSO): δ (ppm) 10.0-9.7 (bs, 1H); 7.46 (s, 1H); 7.00 (s, 2H); 7.30 (m, 4H); 5.56 (q, 1H); 3.2-1.8 (m, 16H); 1.17 (d, 3H).
MS (ES/+): m/z= 453 [MH-HCl]⁺.

### Example 101

### 2-[4-(3-Chlorophenyl)-4-piperidinyl]-N-[(3,5-dibromophenyl)methyl]-N-methylacetamide

Intermediate **107** (170 mg) was dissolved in anhydrous DCM (7 mL), cooled at 0°C under a Nitrogen atmosphere and treated with TFA (1.8 mL). The solution was stirred at 0°C for 2 hours, then it was concentrated *in vacuo* at 0°C. The residue was taken up with a saturated potassium carbonate solution (10 mL) and diluted with DCM (50 mL). The organic phase was separated, dried and concentrated *in vacuo* to give the title compound (120 mg) as a white foam.
MS (ES/+): m/z=515 [M+H]⁺.

### Example 102

### 2-[4-(3-Chlorophenyl)-1-methyl-4-piperidinyl]-N-[(3,5-dibromophenyl)methyl]-N-methylacetamide

A solution of formaldehyde in water (37% w/w; 35 µL) was added to a stirred solution of Example **101** (120 mg) in CH₃CN (5 mL) under a Nitrogen atmosphere at 23°C. After 15 minutes sodium triacetoxyborohydride (74 mg) was added. The mixture was stirred for 1 hour then a solution of formaldehyde in water (37% w/w; 35 µL) and sodium triacethoxyborohydride (74 mg) were added and the mixture was stirred at r.t. for additional 2 hours. The solvent was removed *in vacuo* and the residue was dissolved in saturated sodium hydrogen carbonate solution (5 mL) and extracted with DCM (2 x 15mL). The organic layer was dried, concentrated *in vacuo* and the residue was purified by flash chromatography (DCM, DCM/MeOH from 95:5 to 8:2) to give the title compound (60 mg) as a white foam.
T.I.c.: DCM/MeOH 7:3 Rf=0.2 (detection with ninhydrine).
NMR (CDCl₃): δ (ppm) 7.55 (s, 1H); 7.33-7.01 (m, 6H); 4.30 (s, 2H); 2.64 (s, 3H); 2.7-2.1 (m, 8H); 2.36 (s, 2H); 2.25 (s, 3H).
MS (ES/+): m/z=529 [M+H]⁺.

### Example 103

### N-[1-(3,5-Dichlorophenyl)ethyl]-2-[4-(4-fluoro-3-methylphenyl)-4-piperidinyl]-N-methylacetamide (enantiomer 1)

TFA (2.3 mL) was added to a solution of intermediate **108** (103 mg) in anhydrous DCM (10 mL) previously cooled to 0°C under a Nitrogen atmosphere. The solution was stirred at 0°C for 1 hour, then it was concentrated *in vacuo.* The residue was dissolved in DCM (10 mL) and washed with saturated sodium carbonate solution (10 mL). The organic layer was dried and concentrated in vacuo to give the title compound (95 mg) as a white solid.
T.I.c.: DCM/MeOH 7:3, Rf=0.22 (detection with ninhydrine).
MS (ES/+): m/z= 437 [M+H]⁺.

### Example 104

### N-[1-(3,5-Dichlorophenyl)ethyl]-2-[4-(4-fluoro-3-methylphenyl)-1-methyl-4-piperidinyl]-N-methylacetamide (enantiomer 1)

A solution of formaldehyde in water (37% w/w; 30 µL) was added to a stirred solution of Example **103** (90 mg) in CH₃CN (3.5 mL) under a Nitrogen atmosphere at 23°C. After 10 minutes sodium triacetoxyborohydride (70 mg) was added. The mixture was stirred for further 2 hours then it was quenched with a 5% sodium hydrogen carbonate solution (5 mL) and extracted with AcOEt (3 x 10 mL). The combined organic phases were dried and concentrated *in vacuo,* and the residue was purified by flash chromatography (DCM/MeOH 8:2) to give the title compound (57 mg) as a white solid.
T.I.c.: DCM/MeOH 7:3, Rf=0.43 (detection with ninhydrine).
MS (ES/+): m/z = 451 [M+H]⁺.

### Example 105

### N-[1-(3,5-Dichlorophenyl)ethyl]-2-[4-(4-fluoro-3-methylphenyl)-1-methyl-4-piperidinyl]-N-methylacetamide hydrochloride (enantiomer 1)

The compound of Example **104** (57 mg) was dissolved in dry Et2O (2.5 mL), cooled to 0°C and treated with hydrochloric acid (1 M in Et2O - 140 µL). The mixture was stirred at 0°C for 1.5 hours, then the solvent was removed *in vacuo* and the residue was triturated with pentane (3 x 3 ml) to give the title compound (54 mg) as a white solid.
NMR (d₆-DMSO): δ (ppm) 9.9-9.7 (bs, 1H); 7.46 (s, 1H); 7.02 (s, 2H); 7.35-6.85 (m, 3H); 5.57 (q, 1H); 3.0-1.7 (m, 16H); 1.16 (d, 3H).
MS (ES/+): m/z= 451 [MH-HCl]⁺.

### Example 106

### N-[(3,5-Dibromophenyl)methyl]-2-[4-(4-fluoro-3-methylphenyl)-4-piperidinyl]-N-methylacetamide

Intermediate **109** (167 mg) was dissolved in anhydrous DCM (7.5 mL), cooled at 0°C, under a Nitrogen atmosphere and treated with TFA (1.8 mL). The solution was stirred at 0°C for 1 hour, then it was concentrated *in vacuo* at 0°C. The residue was taken up with a saturated potassium carbonate solution (10 mL) and diluited with DCM (50 mL). The organic phase was separated, dried and concentrated *in vacuo* to give the title compound (140 mg) as a white foam.
MS (ES/+): m/z=513 [M+H]⁺.

### Example 107

### N-[(3,5-Dibromophenyl)methyl]-2-[4-(4-fluoro-3-methylphenyl)-1-methyl-4-piperidinyl]-N-methylacetamide

A solution of formaldehyde in water (37% w/w; 56 µL) was added to a stirred solution of Example **106** (128 mg) in CH₃CN (5 mL) under a Nitrogen atmosphere at 23°C. After 15 minutes sodium triacetoxyborohydride (106 mg) was added. The mixture was stirred at r.t. overnight. The solvent was removed *in vacuo* and the residue was dissolved in saturated sodium hydrogen carbonate solution (5 mL) and extracted with DCM (2 x 15mL). The collected organic layers were dried, concentrated *in vacuo* and the residue was purified by flash chromatography (DCM, DCM/MeOH from 95:5 to 8:2) to give the title compound (87 mg) as a pale yellow foam.
T.I.c.: DCM/MeOH 7:3, containing 1% of conc. NH₄OH, Rf=0.45 (detection with ninhydrine).
NMR (CDCl₃): δµ (ppm) 7.53 (s, 1H); 7.14 (s, 2H); 7.1-6.85 (m, 3H); 4.25 (s, 1 H); 3.7 (s, 1H); 2.7-2.1 (m, 19H).
MS (ES/+): m/z=527 [M+H]⁺.

### Example 108

### N-[(3,5-Dibromophenyl)methyl]-2-[4-(3-fluorophenyl)-4-piperidinyl]-N-methylacetamide

TFA (3 mL) was added to a solution of intermediate **110** (270 mg) in DCM (12 mL) previously cooled to 0°C under a Nitrogen atmosphere. The resulting solution was stirred at -20°C overnight, then a 10% sodium hydroxide solution was added until pH=12. The aqueous layer was extracted with DCM (2 x 8 mL) which was passed through a phase separation cartridge with polypropylene frit and concentrated *in vacuo* to give the title compound (207 mg) as a white foam.
MS (ES/+): m/z=499 [M+H]⁺.

### Example 109

### N-[(3,5-Dibromophenyl)methyl]-2-[4-(3-fluorophenyl)-1-methyl-4-piperidinyl]-N-methylacetamide

Sodium triacethoxyborohydride (264 mg) was added to a solution of Example **108** (207 mg) in anhydrous CH₃CN (10 mL) with a solution of formaldehyde in water (37% w/w, 124 µL) under a Nitrogen atmosphere. The mixture was stirred at r.t. for 1 hour, then the solvent was removed *in vacuo* and the residue was dissolved in 5% sodium hydrogen carbonate solution (5 mL) and extracted with DCM (2 x 10 mL), which was passed through a phase separation cartridge with polypropylene frit and concentrated *in vacuo.* The residue was purified by flash chromatography (DCM/MeOH from 9:1 to 8:2 containing 0.5% of conc. NH₄OH) to give the title compound (152 mg) as a white solid.
NMR (CDCl₃): δ (ppm) 7.52-6.85 (m, 7H); 4.26 (s, 2H); 2.60 (s, 3H); 2.65-2.5 (bm, 2H); 2.32 (s, 3H); 2.4-2.05 (m, 8H).
MS (ES/+): m/z=513 [M+H]⁺.

### Example 110

### N-[(3,5-Dibromophenyl)methyl]-2-[4-(3,4-difluorophenyl)-4-piperidinyl]-N-methylacetamide

TFA (3 mL) was added to a solution of intermediate **111** (240 mg) in DCM (12 mL) previously cooled to 0°C under a Nitrogen atmosphere. The resulting solution was stirred at -20°C overnight, then a 10% sodium hydroxide solution was added until pH=12. The aqueous layer was extracted with DCM (2 x 8 mL) which was passed through a phase separation cartridge with polypropylene frit and concentrated *in vacuo* to give the title compound (170 mg) as a white foam.
MS (ES/+): m/z=517 [M+H]⁺.

### Example 111

### N-[(3,5-Dibromophenyl)methyl]-2-[4-(3,4-difluorophenyl)-1-methyl-4-piperidinyl]-N-methylacetamide

Sodium triacethoxyborohydride (210 mg) was added to a solution of Example **110** (170 mg) in anhydrous CH₃CN (8 mL) with a solution of formaldehyde in water (37% w/w, 110 µL) under a Nitrogen atmosphere. The mixture was stirred at r.t. for 1 hour, then the solvent was removed *in vacuo* and the residue was dissolved in 5% sodium hydrogen carbonate solution (5 mL) and extracted with DCM (2 x 10 mL), which was passed through a phase separation cartridge with polypropylene frit and concentrated *in vacuo.* The residue was purified by flash chromatography (DCM/MeOH from 9:1 to 8:2 containing 0.5% of conc. NH₄OH) to give the title compound (132 mg) as a white solid.
NMR (CDCl₃): δ (ppm) 7.52-6.95 (m, 6H); 4.28 (s, 2H); 2.60 (s, 3H); 2.65-2.45 (bm, 2H); 2.21 (s, 3H);2.4-2.05 (m, 8H).
MS (ES/+): m/z=531 [M+H]⁺.

### Example 112

### 2-[4-(4-Cyanophenyl)-4-piperidinyl]-N-[1-(3,5-dibromophenyl)ethyl]-N-methylacetamide (enantiomer 1)

TFA (0.3 mL) was added to a solution of intermediate **116** (17 mg) in DCM (1.2 mL) previously cooled to 0°C under a Nitrogen atmosphere. The resulting solution was stirred under these conditions for 2 hours, then a 10% sodium hydroxide solution was added until pH=12. The aqueous layer was extracted with DCM (2 x 5 mL) which was passed through a phase separation cartridge with polypropylene frit and concentrated *in vacuo* to give the title compound (116 mg) as a white foam.

T.I.c.: DCM/MeOH 8:2 containing 0.5% of conc. NH₄OH, Rf=0.09 (detection with ninhydrine).
MS (ES/+): m/z=520 [M+H]⁺.

### Example 113

### 2-[4-(4-Cyanophenyl)-1-methyl-4-piperidinyl]-N-[1-(3,5-dibromophenyl)ethyl]-N-methylacetamide (enantiomer 1)

Sodium triacethoxyborohydride (17 mg) was added to a solution of Example **112** (13 mg) in anhydrous CH₃CN (5 mL) with a solution of formaldehyde in water (37% w/w, 8 µL) under a Nitrogen atmosphere. The mixture was stirred at r.t. for 2 hours, then the solvent was removed *in vacuo* and the residue was dissolved in 5% sodium hydrogen carbonate solution (5 mL) and extracted with DCM (2 x 5 mL), which was passed through a phase separation cartridge with polypropylene frit and concentrated *in vacuo.* The residue was purified by flash chromatography (DCM, DCM/MeOH from 95:5 to 7:3) to give the title compound (7 mg) as a white solid.
T.I.c.: DCM/MeOH 8:2 containing 0.5% of conc. NH₄OH, Rf=0.6 (detection with ninhydrine).
NMR (CDCl₃): δ (ppm) 7.57 (d, 2H); 7.48 (s, 1H); 7.42 (d, 2H); 7.05 (s, 2H); 5.73 (q, 1H); 2.64 (d,1H );2.61 (s, 3H); 2.59 (d, 1H); 2.65-2.5 (bm, 2H); 2.18 (s, 3H); 2.35-2.05 (bm, 6H); 1.15 (d, 3H).
MS (ES/+): m/z=534 [M+H]⁺.

### Pharmacy examples

### A. Capsules/ Tablets

| | |
|---|---|
| Active ingredient | 25.0mg |
| PVP | 2.5mg |
| Microcrystalline Cellulose | 198.5mg |
| Croscarmellose Sodium | 2.5mg |
| Magnesium Stearate | 1.5mg |

The active ingredient is blended with the other excipients. The blend can be used to fill gelatin capsules or compressed to form tablets using appropriate punches. The tablets can be coated using conventional techniques and coatings.

### B. Tablets

| | |
|---|---|
| Active ingredient | 25.0mg |
| Microcrystalline Cellulose | 264.0mg |
| Croscarmellose Sodium | 10.0mg |
| Magnesium Stearate | 1.0mg |

The active ingredient is blended with microcrystalline cellulose and croscarmellose sodium. Magnesium stearate is then added to the previous blend. The mixture thus obtained can be compressed using appropriate punches and the tablets coated using conventional techniques and coatings.

### C) Infusion

| | |
|---|---|
| Active ingredient | 2-50 mg/ml |
| Buffer solution pH 4.5 suitable for infusion | qs to 100ml |
| (e.g. sodium citrate in NaCl 0.9% or 5% dextrose) | |

The formulation may be packed in glass vials or plastic bag.

The affinity of the compound of the invention for the NK₁ receptor was determined using the NK₁ receptor binding affinity method measuring in vitro by the compounds' ability to displace [3H] - substance P (SP) from recombinant human NK₁ receptors expressed in Chinese Hamster Ovary (CHO) cell membranes. The affinity values are expressed as negative logarithm of the inhibition constant (Ki) of displacer ligands (pKi).

The pKi values obtained as the average of at least two determinations with representative compounds of the invention are within the range of 9.79 to 6.52.

The affinity of the compound of the invention for the serotonin transporter was determined using the hSERT binding affinity method and measuring in vitro the compounds' ability to displace [³H] - Imipramine from recombinant human serotonin transporter expressed in Human Embryonic Kidney HEK293 cell membranes. The affinity values are expressed as negative logarithm of the inhibition constant (Ki) of displacer ligands (pKi).

The pKi values obtained as the average of at least two determinations with representative compounds of the invention are within the range of 9.50 to 6.89.

The inhibitory activity of the compound of the invention at the human serotonin transporter was determined using the hSERT uptake affinity method and measuring the compounds' ability to inhibit uptake of [3H] 5HT in hSERT-LLCPK cells. The data have been digitally processed to obtain the pIC50 values of the antagonists. The pIC50 values obtained as the average of at least two determinations with representative compounds of the invention are within the range of 8.50 to 4.80.

The inhibitory activity of the compound of the invention at the rat serotonin transporter was determined using the rSERT uptake affinity method and measuring the compounds' ability to inhibit uptake of [3H] 5HT in rSERT-LLCPK cells. The data have been digitally processed to obtain the pIC50 values of the antagonists. The pIC50 values obtained as the average of at least two determinations with representative compounds of the invention are within the range of 7.50 to 5.30.

Particularly preferred compounds of the invention have shown a NK1 receptor binding affinity (pki) greater than 8 and an inhibitory activity (pIC50) at the hSERT greater than 7.

## Claims

1. A compound of formula (I) 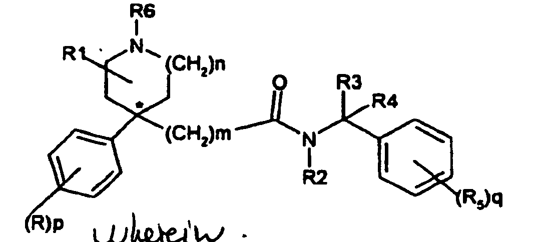 wherein
R represents halogen, C₁₋₄ alkyl, cyano, C₁₋₄ alkoxy, trifluoromethyl or trifluoromethoxy;
R₁ represents hydrogen, halogen, C₃₋₇cycloalkyl, hydroxy, nitro, cyano or C₁₋₄ alkyl optionally substituted by halogen, cyano or C₁₋₄ alkoxy;
R₂ represents hydrogen or C₁₋₄ alkyl;
R₃ and R₄ independently represent hydrogen, cyano, C₁₋₄ alkyl or R₃ together with R₄ represents C₃₋₇ cycloalkyl;
R₅ represents trifluoromethyl, S(O)ₜ C ₁₋₄ alkyl, C₁₋₄ alkyl, C₁₋₄ alkoxy, trifluoromethoxy, halogen or cyano;
R₆ represents hydrogen or (CH₂)rR₇;
R₇ represents hydrogen, C₃₋₇ cycloalkyl, NH(C₁₋₄alkylOC₁₋₄alkoxy), NH(C₁₋₄alkyl), N(C₁₋₄alkyl)₂ , OC(O)NR₉R₈ , NR₈C(O)R₉ or C(O)NR₉R₈;
R₉ and R₈ independently represent hydrogen, C₁₋₄ alkyl or C₃₋₇ cycloalkyl;
m represents 1 ;
n represents 1 or 2;
p is zero or an integer from 1 to 3;
q is an integer from 1 to 3;
r is an integer from 1 to 4;
t is 0 , 1 or 2 ;
and pharmaceutically acceptable salts and solvates thereof.

2. A compound as claimed in claim 1 wherein R is halogen (e.g. fluorine or chlorine), cyano, trifluoromethyl or a C₁₋₄ alkyl (e.g. methyl) group and p is 0 or an integer from 1 to 2.

3. A compound as claimed in claims 1 or 2 wherein R₅ is trifluoromethyl, cyano, methyl or halogen and q is an integer from 1 to 2.

4. A compound as claimed in any claims 1 to 3 wherein R₆ is hydrogen or (CH₂)ᵣR₇ in which r is 1 or 2 and R₇ is hydrogen, cyclopropyl, C(O)N(C ₁₋₄ alkyl)₂ or C(O)NH(C ₁₋₄ alkyl).

5. A compound as claimed in any claims 1 to 4 wherein R is C₁₋₄ alkyl, halogen (i.e chlorine or fluorine), trifluoromethyl or cyano; R₁ is hydrogen, methyl, ethyl or halogen (e.g. fluorine), R₂ is methyl or hydrogen, R₃ and R₄ are independently hydrogen or methyl, R₅ is trifluoromethyl, cyano, methyl, chlorine, bromine or fluorine, R₆ is hydrogen, methyl, ethyl, methylcyclopropyl or CH₂C(O)N(CH₃)₂, p is 0 or an integer from 1 to 2, m is 1, n is 1 or 2, q is 1 or 2.

6. A compound according to claim 1 selected from:
N-(3,5-Dichlorobenzyl)-2-[4-(4-fluorophenyl)-piperidin-4-yl]-N-methyl-acetamide;
N-(3,5-Dichlorobenzyl)-2-[3-fluoro-4-(4-fluorophenyl)-piperidin-4-yl]-N-methyl-acetamide ;
N-(3,5-Bis-trifluoromethyl)-benzyl-2-[(4-fluoro-2-methyl-phenyl)-piperidin-4-yl]-N-methylacetamide;
N-(3,5-Dichlorobenzyl)-2-[4-(4-fluoro-2-methyl-phenyl)-piperidin-4-yl]-N-methyl-acetamide;
N-(3,5-Bis-trifluoromethyl-benzyl)-2-[4-(4-fluorophenyl)-azepin-4-yl]-N-methyl-acetamide;
N-(3,5-Bis-trifluoromethyl-benzyl)-2-[4-(4-fluoro-2-methyl-phenyl)-azepin-4-yl]-N-methylacetamide;
N-(3,5-Dichlorobenzyl)-2-[4-(4-fluoro-2-methyl-phenyl)-azepin-4-yl]-N-methyl-acetamide;
N-(3,5-Bis-trifluoromethyl-benzyl)-2-[3-fluoro-4-(4-fluoro-2-methyl-phenyl)-azepin-4-yl]-N-methyl-acetamide;
N-(3,5-Dichlorobenzyl)-2-[3-fluoro-4-(4-fluoro-2-methyl-phenyl)-azepin-4-yl]-N-methylacetamide;
N-(3,5-Dichlorobenzyl)-2-[3-fluoro-4-(4-fluoro-2-methyl-phenyl)-azepin-4-yl]-N-methylacetamide;
N-(3,5-Bis-trifluoromethyl-benzyl)-2-[3-fluoro-4-(4-fluoro-2-methyl-phenyl)-azepin-4-yl]-N-methyl-acetamide;
N-(3,5-Dibromobenzyl)-2-[4-(4-fluorophenyl)-piperidin-4-yl]-N-methyl-acetamide;
N-(3,5-Dibromo-benzyl)-2-[4-(4-fluoro-phenyl)-1-methyl-piperidin-4-yl]-N-methylacetamide;
N-(3,5-Dibromobenzyl)-2-(4-phenyl-piperidin-4-yl)-N-methyl-acetamide;
N-(3,5-Dibromo-benzyl)-2-(4-phenyl-1-methyl-piperidin-4-yl)-N-methyl-acetamide;
N-[1-(3,5-Dichloro-phenyl)-ethyl]-2-[4-(4-fluoro-phenyl)-piperidin-4-yl]-N-methylacetamide;
N-[1-(3,5-Dichloro-phenyl)-ethyl]-2-[4-(4-fluoro-phenyl)-1-methyl-piperidin-4-yl]-N-methylacetamide;
N-[1-(3,5-Bis-trifluoromethyl-phenyl)-ethyl]-2-[4-(4-fluoro-phenyl)-piperidin4-yl]-N-methylacetamide;
N-[1-(3,5-Bis-trifluoromethyl-phenyl)-ethyl]-2-[4-(4-fluoro-phenyl)-1-methyl-piperidin-4-yl]-N-methyl-acetamide;
N-[1-(3,5-Dibromo-phenyl)-ethyl]-2-[4-(4-fluoro-phenyl)-piperidin-4-yl]-N-methylacetamide;
N-[1-(3,5-Dibromo-phenyl)-ethyl]-2-[4-(4-fluoro-phenyl)-1-methyl-piperidin-4-yl]-N-methylacetamide;
N-[1-(3,5-Bis-trifluoromethyl-phenyl)-ethyl]-2-(4-phenyl-piperidin-4-yl)-N-methyl-acetamide;
N-[1-(3,5- Bis-trifluoromethyl-phenyl)-ethyl]-2-(4-phenyl-1-methyl-piperidin-4-yl)-N-methylacetamide;
N-[1-(3,5-Dibromo-phenyl)-ethyl]-2-(4-phenyl-piperidin-4-yl)-N-methyl-acetamide;
N-[1-(3,5-Dibromo-phenyl)-ethyl]-2-(4-phenyl-1-methyl-piperidin-4-yl)-N-methylacetamide;
N-[1-(3,5-Dibromo-phenyl)-ethyl]-2-[4-(4-fluoro-phenyl)-piperidin-4-yl]-N-methylacetamide;
N-[1-(3,5-Dibromo-phenyl)-ethyl]-2-[4-(4-fluoro-phenyl)-1-methyl-piperidin-4-yl]-N-methylacetamide;
*N*-{1-[3,5-Bis(trifluoromethyl)phenyl]ethyl}-2-[4-(4-fluoro-2-methylphenyl)-4-piperidinyl]-*N-*methylacetamide;
*N*-[(3,5-Dibromophenyl)methyl]-2-[4-(4-fluoro-2-methylphenyl)-4-piperidinyl]-*N-*methylacetamide;
*N*-{[3,5-Bis(trifluoromethyl)phenyl]methyl}-2-[4-(4-fluoro-2-methylphenyl)-1-methyl-4-piperidinyl]-*N*-methylacetamide;
*N*-[(3,5-Dichlorophenyl)methyl]-2-[4-(4-fluoro-2-methylphenyl)-1-methyl-4-piperidinyl]-*N-*methylacetamide;
*N*-{[3,5-Bis(trifluoromethyl)phenyl]methyl}-2-[4-(4-fluoro-2-methylphenyl)-4-piperidinyl]acetamide;
*N*-{[3,5-Bis(trifluoromethyl)phenyl]methyl}-2-[4-(4-fluoro-2-methylphenyl)-1-methyl-4-piperidinyl]acetamide;
*N*-[(3,5-Dibromophenyl)methyl]-2-[4-(4-fluoro-2-methylphenyl)-1-methyl-4-piperidinyl]-*N-*methylacetamide;
*N*-[(3,5-Dibromophenyl)methyl]-*N*-methyl-2-[4-(2-methylphenyl)-4-piperidinyl]acetamide;
*N*-[(3,5-Dibromophenyl)methyl]-*N*-methyl-2-[1-methyl-4-(2-methylphenyl)-4-piperidinyl]acetamide;
*N*-[(3,5-Dichlorophenyl)methyl]-2-[4-(4-fluorophenyl)-1-methyl-4-piperidinyl]-*N-*methylacetamide;
*N*-{[3,5-Bis(trifluoromethyl)phenyl]methyl}-2-[4-(4-fluorophenyl)-1-methyl-4-piperidinyl]-*N-*methylacetamide;
*N*-[1-(3,5-Dibromophenyl)-1-methylethyl]-2-[4-(4-fluorophenyl)-4-piperidinyl]-*N-*methylacetamide;
*N*-[1-(3,5-Dibromophenyl)-1-methylethyl]-2-[4-(4-fluorophenyl)-1-methyl-4-piperidinyl]-*N-*methylacetamide;
N-[1-(3,5-Bis-trifluoromethyl-phenyl)-ethyl]-2-[4-(4-fluoro-phenyl)-piperidin-4-yl]-N-methylacetamide ;
N-[1-(3,5-Bis-trifluoromethyl-phenyl)-ethyl]-2-[4-(4-fluoro-phenyl)-1-methyl-pipeidin-4-yl]-N-methyl-acetamide;
2-[1-(Cyclopropylmethyl)-4-(4-fluorophenyl)-4-piperidinyl]-*N*-[(3,5-dibromophenyl)methyl]-*N*-methylacetamide;
2-[4-{2-[[(3,5-Dibromophenyl)methyl](methyl)amino]-2-oxoethyl}-4-(4-fluorophenyl)-1-piperidinyl]-*N,N*-dimethylacetamide;
*N*-[(3,5-Dibromophenyl)methyl]-2-[1-ethyl-4-(4-fluorophenyl)-4-piperidinyl]-*N-*methylacetamide;
*N*-{1-[3,5-Bis(trifluoromethyl)phenyl]ethyl}-2-[4-(4-fluorophenyl)hexahydro-1*H*-azepin-4-yl]-*N*-methylacetamide;
*N*-{1-[3,5-Bis(trifluoromethyl)phenyl]ethyl}-2-[4-(4-fluorophenyl)-1-methylhexahydro-1*H-*azepin-4-yl]-*N*-methylacetamide;
*N*-[(3,5-Dibromophenyl)methyl]-2-[4-(4-fluorophenyl)hexahydro-1*H*-azepin-4-yl]-*N-*methylacetamide;
*N*-[(3,5-Dibromophenyl)methyl]-2-[4-(4-fluorophenyl)-1-methylhexahydro-1*H*-azepin-4-yl]-*N*-methylacetamide;
*N*-[(3-Bromo-5-cyanophenyl)methyl]-2-[4-(4-fluorophenyl)-4-piperidinyl]-*N-*methylacetamide;
*N*-[(3-Bromo-5-cyanophenyl)methyl]-2-[4-(4-fluorophenyl)-1-methyl-4-piperidinyl]-*N-*methylacetamide;
*N*-[(3,5-Dibromophenyl)methyl]-*N*-methyl-2-{4-[3-(trifluoromethyl)phenyl]-4-piperidinyl}acetamide;
*N*-[(3,5-Dibromophenyl)methyl]-*N*-methyl-2-{1-methyl-4-[3-(trifluoromethyl)phenyl]-4-piperidinyl}acetamide;
*N*-[(3,5-Dibromophenyl)methyl]-2-[4-(3,4-dimethylphenyl)-4-piperidinyl]-*N-*methylacetamide;
*N*-[1-(3,5-Dibromophenyl)ethyl]-2-[4-(3-fluorophenyl)-1-methyl-4-piperidinyl]-*N-*methylacetamide;
*N*-[1-(3,5-Dibromophenyl)ethyl]-2-[4-(4-fluoro-3-methylphenyl)-4-piperidinyl]-*N-*methylacetamide;
*N*-[1-(3,5-Dibromophenyl)ethyl]-2-[4-(4-fluoro-3-methylphenyl)-1-methy-4-piperidinyl]-*N-*methylacetamide;
2-[4-(3-Chlorophenyl)-4-piperidinyl]-*N*-[1-(3,5-dibromophenyl)ethyl]-*N*-methylacetamide;
*N*-[1-(3,5-Dibromophenyl)ethyl]-2-[4-(3,4-difluorophenyl)-4-piperidinyl]-*N-*methylacetamide;
*N*-[1-(3,5-Dibromophenyl)ethyl]-2-[4-(3,4-difluorophenyl)-1-methyl-4-piperidinyl]-*N-*methylacetamide;
*N*-[1-(3,5-Dibromophenyl)ethyl]-2-[4-(3-fluorophenyl)-4-piperidinyl]-*N*-methylacetamide ;
*N*-[1-(3,5-Dibromophenyl)ethyl]-2-[4-(3-fluorophenyl)-1-methyl-4-piperidinyl]-*N-*methylacetamide;
*N*-[1-(3,5-Dibromophenyl)ethyl]-2-[4-(4-fluoro-3-methylphenyl)-4-piperidinyl]-*N-*methylacetamide;
*N*-[1-(3,5-Dibromophenyl)ethyl]-2-[4-(4-fluoro-3-methylphenyl)-1-methyl-4-piperidinyl]-*N-*methylacetamide;
2-[4-(3-Chlorophenyl)-4-piperidinyl]-*N*-[1-(3,5-dibromophenyl)ethyl]-*N*-methylacetamide;
2-[4-(3-Chlorophenyl)-1-methyl-4-piperidinyl]-*N*-[1-(3,5-dibromophenyl)ethyl]-*N-*methylacetamide;
2-[4-(3-Chlorophenyl)-4-piperidinyl]-*N*-[1-(3,5-dichlorophenyl)ethyl]-*N*-methylacetamide;
2-[4-(3-Chlorophenyl)-1-methyl-4-pipeidinyl]-*N*-[1-(3,5-dichlorophenyl)ethyl]-*N-*methylacetamide;
2-[4-(3-Chlorophenyl)-4-piperidinyl]-*N*-[(3,5-dibromophenyl)methyl]-*N*-methylacetamide;.
*N*-[1-(3,5-Dichlorophenyl)ethyl]-2-[4-(4-fluoro-3-methylphenyl)-4-piperidinyl]-*N*-m ethylacetamide;
*N*-[(3,5-Dibromophenyl)methyl]-2-[4-(4-fluoro-3-methylphenyl)-4-piperidinyl]-*N-*methylacetamide;
*N*-[(3,5-Dibromophenyl)methyl]-2-[4-(4-fluoro-3-methylphenyl)-1-methyl-4-piperidinyl]-*N-*methylacetamide ;
*N*-[(3,5-Dibromophenyl)methyl]-2-[4-(3-fluorophenyl)-4-piperidinyl]-*N*-methylacetamide;
*N*-[(3,5-Dibromophenyl)methyl]-2-[4-(3-fluorophenyl)-1-methyl-4-piperidinyl]-*N-*methylacetamide;
*N*-[(3,5-Dibromophenyl)methyl]-2-[4-(3,4-difluorophenyl)-4-piperidinyl]-*N-*methylacetamide;
*N*-[(3,5-Dibromophenyl)methyl]-2-[4-(3,4-difluorophenyl)-1-methyl-4-piperidinyl]-*N-*methylacetamide;
2-[4-(4-Cyanophenyl)-4-piperidinyl]-*N*-[1-(3,5-dibromophenyl)ethyl]-*N*-methylacetamide; diastereoisomers or enantiomers thereof and pharmaceutically acceptable salts (e.g. hydrochloride) thereof .

7. A compound according to claim 1 selected from
[N-(3,5-Dibromo-benzyl)-2-[4-(4-fluoro-phenyl)-1-methyl-piperidin-4-yl]-N-methylacetamide;
N-[1-(*S*)-1-(3,5-Bis-trifluoromethyl-phenyl)-ethyl]-2-[4-(4-fluoro-phenyl)-1-methyl-piperidin-4-yl]-N-methyl-acetamide;
N-[1-(3,5-Dibromo-phenyl)-ethyl]-2-[4-(4-fluoro-phenyl)-1-methyl-piperidin-4-yl]-N-methylacetamide (enantiomer 1);
N-[1-(3,5-Dibromo-phenyl)-ethyl]-2-(1-methyl-4-phenyl-piperidin-4-yl)-N-methyl-acetamide (enantiomer 1);
N-[1-(3,5-Dichloro-phenyl)-ethyl]-2-[4-(4-fluoro-phenyl)-1-methyl-piperidin-4-yl]-N-methylacetamide (enantiomer 1);
and pharmaceutically acceptable salts thereof (e.g. hydrochloride).

8. A process (A) for the preparation of a compound as claimed in claim 1 which comprises reacting an activated derivative of the carboxylic acid of formula (II) wherein R₆ is a nitrogen protecting group or (CH2)rR₇ , with amine (III) 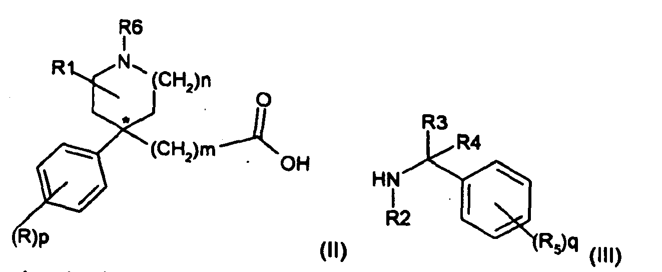 wherein R₂ is hydrogen, C₁₋₄ alkyl or a nitrogen protecting group, followed where necessary by removal of any nitrogen protecting group; or a process B for the preparation of a compound of formula(I) wherein R₂ is C ₁₋₄ alkyl which comprises the reaction of a compound of formula(la), with (C ₁₋₄ alkyl)L wherein L is a suitable leaving group selected from iodine, bromine.

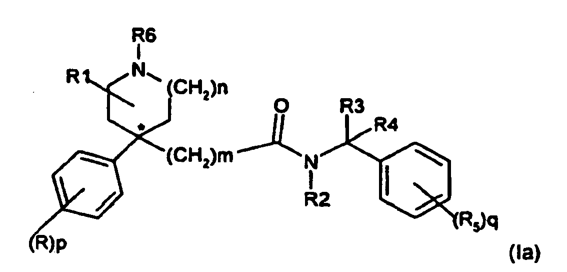

9. A compound as claimed in any claims 1 to 7 for use in therapy.

10. The use of a compound as claimed in any claims 1 to 7 in the preparation of a medicament for use in the treatment of conditions mediated by tachykinins (including substance P and other neurokinins) and/or by selective inhibition of the serotonin reuptake transporter protein.

11. A pharmaceutical composition comprising a compound as claimed in any claims 1 to 7 in admixture with one or more pharmaceutically acceptable carriers or excipients.

## Patentansprüche

1. Verbindung der Formel (I) 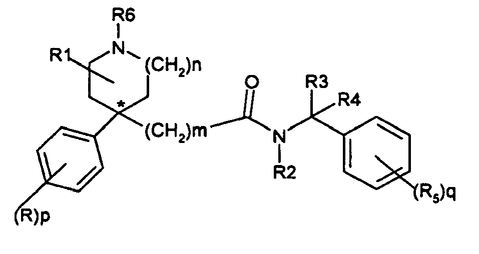 wobei
R Halogen, C₁₋₄-Alkyl, Cyano, C₁₋₄-Alkoxy, Trifluormethyl oder Trifluormethoxy darstellt;
R₁ Wasserstoff, Halogen, C₃₋₇-Cycloalkyl, Hydroxy, Nitro, Cyano oder C₁₋₄-Alkyl, gegebenenfalls substituiert mit Halogen, Cyano oder C₁₋₄-Alkoxy, darstellt;
R₂ Wasserstoff oder C₁₋₄-Alkyl darstellt;
R₃ und R₄ unabhängig Wasserstoff, Cyano, C₁₋₄-Alkyl dar oder R₃ zusammen mit R₄ stellt C₃₋₇-Cycloalkyl darstellen;
R₅ Trifluormethyl, S(O)ₜC₁₋₄-Alkyl, C₁₋₄-Alkyl, C₁₋₄-Alkoxy, Trifluormethoxy, Halogen oder Cyano darstellt;
R₆ Wasserstoff oder (CH₂)ᵣR₇ darstellt;
R₇ Wasserstoff, C₃₋₇-Cycloalkyl, NH(C₁₋₄-AlkylOC₁₋₄-alkoxy), NH(C₁₋₄-Alkyl), N(C₁₋₄-Alkyl)₂, OC(O)NR₉R₈, NR₈C(O)R₉ oder C(O)NR₉R₈ darstellt;
R₉ und R₈ unabhängig Wasserstoff, C₁₋₄-Alkyl oder C₃₋₇-Cycloalkyl darstellen;
m 1 darstellt;
n 1 oder 2 darstellt;
p null ist oder eine ganze Zahl von 1 bis 3;
q eine ganze Zahl von 1 bis 3 ist;
r eine ganze Zahl von 1 bis 4 ist;
t 0, 1 oder 2 ist;
und pharmazeutisch verträgliche Salze und Solvate davon.

2. Verbindung wie in Anspruch 1 beansprucht, wobei R Halogen (z.B. Fluor oder Chlor), Cyano, Trifluormethyl oder ein C₁₋₄-Alkylrest (z.B. eine Methylgruppe) ist und p 0 oder eine ganze Zahl von 1 bis 2 ist.

3. Verbindung wie in den Ansprüchen 1 oder 2 beansprucht, wobei R₅ Trifluormethyl, Cyano, Methyl oder Halogen ist und q eine ganze Zahl von 1 bis 2 ist.

4. Verbindung wie in einem der Ansprüche 1 bis 3 beansprucht, wobei R₆ Wasserstoff oder (CH₂)ᵣR₇ ist, wobei r 1 oder 2 ist und R₇ Wasserstoff, Cyclopropyl, C(O)N(C₁₋₄-Alkyl)₂ oder C(O)NH(C₁₋₄-Alkyl) ist.

5. Verbindung wie in einem der Ansprüche 1 bis 4 beansprucht, wobei R C₁₋₄-Alkyl, Halogen (d.h. Chlor oder Fluor), Trifluormethyl oder Cyano ist; R₁ Wasserstoff, Methyl, Ethyl oder Halogen (z.B. Fluor) ist, R₂ ein Methyl oder Wasserstoff ist, R₃ und R₄ unabhängig Wasserstoff oder Methyl sind, R₅ Trifluormethyl, Cyano, Methyl, Chlor, Brom oder Fluor ist, R₆ Wasserstoff, Methyl, Ethyl, Methylcyclopropyl oder CH₂C(O)N(CH₃)₂ ist, p 0 oder eine ganze Zahl von 1 bis 2 ist, m 1 ist, n 1 oder 2 ist, q 1 oder 2 ist.

6. Verbindung gemäß Anspruch 1, ausgewählt aus:
N-(3,5-Dichlorbenzyl)-2-[4-(4-fluorphenyl)-piperidin-4-yl]-N-methylacetamid;
N-(3,5-Dichlorbenzyl)-2-[3-fluor-4-(4-fluorphenyl)-piperidin-4-yl]-N-methylacetamid;
N-(3,5-Bis-trifluormethyl)-benzyl-2-[(4-fluor-2-methylphenyl)-piperidin-4-yl]-N-methylacetamid;
N-(3,5-Dichlorbenzyl)-2-[4-(4-fluor-2-methylphenyl)-piperidin-4-yl]-N-methylacetamid;
N-(3,5-Bis-trifluormethylbenzyl)-2-[4-(4-fluorphenyl)-azepin-4-yl]-N-methylacetamid;
N-(3,5-Bis-trifluormethylbenzyl)-2-[4-(4-fluor-2-methylphenyl)-azepin-4-yl]-N-methyl-acetamid;
N-(3,5-Dichlorbenzyl)-2-[4-(4-fluor-2-methylphenyl)-azepin-4-yl]-N-methylacetamid;
N-(3,5-Bis-trifluormethylbenzyl)-2-[3-fluor-4-(4-fluor-2-methylphenyl)-azepin-4-yl]-N-methylacetamid;
N-(3,5-Dichlorbenzyl)-2-[3-fluor-4-(4-fluor-2-methylphenyl)-azepin-4-yl]-N-methyl-acetamid;
N-(3,5-Dichlorbenzyl)-2-[3-fluor-4-(4-fluor-2-methylphenyl)-azepin-4-yl]-N-methyl-acetamid;
N-(3,5-Bis-trifluormethylbenzyl)-2-[3-fluor-4-(4-fluor-2-methylphenyl)-azepin-4-yl]-N-methylacetamid;
N-(3,5-Dibrombenzyl)-2-[4-(4-fluorphenyl)-piperidin-4-yl]-N-methylacetamid;
N-(3,5-Dibrombenzyl)-2-[4-(4-fluorphenyl)-1-methylpiperidin-4-yl]-N-methylacetamid;
N-(3,5-Dibrombenzyl)-2-(4-phenylpiperidin-4-yl)-N-methylacetamid;
N-(3,5-Dibrombenzyl)-2-(4-phenyl-1-methylpiperidin-4-yl)-N-methylacetamid;
N-[1-(3,5-Dichlorphenyl)ethyl]-2-[4-(4-fluorphenyl)-piperidin-4-yl]-N-methylacetamid;
N-[1-(3,5-Dichlorphenyl)ethyl]-2-[4-(4-fluorphenyl)-1-methylpiperidin-4-yl]-N-methyl-acetamid;
N-[1-(3,5-Bis-trifluormethylphenyl)ethyl]-2-[4-(4-fluorphenyl)-piperidin-4-yl]-N-methyl-acetamid;
N-[1-(3,5-Bis-trifluormethylphenyl)ethyl]-2-[4-(4-fluorphenyl)-1-methylpiperidin-4-yl]-N-methylacetamid;
N-[1-(3,5-Dibromphenyl)ethyl]-2-[4-(4-fluorphenyl)-piperidin-4-yl]-N-methylacetamid;
N-[1-(3,5-Dibromphenyl)ethyl]-2-[4-(4-fluorphenyl)-1-methylpiperidin-4-yl]-N-methyl-acetamid;
N-[1-(3,5-Bis-trifluormethylphenyl)ethyl]-2-(4-phenylpiperidin-4-yl)-N-methylacetamid;
N-[1-(3,5-Bis-trifluormethylphenyl)ethyl]-2-(4-phenyl-1-methylpiperidin-4-yl)-N-methylacetamid;
N-[1-(3,5-Dibromphenyl)ethyl]-2-(4-phenylpiperidin-4-yl)-N-methylacetamid;
N-[1-(3,5-Dibromphenyl)ethyl]-2-(4-phenyl-1-methylpiperidin-4-yl)-N-methylacetamid;
N-[1-(3,5-Dibromphenyl)ethyl]-2-[4-(4-fluorphenyl)piperidin-4-yl]-N-methylacetamid;
N-[1-(3,5-Dibromphenyl)ethyl]-2-[4-(4-fluorphenyl)-1-methylpiperidin-4-yl]-N-methyl-acetamid;
N-{1-[3,5-Bis(trifluormethyl)phenyl]ethyl}-2-[4-(4-fluor-2-methylphenyl)-4-piperidinyl]-N-methylacetamid;
N-[(3,5-Dibromphenyl)methyl]-2-[4-(4-fluor-2-methylphenyl)-4-piperidinyl]-N-methyl-acetamid;
N-{[3,5-Bis(trifluormethyl)phenyl]methyl}-2-[4-(4-fluor-2-methylphenyl)-1-methyl-4-piperidinyl]-N-methylacetamid;
N-[(3,5-Dichlorphenyl)methyl]-2-[4-(4-fluor-2-methylphenyl)-1-methyl-4-piperidinyl]-N-methylacetamid;
N-{[3,5-Bis(trifluormethyl)phenyl]methyl}-2-[4-(4-fluor-2-methylphenyl)-4-piperidinyl]-acetamid;
N-{[3,5-Bis(trifluormethyl)phenyl]methyl}-2-[4-(4-fluor-2-methylphenyl)-1-methyl-4-piperidinyl] acetamid;
N-[(3,5-Dibromphenyl)methyl]-2-[4-(4-fluor-2-methylphenyl)-1-methyl-4-piperidinyl]-N-methylacetamid;
N-[(3,5-Dibromphenyl)methyl]-N-methyl-2-[4-(2-methylphenyl)-4-piperidinyl]acetamid;
N-[(3,5-Dibromphenyl)methyl]-N-methyl-2-[1-methyl-4-(2-methylphenyl)-4-piperidinyl]acetamid;
N-[(3,5-Dichlorphenyl)methyl]-2-[4-(4-fluorphenyl)-1-methyl-4-piperidinyl]-N-methyl-acetamid;
N-{[3,5-Bis(trifluormethyl)phenyl]methyl}-2-[4-(4-fluorphenyl)-1-methyl-4-piperidinyl]-N-methylacetamid;
N-[1-(3,5-Dibromphenyl)-1-methylethyl]-2-[4-(4-fluorphenyl)-4-piperidinyl]-N-methyl-acetamid;
N-[1-(3,5-Dibromphenyl)-1-methylethyl]-2-[4-(4-fluorphenyl)-1-methyl-4-piperidinyl]-N-methylacetamid;
N-[1-(3,5-Bis-trifluormethylphenyl)ethyl]-2-[4-(4-fluorphenyl)piperidin-4-yl]-N-methyl-acetamid;
N-[1-(3,5-Bis-trifluormethylphenyl)ethyl]-2-[4-(4-fluorphenyl)-1-methylpiperidin-4-yl]-N-methylacetamid;
2-[1-(Cyclopropylmethyl)-4-(4-fluorphenyl)-4-piperidinyl]-N-[(3,5-dibromphenyl)-methyl]-N-methylacetamid;
2-[4-{2-[[(3,5-Dibromphenyl)methyl](methyl)amino]-2-oxoethyl}-4-(4-fluorphenyl)-1-piperidinyl]-N,N-dimethylacetamid;
N-[(3,5-Dibromphenyl)methyl]-2-[1-ethyl-4-(4-fluorphenyl)-4-piperidinyl]-N-methyl-acetamid;
N-{1-[3,5-Bis(trifluormethyl)phenyl]ethyl}-2-[4-(4-fluorphenyl)hexahydro-1H-azepin-4-yl]-N-methylacetamid;
N-{1-[3,5-Bis(trifluormethyl)phenyl]ethyl}-2-[4-(4-fluorphenyl)-1-methylhexahydro-1H-azepin-4-yl]-N-methylacetamid;
N-[(3,5-Dibromphenyl)methyl]-2-[4-(4-fluorphenyl)hexahydro-1H-azepin-4-yl]-N-methylacetamid;
N-[(3,5-Dibromphenyl)methyl]-2-[4-(4-fluorphenyl)-1-methylhexahydro-1H-azepin-4-yl]-N-methylacetamid;
N-[(3-Brom-5-cyanophenyl)methyl]-2-[4-(4-fluorphenyl)-4-piperidinyl]-N-methyl-acetamid;
N-[(3-Brom-5-cyanophenyl)methyl]-2-[4-(4-fluorphenyl)-1-methyl-4-piperidinyl]-N-methylacetamid;
N-[(3,5-Dibromphenyl)methyl]-N-methyl-2-{4-[3-(trifluormethyl)phenyl]-4-piperidinyl} acetamid;
N-[(3,5-Dibromphenyl)methyl]-N-methyl-2-{1-methyl-4-[3-(trifluormethyl)phenyl]-4-piperidinyl}acetamid;
N-[(3,5-Dibromphenyl)methyl]-2-[4-(3,4-dimethylphenyl)-4-piperidinyl]-N-methyl-acetamid;
N-[1-(3,5-Dibromphenyl)ethyl]-2-[4-(3-fluorphenyl)-1-methyl-4-piperidinyl]-N-methyl-acetamid;
N-[1-(3,5-Dibromphenyl)ethyl]-2-[4-(4-fluor-3-methylphenyl)-4-piperidinyl]-N-methyl-acetamid;
N-[1-(3,5-Dibromphenyl)ethyl]-2-[4-(4-fluor-3-methylphenyl)-1-methyl-4-piperidinyl]-N-methylacetamid;
2-[4-(3-Chlorphenyl)-4-piperidinyl]-N-[1-(3,5-dibromphenyl)ethyl]-N-methylacetamid;
N-[1-(3,5-Dibromphenyl)ethyl]-2-[4-(3,4-difluorphenyl)-4-piperidinyl]-N-methyl-acetamid;
N-[1-(3,5-Dibromphenyl)ethyl]-2-[4-(3,4-difluorphenyl)-1-methyl-4-piperidinyl]-N-methylacetamid;
N-[1-(3,5-Dibromphenyl)ethyl]-2-[4-(3-fluorphenyl)-4-piperidinyl]-N-methylacetamid;
N-[1-(3,5-Dibromphenyl)ethyl]-2-[4-(3-fluorphenyl)-1-methyl-4-piperidinyl]-N-methyl-acetamid;
N-[1-(3,5-Dibromphenyl)ethyl]-2-[4-(4-fluor-3-methylphenyl)-4-piperidinyl]-N-methyl-acetamid;
N-[1-(3,5-Dibromphenyl)ethyl]-2-[4-(4-fluor-3-methylphenyl)-1-methyl-4-piperidinyl]-N-methylacetamid;
2-[4-(3-Chlorphenyl)-4-piperidinyl]-N-[1-(3,5-dibromphenyl)ethyl]-N-methylacetamid;
2-[4-(3-Chlorphenyl)-1-methyl-4-piperidinyl]-N-[1-(3,5-dibromphenyl)ethyl]-N-methyl-acetamid;
2-[4-(3-Chlorphenyl)-4-piperidinyl]-N-[1-(3,5-dichlorphenyl)ethyl]-N-methylacetamid;
2-[4-(3-Chlorphenyl)-1-methyl-4-piperidinyl]-N-[1-(3,5-dichlorphenyl)ethyl]-N-methyl-acetamid;
2-[4-(3-Chlorphenyl)-4-piperidinyl]-N-[(3,5-dibromphenyl)methyl]-N-methylacetamid;
N-[1-(3,5-Dichlorphenyl)ethyl]-2-[4-(4-fluor-3-methylphenyl)-4-piperidinyl]-N-methyl-acetamid;
N-[(3,5-Dibromphenyl)methyl]-2-[4-(4-fluor-3-methylphenyl)-4-piperidinyl]-N-methyl-acetamid;
N-[(3,5-Dibromphenyl)methyl]-2-[4-(4-fluor-3-methylphenyl)-1-methyl-4-piperidinyl]-N-methylacetamid;
N-[(3,5-Dibromphenyl)methyl]-2-[4-(3-fluorphenyl)-4-piperidinyl]-N-methylacetamid;
N-[(3,5-Dibromphenyl)methyl]-2-[4-(3-fluorphenyl)-1-methyl-4-piperidinyl]-N-methyl-acetamid;
N-[(3,5-Dibromphenyl)methyl]-2-[4-(3,4-difluorphenyl)-4-piperidinyl]-N-methyl-acetamid;
N-[(3,5-Dibromphenyl)methyl]-2-[4-(3,4-difluorphenyl)-1-methyl-4-piperidinyl]-N-methylacetamid;
2-[4-(4-Cyanophenyl)-4-piperidinyl]-N-[1-(3,5-dibromphenyl)ethyl]-N-methylacetamid;
Diastereoisomeren oder Enantiomeren davon und pharmazeutisch verträglichen Salzen (z.B. Hydrochlorid) davon.

7. Verbindung gemäß Anspruch 1, ausgewählt aus
[N-(3,5-Dibrombenzyl)-2-[4-(4-fluorphenyl)-1-methylpiperidin-4-yl]-N-methyl-acetamid;
N-[1-(S)-1-(3,5-Bis-trifluormethylphenyl)ethyl]-2-[4-(4-fluorphenyl)-1-methylpiperidin-4-yl]-N-methylacetamid;
N-[1-(3,5-Dibromphenyl)ethyl]-2-[4-(4-fluorphenyl)-1-methylpiperidin-4-yl]-N-methyl-acetamid (Enantiomer 1);
N-[1-(3,5-Dibromphenyl)ethyl]-2-(1-methyl-4-phenylpiperidin-4-yl)-N-methylacetamid (Enantiomer 1);
N-[1-(3,5-Dichlorphenyl)ethyl]-2-[4-(4-fluorphenyl)-1-methylpiperidin-4-yl]-N-methyl-acetamid (Enantiomer 1);
und pharmazeutisch verträglichen Salzen davon (z.B. Hydrochlorid).

8. Verfahren (A) zur Herstellung einer Verbindung, wie in Anspruch 1 beansprucht, welches das Umsetzen eines aktivierten Derivats der Carbonsäure der Formel (II), wobei R₆ eine Stickstoff-Schutzgruppe oder (CH₂)ᵣR₇ ist, mit Amin (III) 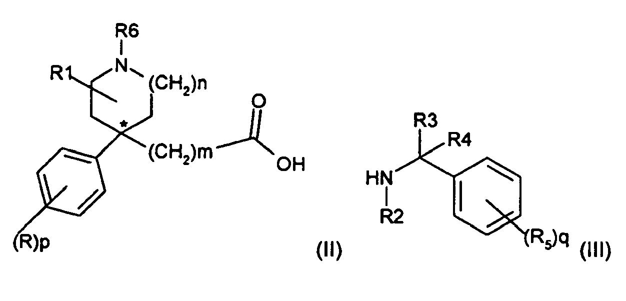 umfasst, wobei R₂ Wasserstoff, C₁₋₄-Alkyl oder eine Stickstoff-Schutzgruppe ist, gefolgt von, wenn nötig, dem Entfernen jeglicher Stickstoff-Schutzgruppe; oder ein Verfahren (B) zur Herstellung einer Verbindung der Formel (I), wobei R₂ C₁₋₄-Alkyl ist, welches das Umsetzen einer Verbindung der Formel (Ia) mit (C₁₋₄)-Alkyl)L umfasst, wobei L eine geeignete Abgangsgruppe, ausgewählt aus Iod, Brom, ist

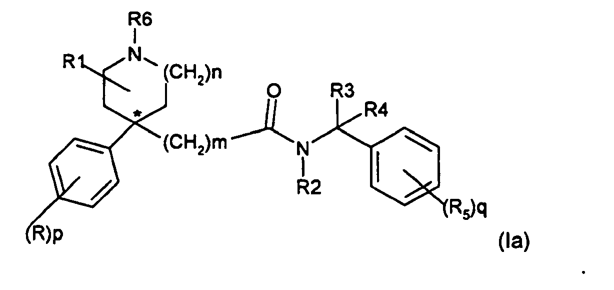

9. Verbindung wie in einem der Ansprüche 1 bis 7 beansprucht, zur Verwendung in der Therapie.

10. Verwendung einer Verbindung, wie in einem der Ansprüche 1 bis 7 beansprucht, zur Herstellung eines Medikaments zur Verwendung in der Behandlung von durch Tachykininen (einschließlich Substanz P und anderen Neurokininen) und/oder durch selektive Hemmung des Serotonin-Wiederaufnahmetransportproteins vermittelten Zuständen.

11. Arzneimittel, umfassend eine Verbindung, wie in einem der Ansprüche 1 bis 7 beansprucht, in Mischung mit einem oder mehreren pharmazeutisch verträglichen Trägem oder Exzipienten.

## Revendications

1. Composé de formule (I) 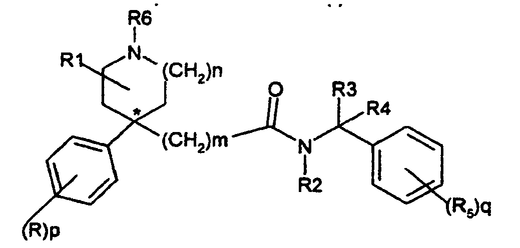 dans laquelle
R représente un groupe halogéno, alkyle en C₁ à C₄, cyano, alkoxy en C₁ à C₄, trifluorométhyle ou trifluorométhoxy ;
R₁ représente un atome d'hydrogène, un groupe halogéno, cycloalkyle en C₃ à C₇, hydroxy, nitro, cyano, ou alkyle en C₁ à C₄ facultativement substitué avec un substituant halogéno, cyano ou alkoxy en C₁ à C₄ ;
R₂ représente un atome d'hydrogène ou un groupe alkyle en C₁ à C₄ ;
R₃ et R₄ représentent indépendamment un atome d'hydrogène, un groupe cyano ou alkyle en C₁ à C₄ ou bien R₃, conjointement avec R₄, représente un groupe cycloalkyle en C₃ à C₇ ;
R₅ représente un groupe trifluorométhyle, S(O)ₜ(alkyle en C₁ à C₄), alkyle en C₁ à C₄, alkoxy en C₁ à C₄, trifluorométhoxy, halogéno ou cyano ;
R₆ représente un atome d'hydrogène ou un groupe (CH₂)R₇ ;
R₇ représente un atome d'hydrogène, un groupe cycloalkyle en C₃ à C₇, NH((alkyle en C₁ à C₄)O(alkoxy en C₁ à C₄)), NH(alkyle en C₁ à C₄), N(alkyle en C₁ à C₄)₂, OC(O)NR₉R₈, NR₈C(O)R₉ ou C(O)NR₉R₈ ;
R₉ et R₈ représentent indépendamment un atome d'hydrogène, un groupe alkyle en C₁ à C₄ ou cycloalkyle en C₃ à C₇ ;
m est égal à 1 ;
n est égal à 1 ou 2 ;
p est égal à zéro ou un nombre entier de 1 à 3 ;
q représente un nombre entier de 1 à 3 ;
r représente un nombre entier de 1 à 4 ;
t est égal à 0, 1 ou 2 ;
et ses sels et produits de solvatation pharmaceutiquement acceptables.

2. Composé suivant la revendication 1, dans lequel R représente un groupe halogéno (par exemple fluoro ou chloro), cyano ou trifluorométhyle ou un groupe alkyle en C₁ à C₄ (par exemple méthyle) et p et égal à 0 ou au nombre entier 1 ou 2.

3. Composé suivant la revendication 1 ou 2, dans lequel R₅ représente un groupe trifluorométhyle, cyano, méthyle ou halogéno et q représente le nombre entier 1 ou 2.

4. Composé suivant l'une quelconque des revendications 1 à 3, dans lequel R₆ représente un atome d'hydrogène ou un groupe (CH₂)ᵣR₇ dans lequel r est égal à 1 ou 2 et R₇ représente un atome d'hydrogène, un groupe cyclopropyle, C(O)N(alkyle en C₁ à C₄) ou C(O)NH(alkyle en C₁ à C₄).

5. Composé suivant l'une quelconque des revendications 1 à 4, dans lequel R représente un groupe alkyle en C₁ à C₄, halogéno (c'est-à-dire chloro ou fluoro), trifluorométhyle ou cyano ; R₁ représente un atome d'hydrogène, un groupe méthyle, éthyle ou halogéno (par exemple fluoro), R₂ représente un groupe méthyle ou un atome d'hydrogène, R₃ et R₄ représentent indépendamment un atome d'hydrogène ou un groupe méthyle, R₅ représente un groupe trifluorométhyle, cyano, méthyle, chloro, bromo ou fluoro, R₆ représente un atome d'hydrogène, un groupe méthyle, éthyle, méthylcyclo-propyle ou CH₂C(O)N(CH₃)₂, p est égal à 0 ou au nombre entier 1 ou 2, m est égal à 1, n est égal à 1 ou 2, q est égal à 1 ou 2.

6. Composé suivant la revendication 1, choisi entre :
le N-(3,5-dichlorobenzyl)-2-[4-(4-fluorophényl)-pipéridine-4-yl]-N-méthylacétamide ;
le N-(3,5-dichlorobenzyl)-2-[3-fluoro-4-(4-fluorophényl)-pipéridine-4-yl]-N-méthylacétamide ;
le N-(3,5-bis-trifluorométhyl)benzyl-2-[(4-fluoro-2-méthylphényl)pipéridine-4-yl]-N-méthyl-acétamide ;
le N-(3,5-dichlorobenzyl)-2-[4-(4-fluoro-2-méthylphényl)-pipéridine-4-yl]-N-méthylacétamide ;
le N-(3,5-bis-trifluorométhylbenzyl)-2-[4-(4-fluorophényl)-azépine-4-yl]-N-méthylacétamide ;
le N-(3,5-bis-trifluorométhylbenzyl)-2-[4-(4-fluoro-2-méthylphényl)azépine-4-yl]-N-méthylacétamide ;
le N-(3,5-dichlorobenzyl)-2-[4-(4-fluoro-2-méthylphényl)-azépine-4-yl]-N-méthylacétamide ;
le N-(3,5-bis-trifluorométhylbenzyl)-2-[3-fluoro-4-(4-fluoro-2-méthylphényl)azépine-4-yl]-N-méthylacétamide ;
le N-(3,5-dichlorobenzyl)-2-[3-fluoro-4-(4-fluoro-2-méthylphényl)azépine-4-yl]-N-méthylacétamide ;
le N-(3,5-dichlorobenzyl)-2-[3-fluoro-4-(4-fluoro-2-méthylphényl)azépine-4-yl]-N-méthylacétamide ;
le N-(3,5-bis-trifluorométhylbenzyl)-2-[3-fluoro-4-(4-fluoro-2-méthylphényl)azépine-4-yl]-N-méthylacétamide ;
le N-(3,5-dibromobenzyl)-2-[4-(4-fluorophényl)pipéridine-4-yl]-N-méthylacétamide ;
le N-(3,5-dibromobenzyl)-2-[4-(4-fluorophényl)-1-méthyl-pipéridine-4-yl]-N-méthylacétamide ;
le N-(3,5-dibromobenzyl)-2-(4-phénylpipéridine-4-yl)-N-méthylacétamide ;
le N-(3,5-dibromobenzyl)-2-(4-phényl-1-méthylpipéridine-4-yl)-N-méthylacétamide ;
le N-[1-(3,5-dichlorophényl)éthyl]-2-[4-(4-fluorophényl)-pipéridine-4-yl]-N-méthylacétamide ;
le N-[1-(3,5-dichlorophényl)éthyl]-2-[4-(4-fluorophényl)-1-méthylpipéridine-4-yl]-N-méthylacétamide ;
le N-[1-(3,5-bis-trifluorométhylphényl)éthyl]-2-[4-(4-fluorophényl)pipéridine-4-yl]-N-méthylacétamide ;
le N-[1-(3,5-bis-trifluorométhylphényl)éthyl]-2-[4-(4-fluorophényl)pipéridine-4-yl]-N-méthylacétamide ;
le N-[1-(3,5-dibromophényl)éthyl]-2-[4-(4-fluorophényl)-pipéridine-4-yl]-N-méthylacétamide ;
le N-[1-(3,5-dibromophényl)éthyl]-2-[4-(4-fluorophényl)-1-méthylpipéridine-4-yl]-N-méthylacétamide ;
le N-[1-(3,5-bis-trifluorométhylphényl)éthyl]-2-(4-phénylpipéridine-4-yl)-N-méthylacétamide;
le N-[1-(3,5-bis-trifluorométhylphényl)éthyl]-2-(4-phényl-1-méthylpipéridine-4-yl)-N-méthylacétamide ;
le N-[1-(3,5-dibromophényl)éthyl]-2-(4-phénylpipéridine-4-yl)-N-méthylacétamide ;
le N-[1-(3,5-dibromophényl)éthyl]-2-(4-phényl-1-méthyl-pipéridine-4-yl)-N-méthylacétamide ;
le N-[1-(3,5-dibromophényl)éthyl]-2-[4-(4-fluorophényl)-pipéridine-4-yl]-N-méthylacétamide ;
le N-[1-(3,5-dibromophényl)éthyl]-2-[4-(4-fluorophényl)-1-méthylpipéridine-4-yl]-N-méthylacétamide ;
le *N*-{1-[3,5-bis(trifluorométhyl)phényl]éthyl}-2-[4-(4-fluoro-2-méthylphényl)-4-pipéridinyl]-N-méthylacétamide ;
le *N*-[(3,5-dibromophényl)méthyl]-2-[4-(4-fluoro-2-méthyl-phényl)-4-pipéridinyl]-N-méthylacétamide ;
le *N*-{[3,5-bis(trifluorométhyl)phényl]méthyl}-2-[4-(4-fluoro-2-méthylphényl)-1-méthyl-4-pipéridinyl]-N-méthylacétamide ;
le *N*-[(3,5-dichlorophényl)méthyl]-2-[4-(4-fluoro-2-méthylphényl)-1-méthyl-4-pipéridinyl]-*N*-méthylacétamide ;
le *N*-{[3,5-bis(trifluorométhyl)phényl]méthyl}-2-[4-(4-fluoro-2-méthylphényl)-4-pipéridinyl]acétamide ;
le *N*-{[3,5-bis(trifluorométhyl)phényl]méthyl}-2-[4-(4-fluoro-2-méthylphényl)-1-méthyl-4-pipéridinyl]acétamide ;
le *N*-[(3,5-dibromophényl)méthyl]-2-[4-(4-fluoro-2-méthyl-phényl)-1-méthyl-4-pipéridinyl]-*N*-méthylacétamide ;
le *N*-[(3,5-dibromophényl)méthyl]-*N*-méthyl-2-[4-(2-méthyl-phényl)-4-pipéridinyl]acétamide ;
le *N*-[(3,5-dibromophényl)méthyl]-*N*-méthyl-2-[1-méthyl-4-(2-méthylphényl)-4-pipéridinyl]acétamide ;
le *N*-[(3,5-dichlorophényl)méthyl]-2-[4-(4-fluorophényl)-1-méthyl-4-pipéridinyl]-*N*-méthylacétamide ;
le *N*-{[3,5-bis(trifluorométhyl)phényl]méthyl}-2-[4-(4-fluorophényl)-1-méthyl-4-pipéridinyl]-*N*-méthylacétamide ;
le *N*-[1-(3,5-dibromophényl)-1-méthyléthyl]-2-[4-(4-fluorophényl)-4-pipéridinyl]-*N*-méthylacétamide ;
le *N*-[1-(3,5-dibromophényl)-1-méthyléthyl]-2-[4-(4-fluorophényl)-1-méthyl-4-pipéridinyl]-*N*-méthylacétamide ;
le *N*-[1-(3,5-bis-trifluorométhylphényl)éthyl]-2-[4-(4-fluorophényl)pipéridine-4-yl]-*N*-méthylacétamide ;
le *N*-[1-(3,5-bis-trifluorométhylphényl)éthyl]-2-[4-(4-fluorophényl)-1-méthylpipéridine-4-yl]-*N*-méthylacétamide ;
le 2-[1-(cyclopropylméthyl)-4-(4-fluorophényl)-4-pipéridinyl]-*N*-[(3,5-dibromophényl)méthyl]-*N*-méthylacétamide ;
le 2-[4-{2-[[(3,5-dibromophényl)méthyl](méthyl)amino]-2-oxoéthyl}-4-(4-fluorophényl)-1-pipéridinyl]-*N,N*-diméthyl-acétamide ;
le *N*-[(3,5-dibromophényl)méthyl-2-[1-éthyl-4-(4-fluoro-phényl)-4-pipéridinyl]-*N*-méthylacétamide ;
le *N*-{1-[3,5-bis(trifluorométhyl)phényl]éthyl}-2-[4-(4-fluorophényl)hexahydro-1*H*-azépine-4-yl]-*N*-méthylacétamide ;
le *N*-{1-[3,5-bis(trifluorométhyl)phényl]éthyl}-2-[4-(4-fluorophényl)-1-méthylhexahydro-1*H*-azépine-4-yl]-*N*-méthylacétamide ;
le *N*-[(3,5-dibromophényl)méthyl]-2-[4-(4-fluorophényl)-hexahydro-1*H*-azépine-4-yl]-*N*-méthylacétamide ;
le *N*-[(3,5-dibromophényl)méthyl]-2-[4-(4-fluorophényl)-1-méthylhexahydro-1*H*-azépine-4-yl]-*N*-méthylacétamide ;
le *N*-[(3-bromo-5-cyanophényl)méthyl]-2-[4-(4-fluorophényl)-4-pipéridinyl]-*N*-méthylacétamide ;
le *N*-[(3-bromo-5-cyanophényl)méthyl]-2-[4-(4-fluoro-phényl)-1-méthyl-4-pipéridinyl]-*N*-méthylacétamide ;
le *N*-[(3,5-dibromophényl)méthyl]-*N*-méthyl-2-{4-[3-(trifluorométhyl)phényl]-4-pipéridinyl}acétamide ;
le *N*-[(3,5-dibromophényl)méthyl]-*N*-méthyl-2-{1-méthyl-4-[3-(trifluorométhyl)phényl]-4-pipéridinyl}acétamide ;
le *N*-[(3,5-dibromophényl)méthyl]-2-[4-{3,4-diméthyl-phényl)-4-pipéridinyl]-*N*-méthylacétamide ;
le *N*-[1-(3,5-dibromophényl)éthyl]-2-[4-(3-fluorophényl)-1-méthyl-4-pipéridinyl]-*N*-méthylacétamide ;
le *N*-[1-(3,5-dibromophényl)éthyl]-2-[4-{4-fluoro-3-méthyl-phényl)-4-pipéridinyl]-*N*-méthylacétamide ;
le *N*-[1-(3,5-dibromophényl)éthyl]-2-[4-(4-fluoro-3-méthyl-phényl)-1-méthyl-4-pipéridinyl]-*N*-méthylacétamide ;
le 2-[4-(3-chlorophényl)-4-pipéridinyl]-*N*-[1-(3,5-dibromo-phényl)éthyl]-*N*-méthylacétamide ;
le *N*-[1-(3,5-dibromophényl)éthyl]-2-[4-(3,4-difluoro-phényl)-4-pipéridinyl]-*N*-méthylacétamide ;
le *N*-[1-{3,5-dibromophényl)éthyl]-2-[4-(3,4-difluoro-phényl)-1-méthyl-4-pipéridinyl]-*N*-méthylacétamide ;
le *N*-[1-(3,5-dibromophényl)éthyl]-2-[4-{3-fluorophényl)-4-pipéridinyl]-*N*-méthylacétamide ;
le *N*-[1-(3,5-dibromophényl)éthyl]-2-[4-(3-fluorophényl)-1-méthyl-4-pipéridinyl]-*N*-méthylacétamide ;
le *N*-[1-(3,5-dibromophényl)éthyl]-2-[4-(4-fluoro-3-méthyl-phényl)-4-pipéridinyl]-*N*-méthylacétamide ;
le *N*-[1-(3,5-dibromophényl)éthyl]-2-[4-(4-fluoro-3-méthyl-phényl)-1-méthyl-4-pipéridinyl]-*N*-méthylacétamide ;
le 2-[4-{3-chlorophényl)-4-pipéridinyl]-*N*-[1-(3,5-dibromo-phényl)éthyl]-*N*-méthylacétamide ;
le 2-[4-(3-chlorophényl)-1-méthyl-4-pipéridinyl]-*N*-[1-(3,5-dibromophényl)éthyl]-*N*-méthylacétamide ;
le 2-[4-{3-chlorophényl)-4-pipéridinyl]-*N*-[1-(3,5-dichloro-phényl)éthyl]-*N*-méthylacétamide ;
le 2-[4-(3-chlorophényl)-1-méthyl-4-pipéridinyl]-*N*-[1-(3,5-dichlorophényl)éthyl]-*N*-méthylacétamide ;
le 2-[4-(3-chlorophényl)-4-pipéridinyl]-*N*-[(3,5-dibromo-phényl)méthyl]-*N*-méthylacétamide ;
le *N*-[1-(3,5-dichlorophényl)éthyl}-2-[4-(4-fluoro-3-méthylphényl)-4-pipéridinyl]-*N*-m-éthylacétamide ;
le *N*-[(3,5-dibromophényl)méthyl]-2-[4-(4-fluoro-3-méthyl-phényl)-4-pipéridinyl]-*N*-méthylacétamide ;
le *N*-[(3,5-dibromophényl)méthyl]-2-[4-(4-fluoro-3-méthyl-phényl}-1-méthyl-4-pipéridinyl]-*N*-méthylacétamide ;
le *N*-[(3,5-dibromophényl)méthyl]-2-[4-(3-fluorophényl)-4-pipéridinyl]-*N*-méthylacétamide ;
le *N*-[(3,5-dibromophényl)méthyl]-2-[4-(3-fluorophényl)-1-méthyl-4-pipéridinyl]-*N*-méthylacétamide ;
le *N*-[(3,5-dibromophényl)méthyl]-2-[4-(3,4-difluorophényl)-4-pipéridinyl]-*N*-méthylacétamide ;
le *N*-[(3,5-dibromophényl)méthyl]-2-[4-(3,4-difluorophényl)-1-méthyl-4-pipéridinyl]-*N*-méthylacétamide ;
le 2-[4-(4-cyanophényl)-4-pipéridinyl]-*N*-[1-(3,5-dibromo-phényl)éthyl]-*N*-méthylacétamide ;
ses diastéréoisomères ou énantiomères et leurs sels pharmaceutiquement acceptables (par exemple le chlorhydrate).

7. Composé suivant la revendication 1, choisi entre :
le [N-(3,5-dibromobenzyl)-2-[4-(4-fluorophényl)-1-méthyl-pipéridine-4-yl]-N-méthylacétamide ;
le N-[1-(*S*)-1-(3,5-bis-trifluorométhylphényl)éthyl]-2-[4-(4-fluorophényl)-1-méthylpipéridine-4-yl]-N-méthylacétamide ;
le N-[1-(3,5-dibromophényl)éthyl]-2-[4-(4-fluorophényl)-1-méthylpipéridine-4-yl]-N-méthylacétamide (énantiomère 1) ;
le N-[1-(3,5-dibromophényl)éthyl]-2-(1-méthyl-4-phényl-pipéridine-4-yl)-N-méthylacétamide (énantiomère 1) ;
le N-[1-(3,5-dibromophényl)éthyl]-2-[4-(4-fluorophényl)-1-méthylpipéridine-4-yl]-N-méthylacétamide (énantiomère 1) ;
et ses sels pharmaceutiquement acceptables (par exemple le chlorhydrate).

8. Procédé (A) pour la préparation d'un composé suivant la revendication 1, qui comprend la réaction d'un dérivé activé de l'acide carboxylique de formule (II) dans laquelle R₆ représente un groupe protecteur de l'atome d'azote ou un groupe (CH₂)rR₇, avec une amine (III) 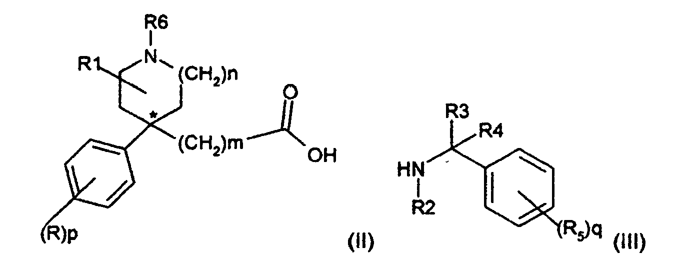 dans laquelle R₂ représente un atome d'hydrogène, un groupe alkyle en C₁ à C₄ ou un groupe protecteur de l'atome d'azote, avec ensuite, lorsque cela est nécessaire, l'élimination de n'importe quel groupe protecteur de l'atome d'azote ; ou un procédé B pour la préparation d'un composé de formule (I) dans laquelle R₂ représente un groupe alkyle en C₁ à C₄, qui comprend la réaction d'un composé de formule (Ia), avec un composé de formule (alkyle en C₁ à C₄) L dans laquelle L représente un groupe partant convenable choisi entre l'iode et le brome.

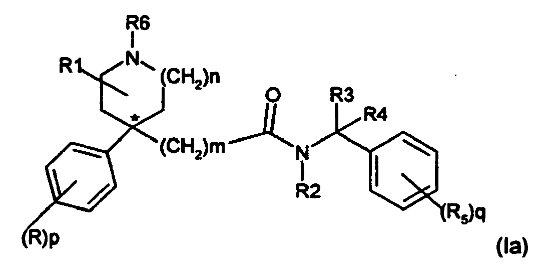

9. Composé suivant l'une quelconque des revendications 1 à 7, destiné à être utilisé en thérapeutique.

10. Utilisation d'un composé suivant l'une quelconque des revendications 1 à 7 dans la préparation d'un médicament destiné à être utilisé dans le traitement d'affections à médiation par des tachykinines (comprenant la substance P et d'autres neurokinines) et/ou par inhibition sélective de la protéine servant de transporteur de réabsorption de sérotonine.

11. Composition pharmaceutique comprenant un composé suivant l'une quelconque des revendications 1 à 7 en mélange avec un ou plusieurs supports ou excipient pharmaceutiquement acceptables.
